(19) **Europäisches Patentamt / European Patent Office / Office européen des brevets**

(11) **EP 1 809 172 B1**

(12) **EUROPEAN PATENT SPECIFICATION**

(45) Date of publication and mention
of the grant of the patent:
**12.07.2017 Bulletin 2017/28**

(21) Application number: **05806702.6**

(22) Date of filing: **30.09.2005**

(51) Int Cl.:
*A61B 5/04* (2006.01)   *A61N 1/362* (2006.01)
*A61N 1/365* (2006.01)   *A61N 1/37* (2006.01)

(86) International application number:
**PCT/US2005/035638**

(87) International publication number:
**WO 2006/039693 (13.04.2006 Gazette 2006/15)**

(54) **CARDIAC ACTIVATION SEQUENCE MONITORING AND TRACKING**

MONITORING UND VERFOLGUNG VON KARDIALER AKTIVIERUNGSSEQUENZ

SURVEILLANCE ET SUIVI DE SEQUENCES D'ACTIVATION CARDIAQUE

(84) Designated Contracting States:
**AT BE BG CH CY CZ DE DK EE ES FI FR GB GR HU IE IS IT LI LT LU LV MC NL PL PT RO SE SI SK TR**

(30) Priority:  **30.09.2004 US 955397**
**30.11.2004 US 631742 P**
**14.03.2005 US 79744**
**09.05.2005 US 124950**
**09.05.2005 US 124972**
**09.05.2005 US 125020**
**09.05.2005 US 125068**

(43) Date of publication of application:
**25.07.2007 Bulletin 2007/30**

(73) Proprietor: **CARDIAC PACEMAKERS, INC.**
**St. Paul, MN 55112 (US)**

(72) Inventors:
• **ZHANG, Yi**
**Blaine, MN 55449 (US)**
• **MCCABE, Aaron R.**
**Minneapolis, MN 55403 (US)**
• **MEYER, Scott A.**
**Lakeville, MN 55044 (US)**

• **STAHMANN, Jeffrey E.**
**Ramsey, MN 55303 (US)**
• **RICCI, Carlos Alberto**
**Apple Valley, MN 55124 (US)**
• **YU, Yinghong**
**Shoreview, MN 55126 (US)**
• **DING, Jiang**
**Shoreview, MN 55126 (US)**
• **BROCKWAY, Marina**
**Shoreview, MN 55126 (US)**
• **HOPPER, Donald I.**
**Maple Grove, MN 55369 (US)**
• **SWEENEY, Robert J.**
**Woodbury, MN 55125 (US)**

(74) Representative: **Pfenning, Meinig & Partner mbB**
**Patent- und Rechtsanwälte**
**Joachimsthaler Straße 12**
**10719 Berlin (DE)**

(56) References cited:
**WO-A-92/17240**       **WO-A2-03/003905**
**US-A1- 2003 204 146**   **US-A1- 2004 111 021**
**US-B1- 6 505 067**

**Description**

FIELD OF THE INVENTION

The present invention relates generally to implantable medical devices employing signal separation and, more particularly, to cardiac sensing and/or stimulation devices employing cardiac activation sequence monitoring and tracking.

BACKGROUND OF THE INVENTION

The healthy heart produces regular, synchronized contractions. Rhythmic contractions of the heart are normally initiated by the sinoatrial (SA) node, which is a group of specialized cells located in the upper right atrium. The SA node is the normal pacemaker of the heart, typically initiating 60-100 heartbeats per minute. When the SA node is pacing the heart normally, the heart is said to be in normal sinus rhythm.

If the heart's electrical activity becomes uncoordinated or irregular, the heart is denoted to be arrhythmic. Cardiac arrhythmia impairs cardiac efficiency and may be a potential life-threatening event. Cardiac arrhythmias have a number of etiological sources, including tissue damage due to myocardial infarction, infection, or degradation of the heart's ability to generate or synchronize the electrical impulses that coordinate contractions.

Bradycardia occurs when the heart rhythm is too slow. This condition may be caused, for example, by impaired function of the SA node, denoted sick sinus syndrome, or by delayed propagation or blockage of the electrical impulse between the atria and ventricles. Bradycardia produces a heart rate that is too slow to maintain adequate circulation.

When the heart rate is too rapid, the condition is denoted tachycardia. Tachycardia may have its origin in either the atria or the ventricles. Tachycardias occurring in the atria of the heart, for example, include atrial fibrillation and atrial flutter. Both conditions are characterized by rapid contractions of the atria. Besides being hemodynamically inefficient, the rapid contractions of the atria may also adversely affect the ventricular rate.

Ventricular tachycardia occurs, for example, when electrical activity arises in the ventricular myocardium at a rate more rapid than the normal sinus rhythm. Ventricular tachycardia may quickly degenerate into ventricular fibrillation. Ventricular fibrillation is a condition denoted by extremely rapid, uncoordinated electrical activity within the ventricular tissue. The rapid and erratic excitation of the ventricular tissue prevents synchronized contractions and impairs the heart's ability to effectively pump blood to the body, which is a fatal condition unless the heart is returned to sinus rhythm within a few minutes.

Implantable cardiac rhythm management systems have been used as an effective treatment for patients with serious arrhythmias, as well as for patients with conditions such as heart failure. These systems typically include one or more leads and circuitry to sense signals from one or more interior and/or exterior surfaces of the heart. Such systems also include circuitry for generating electrical pulses that are applied to cardiac tissue at one or more interior and/or exterior surfaces of the heart. For example, leads extending into the patient's heart are connected to electrodes that contact the myocardium for sensing the heart's electrical signals and for delivering pulses to the heart in accordance with various therapies for treating arrhythmias.

Typical implantable cardioverter/defibrillators include one or more endocardial leads to which at least one defibrillation electrode is connected. Such implantable cardioverter/defibrillators are capable of delivering high-energy shocks to the heart, interrupting the ventricular tachyarrhythmia or ventricular fibrillation, and allowing the heart to resume normal sinus rhythm. Implantable cardioverter/defibrillators may also include pacing functionality.

Document WO 03/003905 discloses medical devices for recording cardiac signals and separating the recorded cardiac signals.

SUMMARY OF THE INVENTION

The present invention is defined in the claims; it is directed to physiological signal monitoring and/or stimulation systems, such as those that provide monitoring, diagnosing, defibrillation therapies, pacing therapies, drug therapies, neurostimulation therapies or a combination of these capabilities. Embodiments of the present invention relate generally to implantable medical devices employing signal separation and, more particularly, to monitoring and/or stimulation devices employing automated cardiac activation sequence monitoring and/or tracking.

Embodiments of the invention are directed to devices having a signal processor that receives two or more composite signals associated with two or more sources, performs a source separation, and produces one or more cardiac signal vectors associated with all or a portion of one or more cardiac activation sequences based on the source separation. One or both of performing the source separation and producing the cardiac signal vectors may be patient-internal or patient-external operations.

A change may be detected in a characteristic of the cardiac signal vectors relative to a baseline, such as established by an initial source separation and/or clinical information. The change may be detected using a subsequent source separation. The change detected may involve one or more of: an angle change of one or more cardiac signal vectors; a magnitude change of one or more cardiac signal vectors; a variance change of one or more cardiac signal vectors; a power spectral density change of the angle of one or more cardiac signal vectors; a power spectral density change of the magnitude of one or more cardiac signal vectors; a trajectory change of one or more cardiac signal vectors; a temporal profile change of one or more cardiac signal vectors; a rate of change of angle of one or more cardiac signal vectors; a rate of change of magnitude of one or more cardiac signal vectors; a rate of change of variance of one or more cardiac signal vectors; a rate of change of temporal profile of one or more cardiac signal vectors; a trend of the angle of one or more cardiac signal vectors; a trend of the magnitude of one or more cardiac signal vectors; a trend of the variance of one or more cardiac signal vectors; and a trend of the temporal profile of one or more cardiac signal vectors.

The change may be detected beat-to-beat, within a cardiac cycle, over a predetermined time period, over two or more cardiac cycles, at a pre-determined time, upon the reception of an external stimulus, and upon the reception of a patient-activated stimulus, for example. The detected change may be used to detect anomalous cardiac activity, diagnose an anomalous cardiac conduction, and/or diagnose a cardiac disease or condition.

The cardiac activation sequences may be indicative of about a full cardiac cycle, a predetermined period of a cardiac cycle, a predetermined period of two or more cardiac cycles, two or more cardiac cycles, about one third of a cardiac cycle, about a QRS complex of a cardiac cycle and/or an ST segment of a cardiac cycle, for example.

Methods may further involve storing information associated with one or more cardiac signal vectors, such as cardiac activation sequence information. One or more vectors and/or activation sequences may be selected, and information associated with the vectors and/or activation sequences may be stored and tracked. Methods may also involve transmitting information associated with one or more cardiac signal vectors to a patient-external device, such as cardiac activation sequence information. Embodiments of the invention involve acquiring information associated with one or more of a patient's posture, activity, movement, heart-rate, heart rhythm, respiration, blood-pressure, blood gas concentration, blood chemistry, temperature, heart-sound, cardiac output, cardiac stroke volume, cardiac wall motion, peripheral or pulmonary fluid status, autonomic system status, and heart-rate variability. The acquired information may be used to facilitate interpretation of one or more cardiac signal vectors.

Additional methods of the present disclosure involve sensing two or more composite signals using three or more cardiac electrodes, and performing a source separation that produces two or more vectors. One or more vectors are selected after performing the source separation, and information associated with the vectors may be stored and used to track the selected vectors. Tracking the selected vectors may be useful to determine a cardiac activation sequence. Information associated with one or both of the selected vectors and the cardiac activation sequence may be transmitted to a patient-external device, and displayed. Subsequent source separations may be performed to detect changes in selected vectors.

Devices in accordance with the present invention include three or more electrodes, the electrodes configured for sensing a composite signal, thereby providing two or more composite signals. A housing configured for implantation in a patient includes a controller. A signal processor and a memory are coupled to the controller and configured to perform a source separation, such as a blind source separation algorithm, using the sensed two or more composite signals. The source separation produces one or more cardiac signal vectors associated with all or a portion of one or more cardiac activation sequences, information from which may be stored in the memory.

The signal processor and memory may be provided in the housing, or may be provided in a patient-external device or system such as a network server system and/or an advanced patient management system. The signal processor and the controller may be coupled to respective communication devices to facilitate wireless communication between the signal processor and controller. The controller may detect a cardiac condition using the vector information.

The system may include a header configured for coupling a lead to the housing, and one or more of the electrodes may be provided on the header. Embodiments of systems may include at least four electrodes, wherein one of the at least four electrodes does not lie spatially in the same plane as the other electrodes. The system may further include a lead configured for subcutaneous non-intrathoracic placement in a patient, which may support one or more electrodes and/or other sensors. The sensors may be configured to measure one or more of a patient's posture, activity, movement, heart rate, heart rhythm, respiration, blood pressure, blood gas concentration, blood chemistry, temperature, heart-sound, cardiac output, stroke volume, cardiac wall motion, peripheral or pulmonary fluid status, autonomic system status, and heart-rate variability. Information from the sensors may be used to interpret the cardiac signal vectors.

Embodiments of the invention are directed to devices involving sensing a plurality of composite cardiac signals using a plurality of implantable electrodes. A source separation is performed using the sensed plurality of composite cardiac signals. One or more cardiac signal vectors are produced that are associated with all or a portion of one or more cardiac activation sequences based on the source separation. A cardiac response to the one or more pacing pulses is classified using one or more characteristics associated with one or both of the one or more cardiac signal vectors and the signals associated with the one or more cardiac signal vectors.

Further embodiments may involve classifying the cardiac response as capture or non-capture, or classifying the cardiac response as fusion or intrinsic cardiac activity. The one or more characteristics may include an angle or an angle change of the one or more cardiac signal vectors. In other embodiments, the one or more characteristics may include a predetermined range of angles of the one or more cardiac signal vectors, and classifying the cardiac response may involve determining if the vector falls within the predetermined range. For example, the one or more characteristics may include a morphology of one or more signals associated with the one or more cardiac signal vectors, and/or a morphological change, relative to a baseline, of the one or more signals associated with the one or more cardiac signal vectors.

Embodiments of the invention are directed to devices involving sensing a plurality of composite cardiac signals using a plurality of implantable electrodes. A source separation is performed using the sensed plurality of composite cardiac signals and the separation produces one or more cardiac signal vectors associated with one or more cardiac activation sequences that is indicative of arrhythmia. A change of the one or more cardiac signal vectors is detected using the one or more cardiac signal vectors. Cardiac arrhythmias are discriminated using the one or more cardiac signal vectors.

Specific embodiments are directed to discriminating between one or more of: arrhythmia and non-arrhythmia; supra-ventricular tachycardia and ventricular tachycardia; polymorphic ventricular tachycardia and monomorphic ventricular tachycardia; left-ventricular tachycardia and right-ventricular tachycardia; and premature atrial contraction and premature ventricular contraction. Further embodiments determine the beat-to-beat variance of a characteristic of the one or more cardiac signal vectors to aid in discrimination.

Example embodiments include discriminating premature atrial contraction by detecting a positive phase of a dominant orientation of the one or more cardiac signal vectors, and discriminating premature ventricular contraction by detecting a negative phase of a dominant orientation of one or more cardiac signal vectors. Further embodiments may establish a baseline from the performed source separation, wherein the change may be detected using a subsequent source separation. Examples of detected changes in accordance with embodiments of the present invention include one or more of: a phase angle change of the one or more cardiac signal vectors, a magnitude change of the one or more cardiac signal vectors, a variance change of the one or more cardiac signal vectors, a power spectral density change of the phase of the one or more cardiac signal vectors, a power spectral density change of the magnitude of the one or more cardiac signal vectors, a trajectory change of the one or more cardiac signal vectors, a temporal profile change of the one or more cardiac signal vectors, a rate of change of phase angle of the one or more cardiac signal vectors, a rate of change of magnitude of the one or more cardiac signal vectors, a rate of change of variance of the one or more cardiac signal vectors, a rate of change of temporal profile of the one or more cardiac signal vectors, a trend of the phase angle of the one or more cardiac signal vectors, a trend of the magnitude of the one or more cardiac signal vectors, a trend of the variance of the one or more cardiac signal vectors, and a trend of the temporal profile of the one or more cardiac signal vectors.

Other methods in accordance with the present disclosure involve sensing a plurality of composite cardiac signals using a plurality of implantable electrodes, performing a source separation that produces a plurality of vectors, and selecting one or more vectors from the plurality of vectors associated with the specific segment of a cardiac cycle. Information associated with the selected one or more vectors is stored and used to track the selected one or more vectors, which may be used for discriminating cardiac arrhythmias.

Embodiments of the invention are directed to devices involving sensing a plurality of composite cardiac signals using a plurality of implantable electrodes. A source separation is performed using the sensed plurality of composite signals and the separation produces one or more cardiac signal vectors associated with one or more cardiac activation sequences that is indicative of ischemia. A change of the one or more cardiac signal vectors is detected using the one or more cardiac signal vectors. For example, the change may be an elevation of the ST segment of a cardiac cycle or other change indicative of myocardial ischemia, myocardial infarction, or other pathologic change. The change may be used to predict, quantify, and/or qualify the risk of an event such as an arrhythmia, a myocardial infarction, or other pathologic change.

Other methods in accordance with the present disclosure involve sensing a plurality of composite cardiac signals using a plurality of implantable electrodes, performing a source separation that produces a plurality of vectors, and selecting one or more vectors from the plurality of vectors associated with the specific segment of a cardiac cycle. Information associated with the selected one or more vectors is stored and used to track the selected one or more vectors, which may be used in detecting ischemia. Tracking the vectors may involve performing a subsequent source separation using a plurality of subsequently detected composite signals, and detecting a change in the selected one or more vectors indicative of ischemia.

Embodiments of cardiac systems in accordance with the present invention include implantable electrodes configured for sensing composite signals. A housing may be configured for implantation in a patient that houses a memory and a controller that is coupled to the memory and the implantable electrodes. The controller may be configured to perform a source separation using the sensed composite signals, the source separation producing one or more cardiac signal vectors associated with a segment of one or more cardiac activation sequences, and to detect ischemia using the one or more cardiac signal vectors.

The controller may predict one or both of myocardial infarction and cardiac arrhythmia using information provided from the cardiac signal vectors associated with a segment of one or more cardiac activation sequences. A filter window may be defined by one or more features of a cardiac waveform separated from the plurality of composite signals. The window may be centered on the ST segment of the cardiac cycle, for example, to detect ischemia. Additionally or alternately, a signal processor may be provided in a patient-external device or system, such as a network server system, the signal processor and the controller coupled to respective communication devices to facilitate wireless communication between the signal processor and controller. The signal processor may additionally or optionally be used to perform the source separation, vector monitoring and/or tracking, and/or the ischemia detection.

Embodiments of the invention are directed to devices involving sensing a plurality of composite cardiac signals using a plurality of implantable electrodes. A source separation is performed using the sensed plurality of composite cardiac signals, which produces one or more cardiac signal vectors associated with all or a portion of one or more cardiac activation sequences. A change in a patient's posture is detected using the one or more cardiac signal vectors.

Further embodiments involve sensing the plurality of composite cardiac signals during the patient's predominant cardiac rhythm before detecting the change in the patient's posture. Other embodiments involve detecting a change in the one or more cardiac signal vectors, and discriminating between one of a postural related change and a cardiac rhythm related change using the detected change in the one or more cardiac signal vectors. In further embodiments, the patient's posture change is used to interpret the one or more cardiac signal vectors.

Other embodiments of the invention are directed to devices having a plurality of electrodes configured for sensing a composite cardiac signal, thereby providing a plurality of composite cardiac signals. A housing is configured for implantation in a patient, with a controller provided in the housing and coupled to the plurality of electrodes. A signal processor is provided, coupled to a memory. The signal processor is configured to perform a source separation using the sensed plurality of composite signals, the source separation producing one or more cardiac signal vectors associated with all or a portion of one or more cardiac activation sequences, and to store vector information in the memory, the vector information used for characterizing the patient's posture. For example, the controller may determine the patient's posture using the vector information.

Embodiments of the invention are directed to devices that involve the sensing of composite cardiac signals using a implantable electrodes. A source separation is performed using the sensed composite cardiac signals, producing one or more cardiac signal vectors associated with all or a portion of one or more cardiac activation sequences. A cardiac resynchronization therapy is adjusted using one or both of the one or more cardiac signal vectors and the signals associated with the one or more cardiac signal vectors.

In further embodiments, the cardiac resynchronization therapy may be initiated, terminated, or one or more parameters of the resynchronization therapy may be altered. In specific embodiments, an angle of the one or more cardiac signal vectors may be determined, wherein adjusting the cardiac resynchronization therapy may be based on the determined angle. For example, a dominant orientation of the QRS vector may be moved in a direction of an angle of a baseline QRS vector by adjusting one or more parameters of the cardiac resynchronization therapy.

An embodiment of a cardiac system in accordance with the present invention includes implantable electrodes configured for sensing a composite signal, thereby providing composite signals. A housing, configured for implantation in a patient, includes a controller coupled to the implantable electrodes. A memory may be provided in the housing and coupled to the controller, or may be provided in a patient-external device and coupled to the controller, such as by using wireless communications. The controller is configured to perform a source separation using the sensed plurality of composite signals, the source separation producing one or more cardiac signal vectors associated with all or a portion of one or more cardiac activation sequences, and to providing a closed-loop control of a cardiac resynchronization therapy using the one or more cardiac signal vectors.

The above summary of the present invention is not intended to describe each embodiment or every implementation of the present invention. Advantages and attainments, together with a more complete understanding of the invention, will become apparent and appreciated by referring to the following detailed description and claims taken in conjunction with the accompanying drawings.

BRIEF DESCRIPTION OF THE DRAWINGS

Figures 1A and 1B are pictorial diagrams of an electrocardiogram (ECG) waveform for three consecutive heartbeats (Figure 1A) and a magnified portion of the electrocardiogram (ECG) waveform for the first two consecutive heartbeats (Figure 1B) ;

Figure 2 is a polar plot of a cardiac vector superimposed over a frontal view of a thorax, with the origin of the polar plot located at the AV node of a patient's heart;

Figure 3A is a polar plot of cardiac vectors obtained using a source separation in accordance with the present invention;

Figure 3B illustrates polar plots of cardiac vectors obtained from selected portions of an electrocardiogram using source separation in accordance with the present invention;

Figure 4 is a graph of temporal profiles of a cardiac vector useful for diagnosing a cardiac disease in accordance with the present invention;

Figures 5A through 5D illustrate cardiac vectors superimposed over a sectional view of the ventricles of a patient's heart;

Figure 6A is a block diagram of a method of detecting a change in one or more cardiac signal vectors associated with all or a portion of one or more cardiac activation sequences based on a source separation in accordance with the present invention;

Figure 6B is a block diagram of another embodiment of a method of detecting a change in one or more cardiac signal vectors associated with all or a portion of one or more cardiac activation sequences based on a source separation in accordance with the present invention;

Figure 6C is a graph illustrating the change in vector orientation due to a posture change in accordance with embodiments of the present invention;

Figure 6D is a table of vector orientations possible based on combinations of posture and intrinsic or paced electrophysiology signals in accordance with embodiments of the present invention;

Figure 6E is a flow chart of an embodiment of methods for recording and storing posture information in accordance with the present invention;

Figure 6F is a flow chart of an embodiment of methods for determining a patient's posture in accordance with the present invention;

Figure 6G is a graph illustrating activation sequence vector angles for intrinsic and paced conditions;

Figure 6H is a graph of asynchrony measurements in accordance with the present invention;

Figure 6I is a flow chart of a method of titrating CRT in accordance with the present invention;

Figure 6J is a graph illustrating activation sequence vector angles for intrinsic and paced conditions;

Figure 6K is a flow chart of a method of capture verification in accordance with the present invention;

Figure 6L is a flow chart of a method of capture threshold adjustment in accordance with the present invention;

Figure 6M is a flow chart of a method of verifying left ventricular (LV), right ventricular (RV), and bi-ventricular (BiV) capture in accordance with the present invention;

Figure 6N is a flow chart of a method of differentiating supra-ventricular tachycardia (SVT) from ventricular tachycardia (VT) in accordance with the present invention;

Figure 6O is a graph of vector angles for an example of cardiac activation sequence orientations showing intrinsic, ventricle originated, and atrium originated orientations;

Figure 6P is a graph illustrating the variance of activation sequence vector angles during monomorphic ventricular tachycardia (MVT) and polymorphic ventricular tachycardia (PVT);

Figure 6Q is a flow chart of a method of differentiating MVT from PVT in accordance with the present invention;

Figure 6R is a flow chart of a method of differentiating left ventricular tachycardia (LVT) from right ventricular tachycardia (RVT) in accordance with the present invention;

Figures 6S, 6T, and 6U are graphs illustrating activation sequence vector angles for a rectangular windowed portion of a cardiac signal, starting at 0.001 seconds, from a live porcine subject at baseline, partial occlusion, and complete occlusion of the left anterior descending (LAD) artery on the heart respectively, in accordance with the present invention;

Figures 6V, 6W, and 6X are graphs illustrating activation sequence vector angles for a rectangular windowed portion of a cardiac signal, starting at 0.07 seconds, from a live porcine subject at baseline, partial occlusion, and complete occlusion of the LAD artery on the heart respectively, in accordance with the present invention;

Figures 6Y, 6Z, and 6AA are graphs illustrating activation sequence vector angles for a rectangular windowed portion of a cardiac signal, starting at 0.14 seconds, from a live porcine subject at baseline, partial occlusion, and complete occlusion of the LAD artery on the heart respectively, in accordance with the present invention;

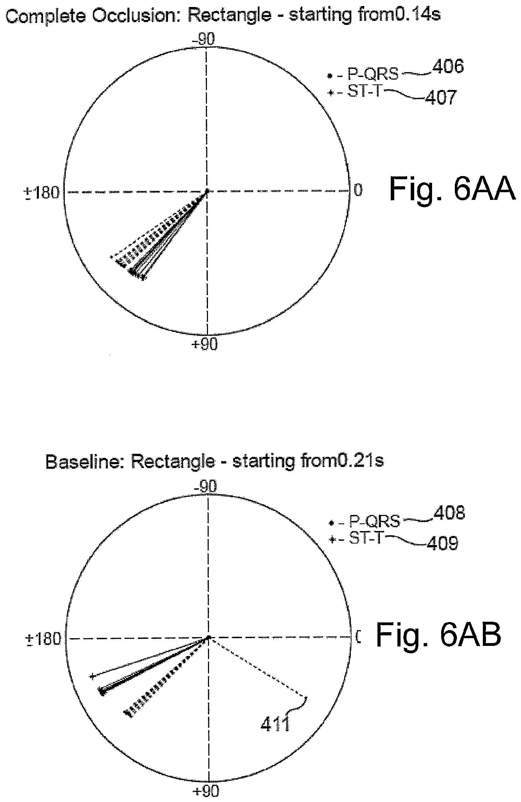

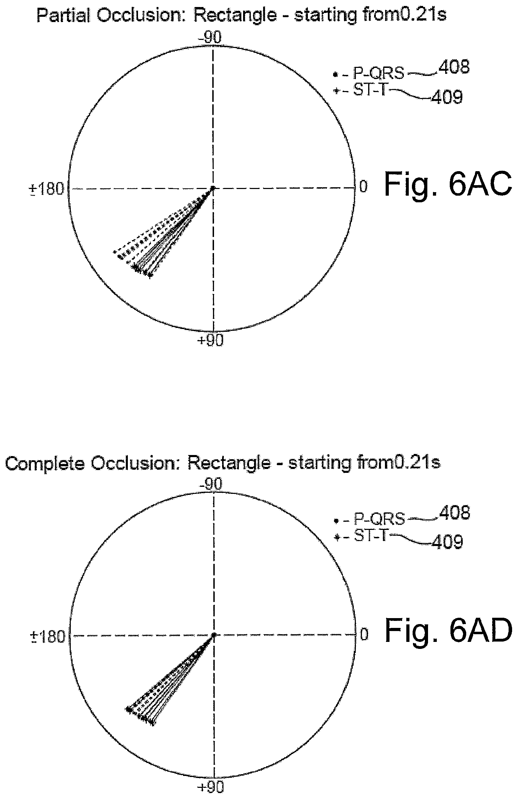

Figures 6AB, 6AC, and 6AD are graphs illustrating activation sequence vector angles for a rectangular windowed portion of a cardiac signal, starting at 0.21 seconds, from a live porcine subject at baseline, partial occlusion, and complete occlusion of the LAD artery on the heart respectively, in accordance with the present invention;

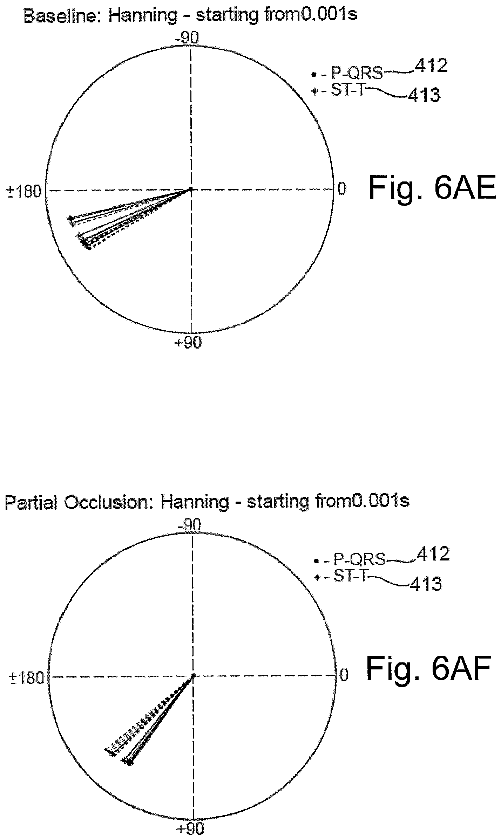

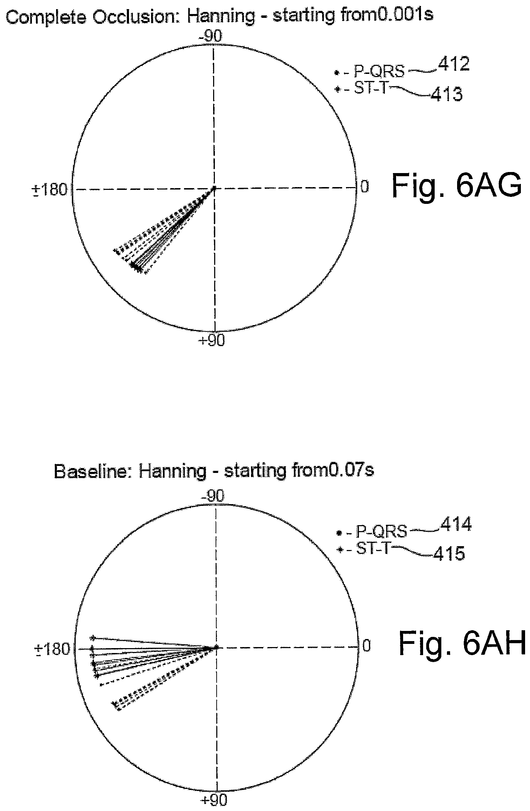

Figures 6AE, 6AF, and 6AG are graphs illustrating activation sequence vector angles for a Hanning windowed portion of a cardiac signal, starting at 0.001 seconds, from a live porcine subject at baseline, partial occlusion, and complete occlusion of the LAD artery on the heart respectively, in accordance with the present invention;

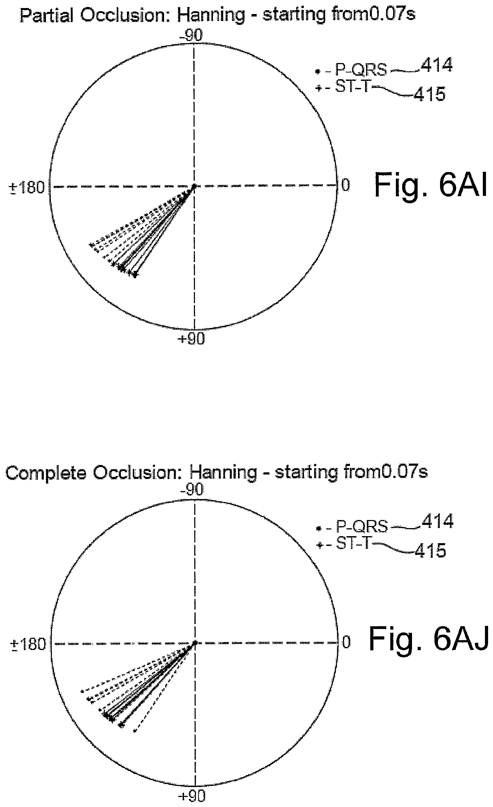

Figures 6AH, 6AI, and 6AJ are graphs illustrating activation sequence vector angles for a Hanning windowed portion of a cardiac signal, starting at 0.07 seconds, from a live porcine subject at baseline, partial occlusion, and complete occlusion of the LAD artery on the heart respectively, in accordance with the present invention;

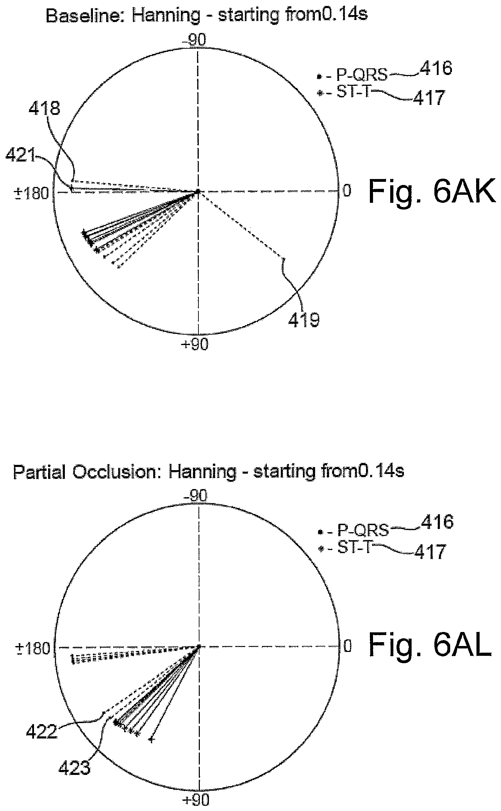

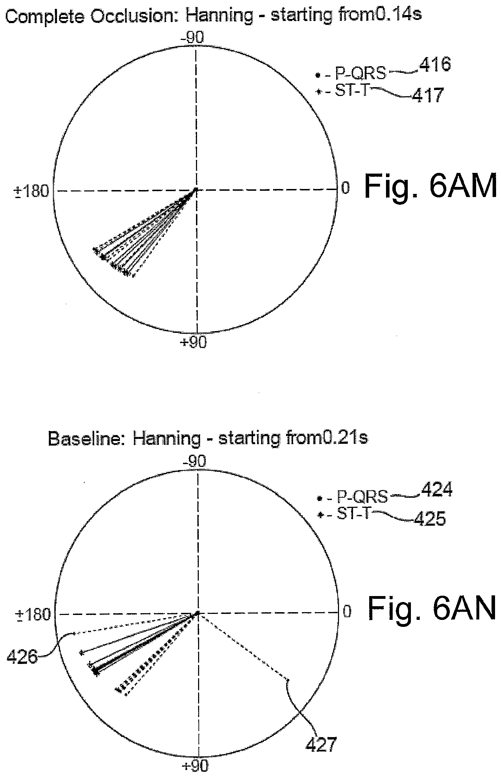

Figures 6AK, 6AL, and 6AM are graphs illustrating activation sequence vector angles for a Hanning windowed portion of a cardiac signal, starting at 0.14 seconds, from a live porcine subject at baseline, partial occlusion, and complete occlusion of the LAD artery on the heart respectively, in accordance with the present invention;

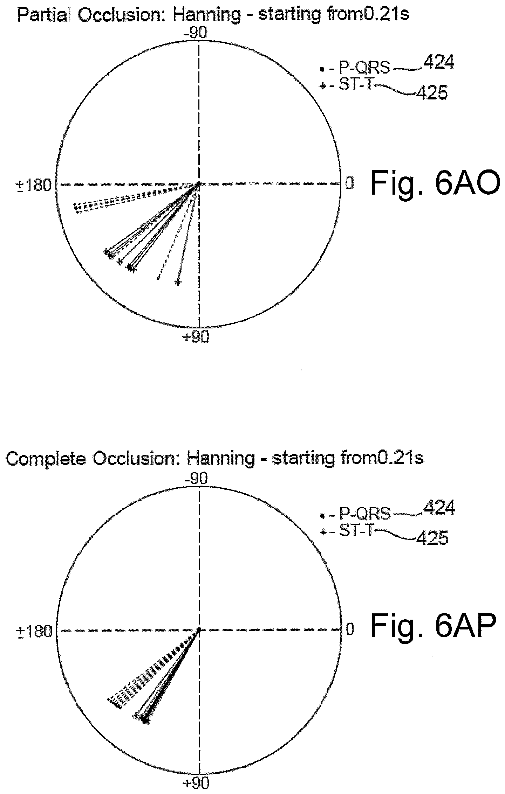

Figures 6AN, 6AO, and 6AP are graphs illustrating activation sequence vector angles for a Hanning windowed portion of a cardiac signal, starting at 0.21 seconds, from a live porcine subject at baseline, partial occlusion, and complete occlusion of the LAD artery on the heart respectively, in accordance with the present invention;

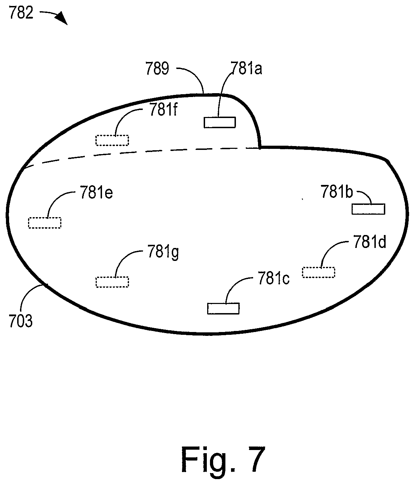

Figure 7 is a top view of an implantable cardiac device in accordance with the present invention, having at least three electrodes;

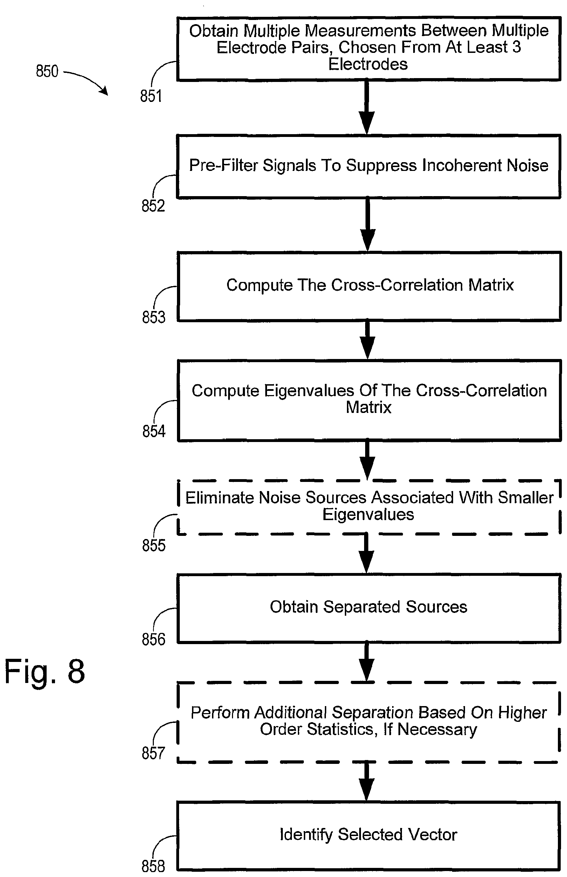

Figure 8 is a block diagram of a cardiac activation sequence monitoring and/or tracking process in accordance with the present invention;

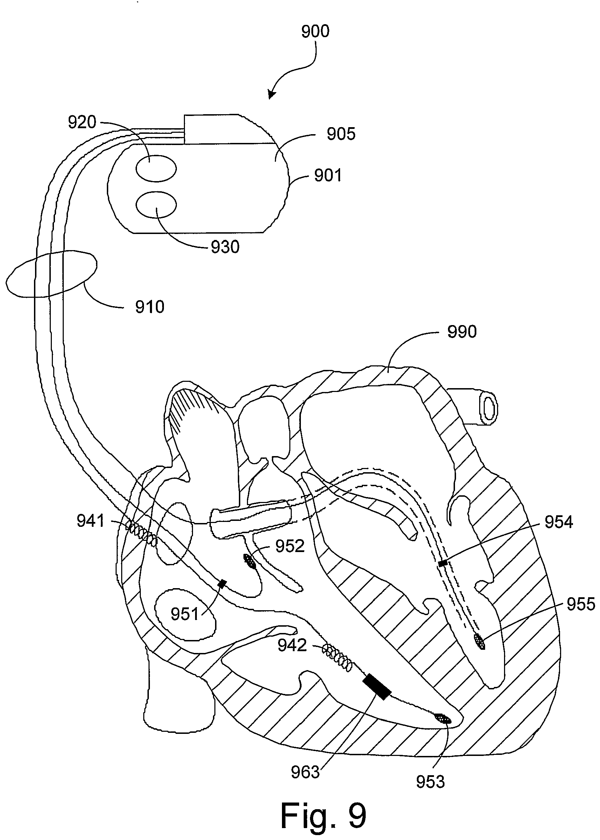

Figure 9 is an illustration of an implantable cardiac device including a lead assembly shown implanted in a sectional view of a heart, in accordance with embodiments of the invention;

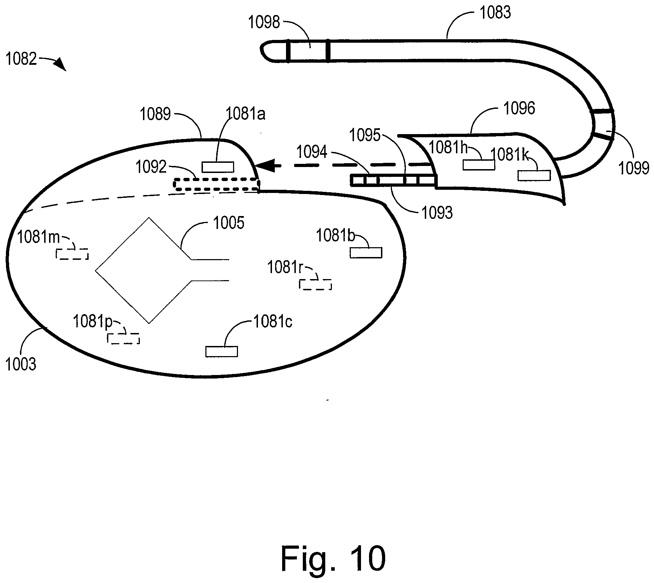

Figure 10 is a top view of an implantable cardiac device in accordance with the present invention, including an antenna electrode and a lead/header arrangement;

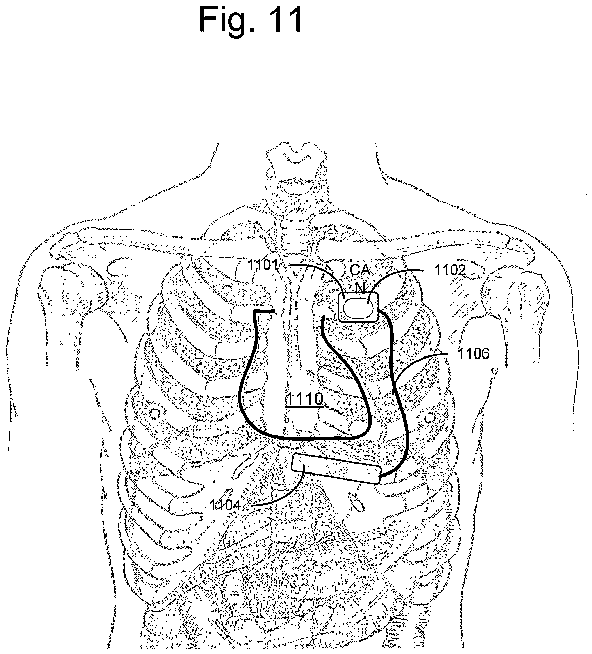

Figure 11 is a diagram illustrating components of a cardiac monitoring and/or stimulation device including an electrode array in accordance with an embodiment of the present invention;

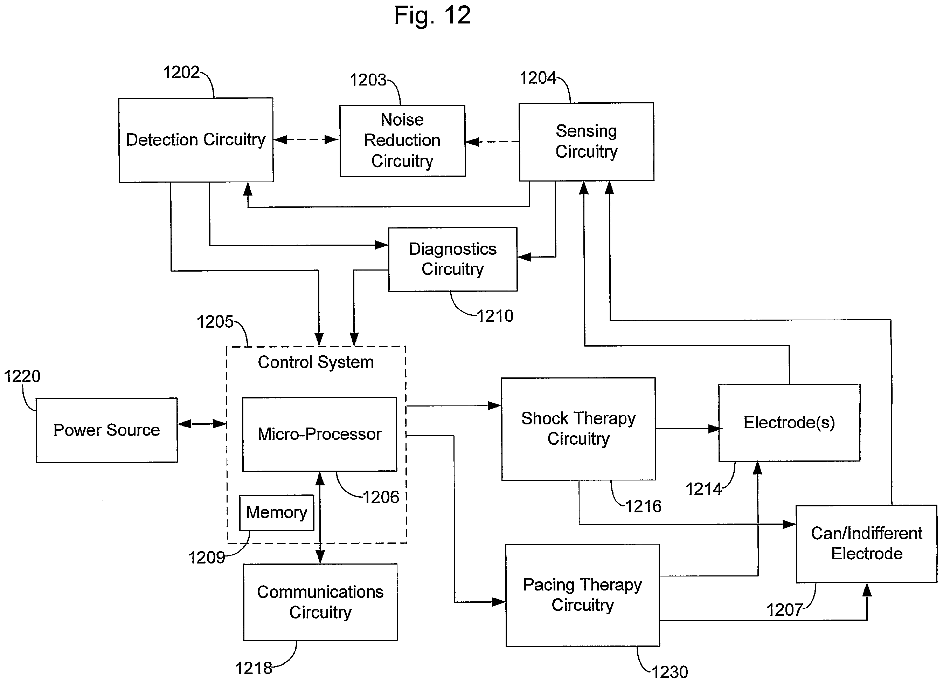

Figure 12 is a block diagram illustrating various components of a cardiac monitoring and/or stimulation device in accordance with an embodiment of the present invention;

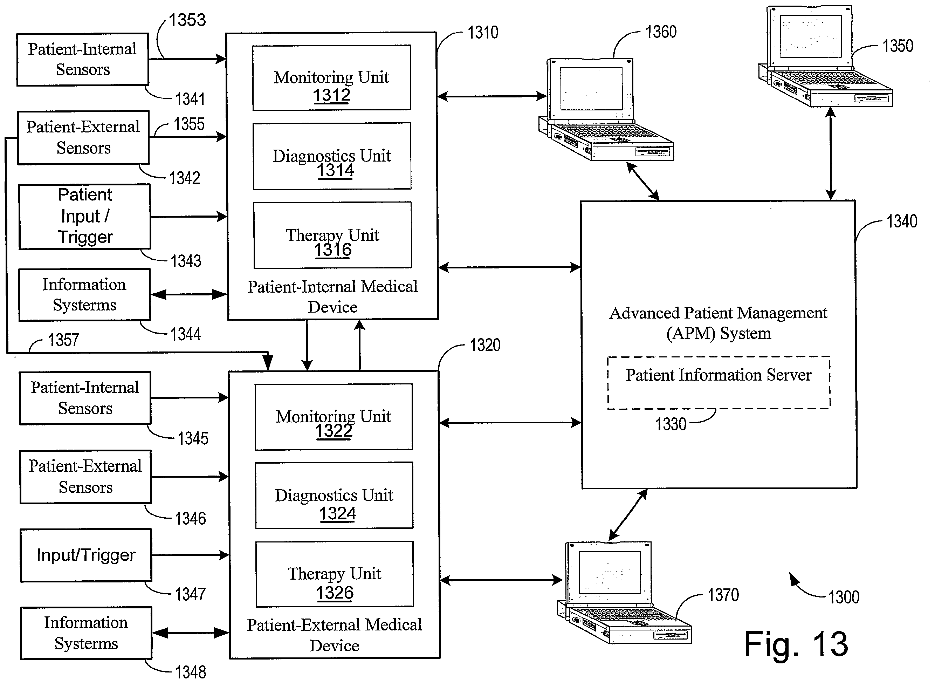

Figure 13 is a block diagram of a medical system that may be used to implement system updating, coordinated patient monitoring, diagnosis, and/or therapy in accordance with embodiments of the present invention;

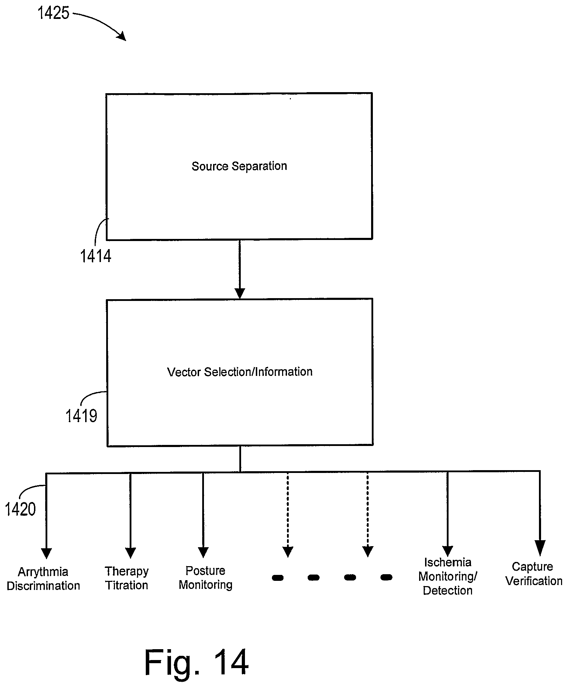

Figure 14 is a block diagram illustrating uses of cardiac activation sequence monitoring and/or tracking in accordance with the present invention;

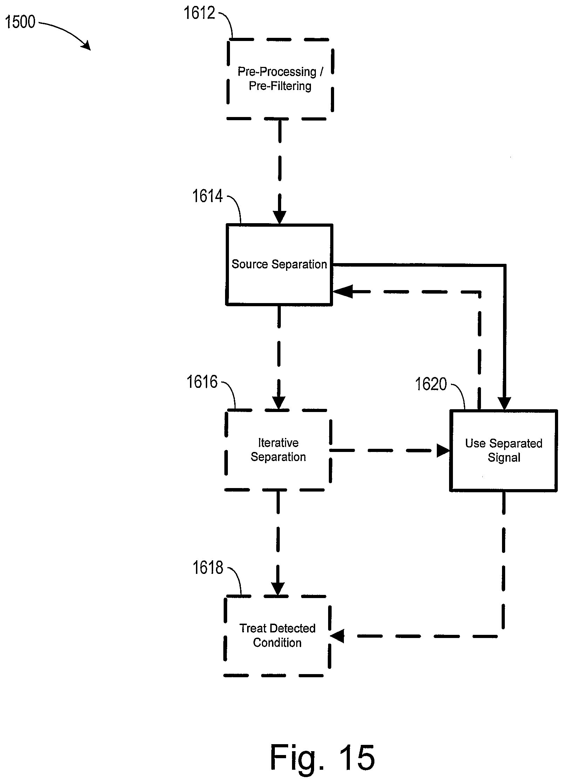

Figure 15 is a block diagram of a signal separation process in accordance with the present invention; and

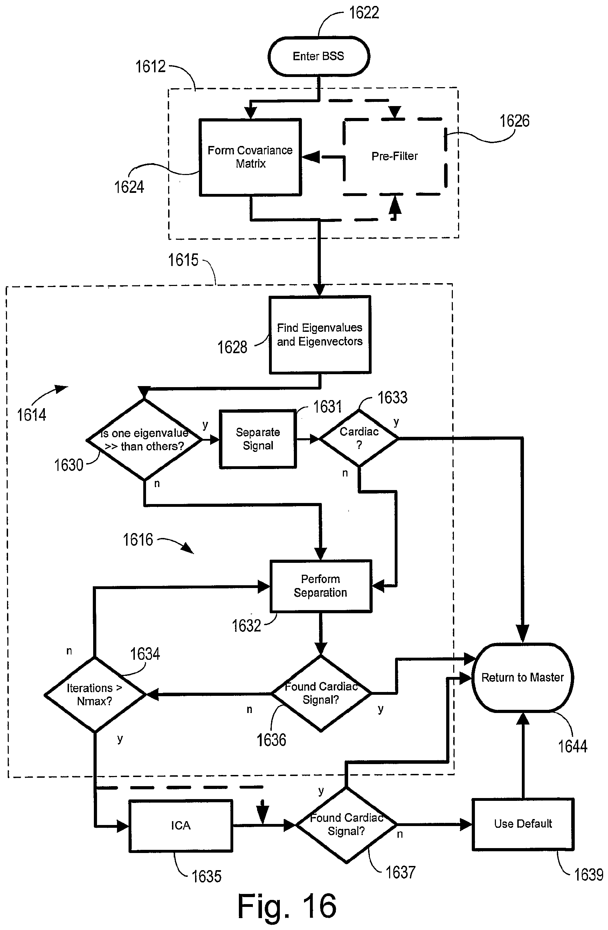

Figure 16 is an expanded block diagram of the process illustrated in Figure 15, illustrating an iterative independent component analysis in accordance with the present invention.

While the invention is amenable to various modifications and alternative forms, specifics thereof have been shown by way of example in the drawings and will be described in detail below. It is to be understood, however, that the intention is not to limit the invention to the particular embodiments described. On the contrary, the invention is intended to cover all modifications, equivalents, and alternatives falling within the scope of the invention as defined by the appended claims.

DETAILED DESCRIPTION OF VARIOUS EMBODIMENTS

In the following description of the illustrated embodiments, references are made to the accompanying drawings, which form a part hereof, and in which is shown by way of illustration, various embodiments in which the invention may be practiced. It is to be understood that other embodiments may be utilized, and structural and functional changes may be made without departing from the scope of the present invention.

An implanted device according to the present invention may include one or more of the features, structures, methods, or combinations thereof described hereinbelow. For example, a cardiac monitor or a cardiac stimulator may be implemented to include one or more of the advantageous features and/or processes described below. It is intended that such a monitor, stimulator, or other implanted or partially implanted device need not include all of the features described herein, but may be implemented to include selected features that provide for unique structures and/or functionality. Such a device may be implemented to provide a variety of therapeutic or diagnostic functions.

A wide variety of implantable cardiac monitoring and/or stimulation devices may be configured to implement a cardiac activation sequence monitoring and/or tracking methodology of the present invention. A non-limiting, representative list of such devices includes cardiac monitors, pacemakers, cardiovertors, defibrillators, resynchronizers, and other cardiac monitoring and therapy delivery devices. These devices may be configured with a variety of electrode arrangements, including transvenous, endocardial, and epicardial electrodes (i.e., intrathoracic electrodes), and/or subcutaneous, non-intrathoracic electrodes, including can, header, and indifferent electrodes, and subcutaneous array or lead electrodes (i.e., non-intrathoracic electrodes).

Embodiments of the present invention may be implemented in the context of a wide variety of cardiac devices, such as those listed above, and are referred to herein generally as patient-internal medical devices (PIMD) for convenience. A PIMD implemented in accordance with the present invention may incorporate one or more of the electrode types identified above and/or combinations thereof.

Cardiac activation sequence monitoring and/or tracking systems of the present invention employ more than two electrodes of varying location, and possibly of varying configuration. In one embodiment, for example, two or more electrodes may conveniently be located on the PIMD header, whereas the can of the PIMD itself may be the third electrode. In another embodiment, one electrode may be located on the PIMD header, another is the can electrode, and a third may be a PIMD antenna used for RF telemetry.

Electrocardiogram (ECG) signals originate from electrophysiological signals propagated through the heart muscle, which provide for the cardiac muscle contraction that pumps blood through the body. A sensed ECG signal is effectively a superposition of all the depolarizations occurring within the heart that are associated with cardiac contraction, along with noise components. The propagation of the depolarizations through the heart may be referred to as a depolarization wavefront. The sequence of depolarization wavefront propagation through the chambers of the heart, providing the sequential timing of the heart's pumping, is designated an activation sequence.

A signal separation algorithm may be implemented to separate activation sequence components of ECG signals, and produce one or more cardiac signal vectors associated with all or a portion of one or more cardiac activation sequences based on the separation. The activation sequence components may be considered as the signal sources that make up the ECG signals, and the signal separation process may be referred to as a source separation process or simply source separation. One illustrative signal source separation methodology useful for producing cardiac signal vectors associated with cardiac activation sequences is designated blind source separation, which will be described in further detail below.

In general, the quality of the electrocardiogram or electrogram sensed from one pair of electrodes of a PIMD depends on the orientation of the electrodes with respect to the depolarization wavefront produced by the heart. The signal sensed on an electrode bi-pole is the projection of the ECG vector in the direction of the bi-pole. Cardiac activation sequence monitoring and/or tracking algorithms of the present invention advantageously exploit the strong correlation of signals from a common origin (the heart) across spatially distributed electrodes.

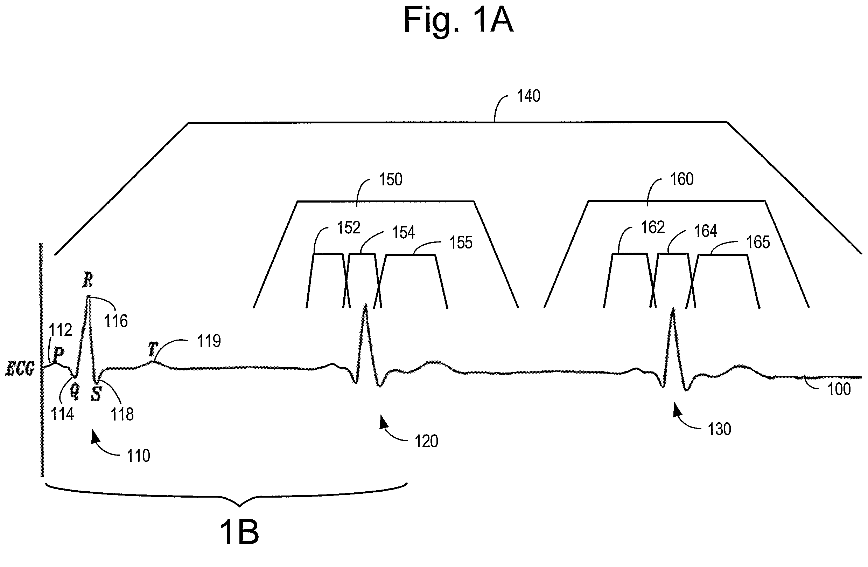

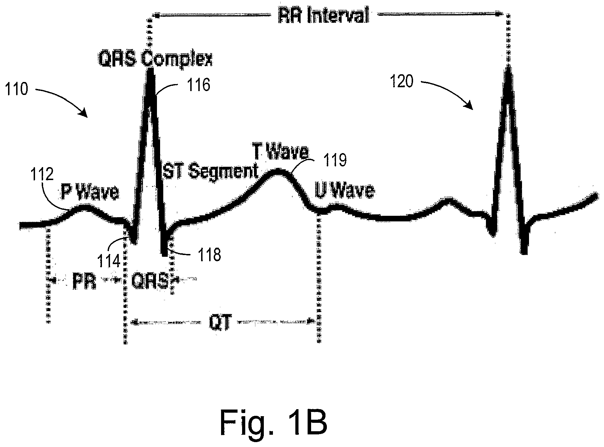

Referring to Figures 1A and 1B, an ECG waveform 100 describes the activation sequence of a patient's heart as recorded, for example, by a bi-polar cardiac sensing electrode. The graph of Figure 1A illustrates an example of the ECG waveform 100 for three heartbeats, denoted as a first heartbeat 110, a second heartbeat 120, and a third heartbeat 130. Figure 1B is a magnified view of the first two heartbeats 110, 120 of the ECG waveform identified by bracket 1B in Figure 1A.

Referring to the first heartbeat 110, the portion of the ECG waveform representing depolarization of the atrial muscle fibers is referred to as a P-wave 112. Depolarization of the ventricular muscle fibers is collectively represented by a Q 114, R 116, and S 118 waves of the ECG waveform 100, typically referred to as the QRS complex, which is a well-known morphologic feature of electrocardiograms. Finally, the portion of the waveform representing repolarization of the ventricular muscle fibers is known as a T wave 119. Between contractions, the ECG waveform returns to an isopotential level.

The sensed ECG waveform 100 illustrated in Figures 1A and 1B is typical of a farfield ECG signal, effectively a superposition of all the depolarizations occurring within the heart that result in contraction. The ECG waveform 100 may also be obtained indirectly, such as by using a signal separation methodology. Signal separation methodologies, such as blind source separation (BSS), are able to separate signals from individual sources that are mixed together into a composite signal. The main principle of signal separation works on the premise that spatially distributed electrodes collect components of a signal from a common origin (e.g., the heart) with the result that these components may be strongly correlated to each other. In addition, these components may also be weakly correlated to components of another origin (e.g., noise). A signal separation algorithm may be implemented to separate these components according to their sources and produce one or more cardiac signal vectors associated with all or a portion of one or more cardiac activation sequences based on the source separation.

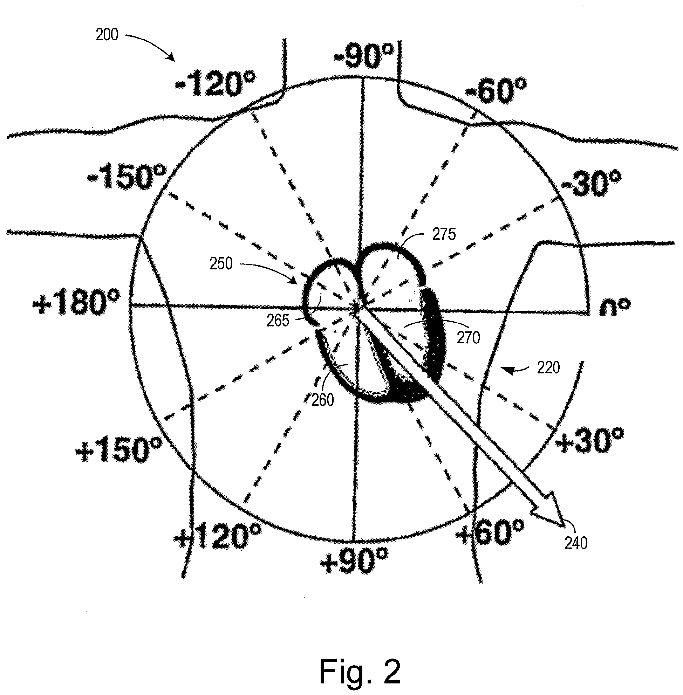

Figure 2 illustrates a convenient reference for describing cardiac signal vectors associated with a depolarization wavefront. Figure 2 is a polar plot 200 of a cardiac vector 240 superimposed over a frontal view of a thorax 220, with the origin of the polar plot located at a patient's heart 250, specifically, the atrioventricular (AV) node of the heart 250. The heart 250 is a four-chambered pump that is largely composed of a special type of striated muscle, called myocardium. Two major pumps operate in the heart, and they are a right ventricle 260, which pumps blood into pulmonary circulation, and a left ventricle 270, which pumps blood into the systemic circulation. Each of these pumps is connected to its associated atrium, called a right atrium 265 and a left atrium 275.

The cardiac vector 240 is describable as having an angle, in degrees, about a circle of the polar plot 200, and having a magnitude, illustrated as a distance from the origin of the tip of the cardiac vector 240. The polar plot 200 is divided into halves by a horizontal line indicating 0 degrees on the patient's left, and +/-180 degrees on the patient's right, and further divided into quadrants by a vertical line indicated by -90 degrees at the patient's head and +90 degrees on the bottom. The cardiac vector 240 is projectable onto the two-dimensional plane designated by the polar plot 200.

The cardiac vector 240 is a measure of all or a portion of the projection of a heart's activation sequence onto the polar plot 200. The heart possesses a specialized conduction system that ensures, under normal conditions, that the overall timing of ventricular and atrial pumping is optimal for producing cardiac output, the amount of blood pumped by the heart per minute. As described earlier, the normal pacemaker of the heart is a self-firing unit located in the right atrium called the sinoatrial node. The electrical depolarization generated by this structure activates contraction of the two atria. The depolarization wavefront then reaches the specialized conduction system using conducting pathways within and between the atria. The depolarization is conducted to the atrioventricular node, and transmitted down a rapid conduction system composed of the right and left bundle branches, to stimulate contraction of the two ventricles.

The normal pacemaker and rapid conduction system are influenced by intrinsic automatic activity and by the autonomic nervous system, which modulates heart rate and the speed with which electrical depolarizations are conducted through the specialized conduction system. There are many diseases that interfere with the specialized conduction system of the heart, and many result in abnormally fast, slow, or irregular heart rhythms.

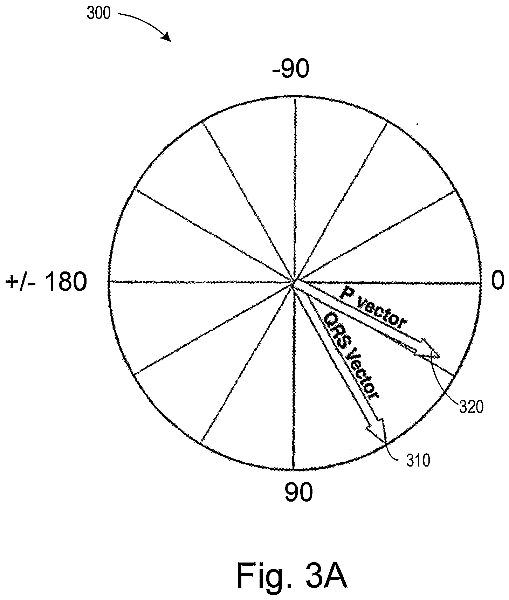

The cardiac vector 240 may be, for example, associated with the entire cardiac cycle, and describe the mean magnitude and mean angle of the cardiac cycle. Referring now to Figure 3A, a polar plot 300 is illustrated of separate portions of the cardiac cycle that may make up the cardiac vector 240 of Figure 2. As is illustrated in Figure 3A, a QRS vector 310 and a P vector 320 are illustrated having approximately 60 degree and 30 degree angles, respectively. The QRS vector 310 may also be referred to as the QRS axis, and changes in the direction of the QRS vector may be referred to as QRS axis deviations.

The QRS vector 310 represents the projection of the mean magnitude and angle of the depolarization wavefront during the QRS portion of the cardiac cycle onto the polar plot 300. The P vector 320 represents the projection of the mean magnitude and angle of the depolarization wavefront during the P portion of the cardiac cycle onto the polar plot 300. The projection of any portion of the depolarization wavefront may be represented as a vector on the polar plot 300.

Further, any number of cardiac cycles may be combined to provide a statistical sample that may be represented by a vector as a projection onto the polar plot 300. Likewise, portions of the cardiac cycle over multiple cardiac cycles may also be combined, such as combining a weighted summation of only the P portion of the cardiac cycle over multiple cardiac cycles, for example.

Referring now to Figures 1 through 3A, the first, second, and third cardiac cycles 110, 120, and 130 may be analyzed using a window 140 (Figure 1) applied concurrently to signals sensed by three or more cardiac sense electrodes. The ECG waveform signals 100 from all the sense electrodes, during the window 140, may be provided to a signal processor. The signal processor may then perform a source separation that provides the cardiac vector 240 (Figure 2). The cardiac vector 240 then represents the orientation and magnitude of the cardiac vector that is effectively an average over all three cardiac cycles 110, 120, and 130.

Other windows are also useful. For example, a window 150 and a window 160 may provide each full cardiac cycle, such as the cardiac cycle 120 and the cardiac cycle 130 illustrated in Figure 1, to a controller for analysis. The windows 150, 160 may be useful for beat-to-beat analysis, where the angle, magnitude, or other useful parameter from the separated cardiac vector 240 is compared between consecutive beats, or trended, for example.

Examples of other useful windows include a P-window 152, a QRS window 154, and an ST window 155 (Figure 1) that provide within-beat vector analysis capability, such as by providing the P-vector 320 and the QRS-vector 310 illustrated in Figure 3A. Providing a P-window 162 and/or a QRS-window 164, and/or an ST window 165 to subsequent beats, such as to the consecutive cardiac cycle 130 illustrated in Figure 1, provides for subsequent separations that may provide information for tracking and monitoring changes and/or trends of windowed portions of the cardiac cycle or statistical samples of P, QRS, or T waves over more than 1 beat.

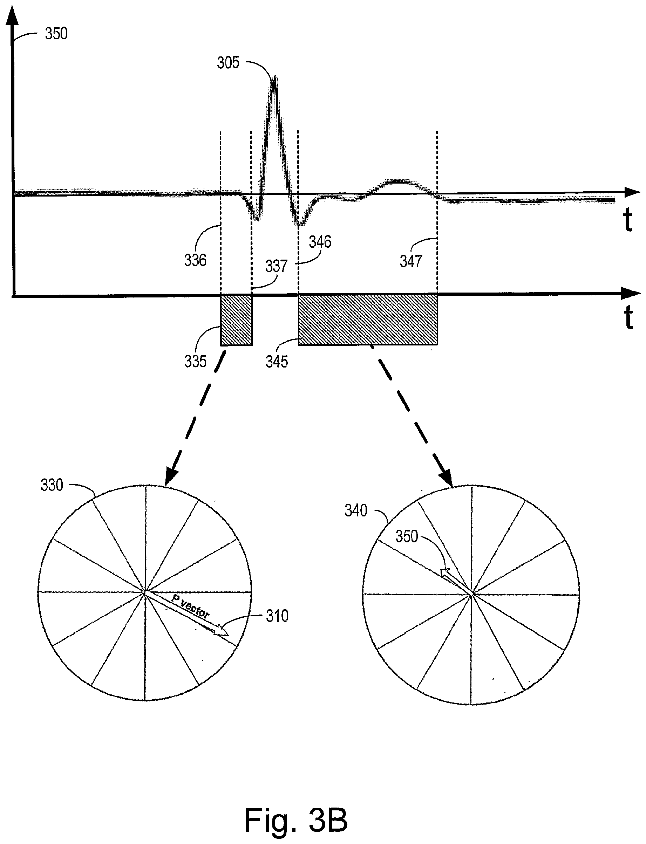

Referring now to Figure 3B, polar plots of cardiac vectors obtained from selected portions of an electrocardiogram are illustrated. In general, it may be desirable to define one or more detection windows associated with particular segments of a given patient's cardiac cycle. The detection windows may be associated with cardiac signal features, such as P, QRS, ST, and T wave features, for example. The detection windows may also be associated with other portions of the cardiac cycle that change in character as a result of changes in the pathology of a patient's heart. Such detection windows may be defined as fixed or triggerable windows.

Detection windows may include unit step functions to initiate and terminate the window, or may be tapered or otherwise initiate and terminate using smoothing functions such as Bartlett, Bessel, Butterworth, Hanning, Hamming, Chebyshev, Welch, or other functions and/or filters. The detection windows associated with particular cardiac signal features or segments may have widths sufficient to sense cardiac vectors resulting from normal or expected cardiac activity. Aberrant or unexpected cardiac activity may result in the failure of a given cardiac vector to fall within a range indicative of normal cardiac behavior. Detection of a given cardiac vector beyond a normal range may trigger one or more operations, including increased monitoring or diagnostic operations, therapy delivery, patient or physician alerting, communication of warning and/or device/physiological data to an external system (e.g., advanced patient management system) or other responsive operation.

An ECG signal 305 is plotted in Figure 3B as a signal amplitude 350 on the ordinate versus time on the abscissa. One cardiac cycle is illustrated. The P portion of the ECG signal 305 may be defined using a P-window 335 that opens at a time 336 and closes at a time 337. A source separation performed on the ECG signal 305 within the P-window 335 produces the P vector 310 illustrated on a polar plot 330. The angle of the P vector 310 indicates the angle of the vector summation of the depolarization wavefront during the time of the P-window 335 for the ECG signal 305.

The ST portion of the ECG signal 305 may be defined using an ST-window 345 that opens at a time 346 and closes at a time 347. A source separation performed on the ECG signal 305 within the ST-window 345 produces the ST vector 350 illustrated on a polar plot 340. The angle of the ST vector 350 indicates the angle of the vector summation of the depolarization wavefront during the time of the ST-window 345 for the ECG signal 305.

The P vector 310 and the ST vector 350 may be acquired as baselines, for future comparisons. If baselines for the P vector 310 and the ST vector 350 are already established, the P vector 310 and ST vector 350 maybe compared relative to their baselines for monitoring and tracking purposes. As indicated above, detection of P vector 310 or ST vector 350 beyond a predetermined range may trigger one or more responsive operations.

Cardiac activation sequence monitoring and tracking, to monitor changes and/or trends as described above, may be useful to determine initial activation sequences, and track acute and chronic changes in the activation sequences. Information from the patient's activation sequence is valuable for identification, discrimination, and trending of conditions such as conduction anomalies (e.g. AV block, bundle branch block, retrograde conduction) and cardiac arrhythmias (e.g. discriminating between supraventricular tachycardia versus ventricular tachycardia, reentrant supraventricular tachycardia versus atrial fibrillation, or other desirable discrimination). In addition to baseline establishment, monitoring, and tracking, activation sequence information may also be useful for determining pace capture for autocapture/autothreshold algorithms, adjustment, optimization, or initiation of cardiac resynchronization therapy, and optimization or initiation of anti-arrhythmia therapies, for example.

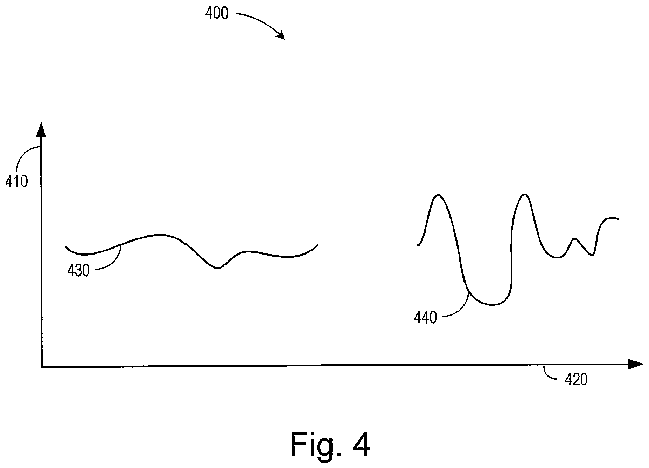

Figure 4 illustrates another convenient reference for describing cardiac signal vectors associated with a depolarization wavefront. Figure 4 is a graph 400 of temporal profiles of a measure of a cardiac vector useful for diagnosing diseases and anomalous conditions in accordance with the present invention. The graph 400 contains a first temporal profile 430 of a cardiac vector, and a second temporal profile 440 of the same cardiac vector after a change has occurred. An abscissa 420 of the graph 400 is time related, and an ordinate 410 of the graph 400 is related to a measure of the cardiac vector.

The ordinate 410 may be, for example, the angle of the cardiac vector. A non-limiting, non-exhaustive list of measures of a vector useful for the ordinate 410 includes: angle; magnitude; variance; power spectral density; rate of change of angle; rate of change of magnitude; rate of change of variance; or other measure indicative of a change in the cardiac activation sequence. As an example, consider the angle of the P vector 320 illustrated in Figure 3A. In this example, the ordinate 410 would be indicated in degrees, with the first temporal profile 430 varying from around 30 degrees. The abscissa 420 may be time, designated in cardiac cycles, with a measure made of the P vector 320 for every cardiac cycle. The angle of the P vector 320 may be plotted on the graph 400 at any interval of cardiac cycles, thereby displaying variance and trends in the angle of the P vector 320 over many cardiac cycles.

After some change occurs, such as a pathological change in the patient's heart, the second temporal profile 440 may be plotted using cardiac cycles occurring after the change. As is evident in the second temporal profile 440 versus the first temporal profile 430, the variance of the second temporal profile 440 is significantly larger than the variance of the first temporal profile 430. Changes such as this may be detected and used to diagnose, verify and/or monitor diseases and/or cardiac conditions in accordance with the present invention.

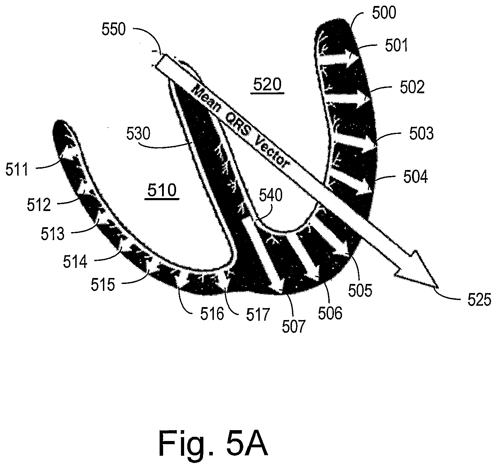

Figures 5A through 5D illustrate cardiac vectors superimposed over a sectional view of the ventricles of a patient's heart 500. Referring to Figure 5A, the ventricular portion of a patient's heart is illustrated having a right ventricle 510 and a left ventricle 520 separated by the heart's septum. The specialized conduction system includes an atrioventricular node 550, which is used as the origin for cardiac vectors, such as a mean QRS vector 525.

A right bundle branch 530 conducts the depolarization wavefront from the atrioventricular node 550 to the wall of the right ventricle 510. Illustrated in the wall of the right ventricle 510 are a series of vectors 511-517, indicating the magnitude and angle of a local portion of the depolarization wavefront as it travels along the right ventricle 510.

A left bundle branch 540 conducts the depolarization wavefront from the atrioventricular node 550 to the wall of the left ventricle 520. Illustrated in the wall of the left ventricle 510 are a series of vectors 501-507, indicating the magnitude and angle of a local portion of the depolarization wavefront as it travels along the left ventricle 520.

The mean QRS vector 525 is the vector summation of the vectors 511-517 and the vectors 501-507. The mean QRS vector 525 may be typical of a healthy heart, here illustrated at about 40 degrees angle if using the polar plot of Figure 3A. The mean QRS vector 525 varies from patient to patient depending on, for example, patient posture, and normal anatomical variation.

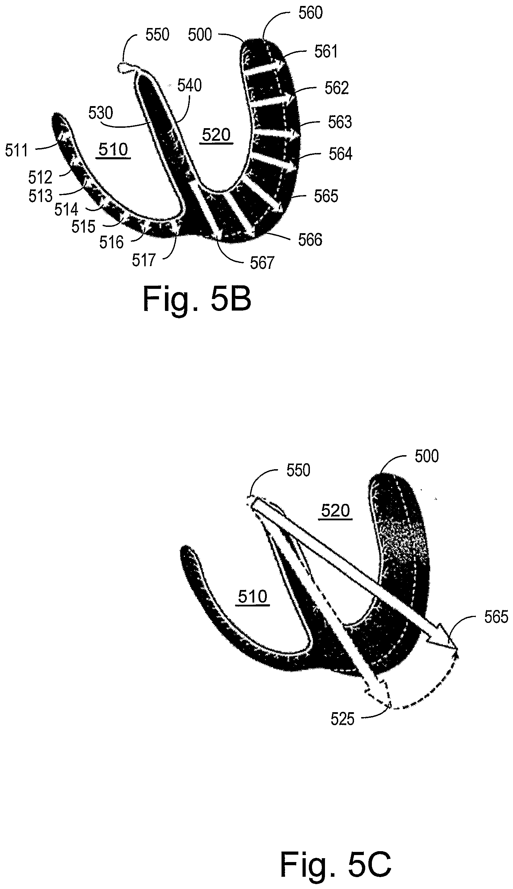

Referring now to Figure 5B, the wall of the left ventricle 520 is enlarged, or hypertrophied, relative to Figure 5A. In Figure 5B, a dotted line 560 represents the wall of the left ventricle 520 in Figure 5A, before hypertrophy. A series of local vectors 561-567 illustrate the larger local contribution to the mean QRS vector 525 from the hypertrophy related vectors 561-567 relative to the normal series of left ventricle vectors 501-507.

Figure 5C illustrates how the mean QRS vector 525 from a normal heart may change to a mean hypertrophied QRS vector 565 after hypertrophy has occurred. For example, a PIMD may be implanted in a patient, and an initial analysis provides a baseline mean QRS vector 525 for the patient, indicative of a normal condition of the left ventricle 520. After a period of time, the patient's heart may be subject to hypertrophy. An analysis performed post-hypertrophy may result in finding the mean hypertrophied QRS vector 565. This change may be used to diagnose, verify and/or monitor hypertrophy of the patient's left ventricle.

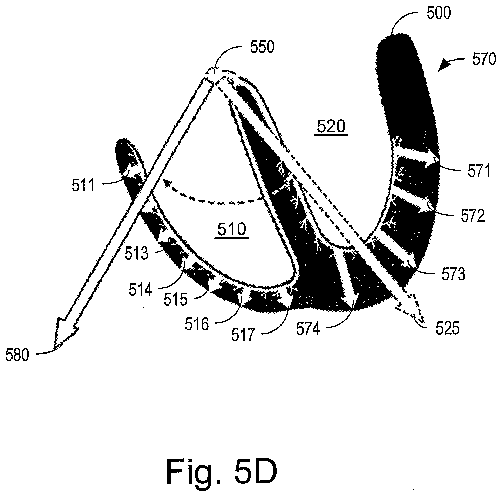

Another example of a pathological change that may be diagnosed and/or verified using embodiments of the present invention is a lessening or loss of blood supply to a portion of the heart, such as through a transient ischemia or myocardial infarction. The sectional view in Figure 5D illustrates the left ventricle 520 having an infarcted portion 570 of the ventricular wall. As is evident in the infarcted portion 570, no depolarization is occurring, so only local depolarization vectors 571-574 contribute to the mean cardiac vector from the left ventricle 520. The infarction results in a change, for example, of the detected mean QRS vector 525 to an infarcted mean QRS vector 580. Other vectors such as the ST vector may also show the change. This change is evident as the angle of the cardiac vector moves from the second quadrant before infarction, to the third quadrant after infarction.

A PIMD that detects a change such as is illustrated in Figure 5D has the potential to alert the patient and/or physician to a loss or lessening of blood supply to a portion of the heart muscle before permanent damage occurs. Early detection may result in greatly reduced morbidity from these kinds of events.

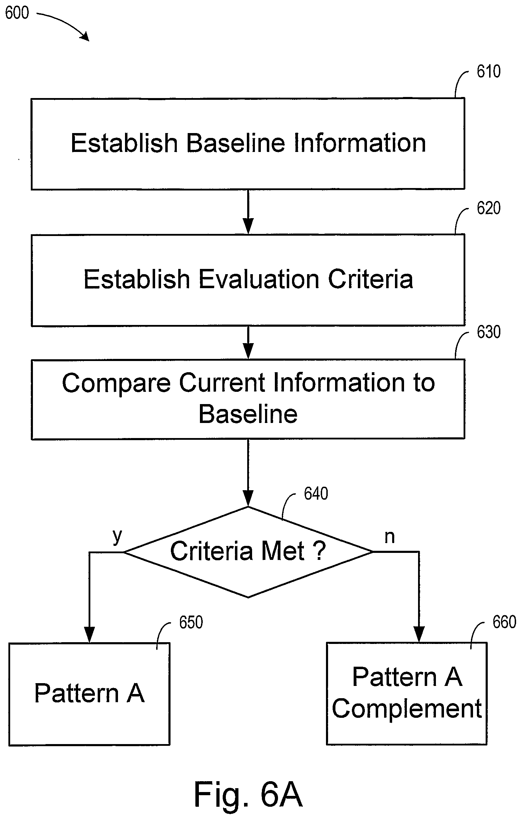

Figure 6A is a block diagram of a method 600 of detecting a change in one or more cardiac signal vectors associated with all or a portion of one or more cardiac activation sequences based on a source separation in accordance with the present invention. A baseline is established 610, providing information that may be monitored or tracked relative to a patient's electrophysiological signals. The baseline 610 may be established from an initial source separation, that provides initial cardiac signal information as a baseline. Alternately, or additionally, the baseline 610 may be established by a PIMD manufacturer from clinical data, or a patient's baseline 610 may be established by a clinician before, during, or after a PIMD implant procedure. The baseline 610 may be established as a rolling average of recent patient information from prior source separations, for example.

Evaluation criteria is established 620 to provide an index for comparison to the baseline 610. For example, the evaluation criteria 620 may be any parameter or characteristic determinable or measurable from the patient's electrophysiology information. A non-exhaustive, non-limiting list of evaluation criteria 620 includes: an angle change of one or more cardiac signal vectors; a magnitude change of one or more cardiac signal vectors; a variance change of one or more cardiac signal vectors; a power spectral density change of the angle of one or more cardiac signal vectors; a power spectral density change of the magnitude of one or more cardiac signal vectors; a trajectory change of one or more cardiac signal vectors; a temporal profile change of one or more cardiac signal vectors; a rate of change of angle of one or more cardiac signal vectors; a rate of change of magnitude of one or more cardiac signal vectors; a rate of change of variance of one or more cardiac signal vectors; a rate of change of temporal profile of one or more cardiac signal vectors; a trend of the angle of one or more cardiac signal vectors; a trend of the magnitude of one or more cardiac signal vectors; a trend of the variance of one or more cardiac signal vectors; and a trend of the temporal profile of one or more cardiac signal vectors.

For example, an initial source separation may be performed by a PIMD on a patient post-implant. The separation may produce the baseline 610 of the patient's average full cardiac cycle, such as the cardiac vector 240 illustrated in Figure 2. The vector 240 may have a characteristic, such as the angle, determined as +45 degrees. The evaluation criteria 620 may be, for example, that the patient's average full cardiac cycle vector's angle should be within +40 to +50 degrees.

A comparison 630 is performed to determine the latest patient information relative to the baseline 610. For example, the results of a latest source separation algorithm may provide the latest average full cardiac cycle vector's angle for the patient. Continuing with the above example, the comparison 630 may check the latest angle of the patient's average full cardiac cycle vector's angle against the +40 to +50 degree criteria.

A decision 640 selects an outcome based on the comparison 630. If the criteria is met, for example if the latest angle is within +40 to +50 degrees as outlined above, then a pattern A 650 is considered to be the patient's latest condition. For example, the pattern A 650 may be defined as an insufficient change to require some sort of action by the PIMD. If the criteria 620 is not met at decision 640, then a pattern A complement 660 condition is considered to be the patient's latest condition. The pattern A complement 660 condition may be defined as requiring some sort of action by the PIMD, such as reporting the condition, further evaluating the patient's cardiac rhythms, preparing a defibrillator for a shock, or other desired action.

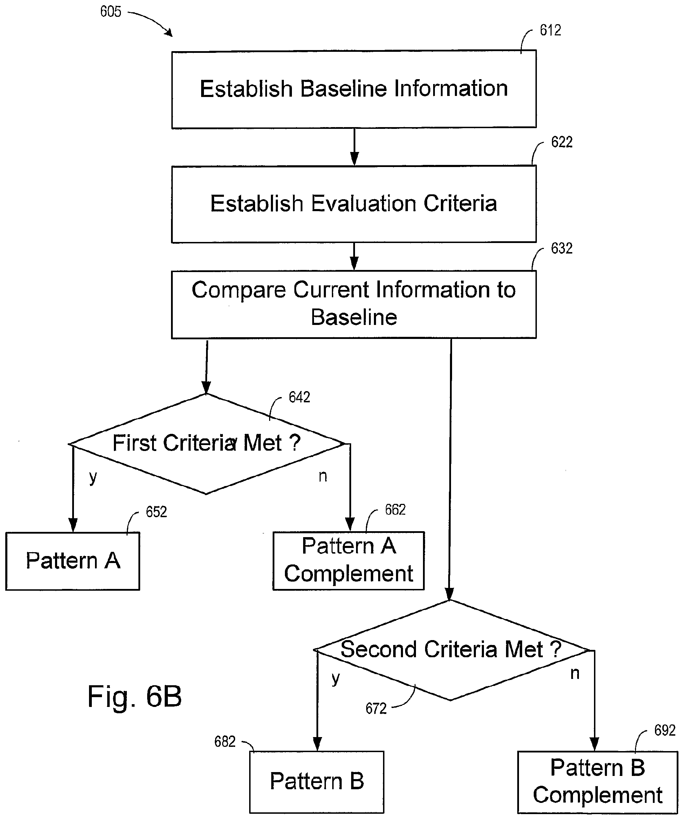

Figure 6B is a block diagram of another embodiment of a method 605 of detecting a change in one or more cardiac signal vectors associated with all or a portion of one or more cardiac activation sequences, when the criteria for baseline shift includes two criteria. It is contemplated that any number of criteria may be used or combined in accordance with the present invention. The use of two criteria with reference to Figure 6B is for purposes of explanation as to how to extend methods of the present invention to multiple criteria, and is not intended as a limiting example.

A baseline is established 612, providing information that may be monitored or tracked from a patient's electrophysiological signals. The baseline 612 may be established from an initial source separation, that provides initial cardiac signal information as a baseline. Alternately, or additionally, the baseline 612 may be established by a PIMD manufacturer from clinical data, or a patient's baseline 612 may be established by a clinician before, during, or after a PIMD implant procedure. The baseline 612 may be established as a rolling average of recent patient information from prior source separations, for example.

Evaluation criteria are established 622 to provide indices for comparison to the baseline 612. For example, the evaluation criteria 622 may be any parameters or characteristics determinable or measurable from the patient's electrophysiology information. A non-exhaustive, non-limiting list of evaluation criteria 622 includes those described previously with respect to Figure 6A. It is further contemplated that a single criterion may be compared with respect to multiple baselines, and/or that multiple criteria may each be compared with respect to their own unique baseline established for each particular criterion.

Baselines may be pre-defmed using, for example, clinical data, and/or baselines may be established using initial source separations. For example, and described in more detail below, a source separation may provide an orthogonal coordinate system, with vectors described using a series of coefficients matched to a series of unit direction vectors. One or more angles may be calculated using trigonometric identities to indicate a vector's direction relative to other vectors in the coordinate system. Subsequent source separations provide revised sets of coefficients, from which changes in vector direction may be determined using the same trigonometric identities. In an n-dimensional space, (n-1) angles may be resolved and used for comparison and tracking in accordance with the present invention.

For example, an initial source separation may be performed by a PIMD on a patient post-implant. The separation may produce the baseline 612 of the patient's cardiac cycle, such as the QRS-vector 310 and the P-vector 320 illustrated in Figure 3A. The QRS-vector 310 may have the angle determined as +45 degrees. The P-vector 320 may have the angle determined as +28 degrees. The evaluation criteria 622 may be, for example, that the patient's QRS-vector's angle should be within +40 to +50 degrees and that the patient's P-vector angle should be within +25 to +30 degrees.

A comparison 632 is performed to determine the latest patient information relative to the baseline 612. For example, the results of a latest source separation algorithm may provide the latest angles of the QRS-vector and P-vector for the patient. Continuing with the above example, the comparison 632 may check the latest angles of the patient's QRS-vector and P-vector against the +40 to +50 degree and +25 to +30 degree criteria respectively.

A first decision 642 selects a first outcome based on the comparison 632. If the first criteria is met, for example if the latest angle of the QRS-vector is within +40 to +50 degrees as outlined above, then a pattern A 652 is considered to be the patient's latest condition. For example, the pattern A 652 may be defined as an insufficient change to require some sort of action by the PIMD. If the criteria 622 is not met at decision 642, then a pattern A complement 662 condition is considered to be the patient's latest condition. The pattern A complement 662 condition may be defined as requiring some sort of action by the PIMD, such as reporting the condition, further evaluating the patient's cardiac rhythms, preparing a defibrillator for a shock, or other desired action.

A second criteria decision 672 is performed to check for a second outcome based on the second criteria. If the second criteria is met, for example if the latest angle of the P-vector is within +25 to +30 degrees as outlined above, then a pattern B 682 is considered to be the patient's latest condition. For example, the pattern B 682 may be defined as an insufficient change to require some sort of second action by the PIMD. If the criteria 622 is not met at decision 672, then a pattern B complement 692 condition is considered to be the patient's latest condition. The pattern B complement 692 condition may be defined as requiring some sort of second action by the PIMD.

Table 1 below provides a non-limiting non-exhaustive list of conditions that may be detected by monitoring and/or tracking cardiac activation sequences in accordance with the present invention.

| Table 1. Conditions associated with QRS Axis Deviations |
| --- |
| |
| **First Source (Normal -30 to +90 degrees)** |
| |
| **Left Axis Deviation (LAD): ≥ -30°** |
| Left Anterior Fascicular Block (LAFB) axis -45° to -90° |
| Some cases of inferior myocardial infarction with QR complex |
| Inferior Myocardial Infarction + LAFB in same patient (QS or QRS complex) |
| Some cases of left ventricular hypertrophy |
| Some cases of left bundle branch block |
| Ostium primum Atrial Septal Defect and other endocardial cushion defects |
| Some cases of Wolff-Parkins on-White syndrome syndrome (large negative *delta* wave) |
| |

(continued)

| |
|---|
| **Right Axis Deviation (RAD):** $\geq$ **+90°** |
| Left Posterior Fascicular Block (LPFB): |
| Many causes of right heart overload and pulmonary hypertension |
| High lateral wall Myocardial Infarction with QR or QS complex |
| Some cases of right bundle branch block |
| Some cases of Wolff-Parkins on-White syndrome syndrome |
| Children, teenagers, and some young adults |
| **Bizarre QRS axis: +150° to -90°** |
| Dextrocardia |
| Some cases of complex congenital heart disease (e.g., transposition) |
| Some cases of ventricular tachycardia |
| |
| **Second Source** |
| |
| **QRS Axis Deviation** |
| Left anterior fascicular block (LAFB) |
| Right ventricular hypertrophy |
| Left bundle branch block Acute Myocardial Infarction: |
| Hypertensive heart disease |
| Coronary artery disease |
| Idiopathic conducting system disease |
| Acute Myocardial Infarction - inferior left ventricular free wall accessory pathway (Wolff-Parkinson-White syndrome) |
| Posteroseptal accessory pathway |
| left posterior fascicular block |
| Chronic Obstructive Pulmonary Disease (uncommon - 10%) |
| Other conduction defects: |
| left ventricular hypertrophy |
| Right bundle branch block |
| Elevated diaphragm: R anterior hemiblock |
| Pregnancy |
| Pacing of R ventricle |
| Abdominal mass |
| Pulmonary conditions |
| Ascites |
| Pulmonary hypertension |
| Tumor |
| Chronic Obstructive Pulmonary Disease |
| Conduction defects: Emphysema/bronchitis |
| R ventricular (apical) pacing |

(continued)

| Second Source |
|---|
| |
| **QRS Axis Deviation** |
| Pulmonary emboli/infarcts |
| Systemic hypertension, esp. chronic |
| Congenital defects |
| Valvular lesions |
| Rheumatic heart disease |
| Pulmonic stenosis |
| Aortic regurgitation |
| Mitral regurgitation |
| Mitral stenosis |
| Coarctation of the aorta |
| Tricuspid regurgitation |
| Hyperkalemia |
| Pulmonic stenosis |
| Normal variant in obese and in elderly |
| Pulmonic regurgitation |

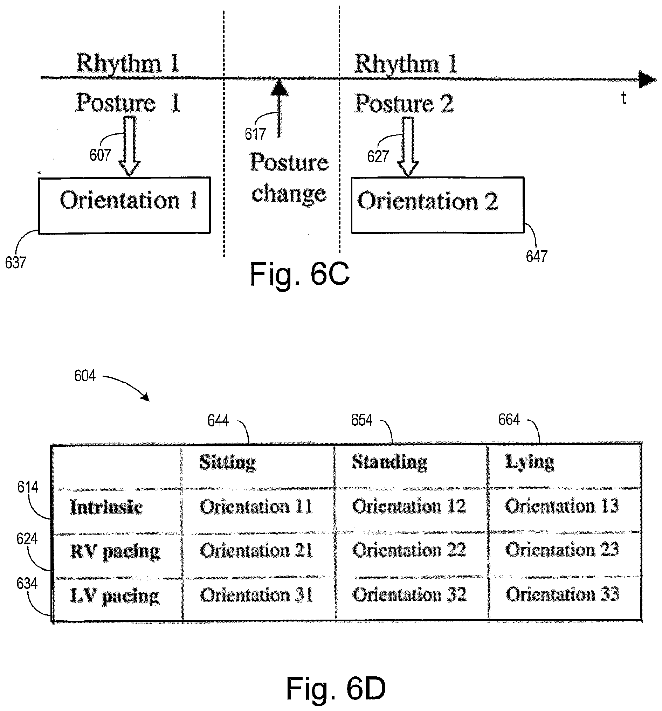

Figure 6C is a graph illustrating the change in vector orientation due to a posture change in accordance with embodiments of the present invention. Posture changes lead to changes in the heart's position in the chest, thus producing changes in the dominant orientation that is computed from multiple subcutaneous ECGs when monitoring and/or tracking cardiac activation sequence information. Figure 6C illustrates the relation between posture and dominant orientation when a posture change occurs during a sustained cardiac rhythm. A first posture 607 produces a first orientation 637 of the dominant activation sequence vector. A posture change 617 occurs during sustained cardiac rhythm, such that any detected changes are a result of the posture change 617, and not due to a change in the cardiac activation sequence. A second posture 627 produces a second orientation 647 of the cardiac sequence vector. The difference between the first orientation 637 and the second orientation 647 provides an indication of the change in the patient's posture.

Figure 6D is a table 604 of vector orientations possible based on combinations of posture and intrinsic or paced electrophysiology signals in accordance with embodiments of the present invention. Table 604 is useful for determining a patient's posture for patients that do not have a predominant rhythm, such as patients with a 50% intrinsic and 50 % paced rhythm. For patients without a readily identifiable predominant rhythm, a table such as the table 604 illustrated in Figure 6D may be established. The table 604 includes a row 614 associated with intrinsic cardiac activity, a row 624 associated with right ventricular paced cardiac activity, and a row 634 associated with left ventricular paced cardiac activity. The table 604 further includes columns 644, 654, and 664 associated with sitting, standing, and lying postures respectively. It is understood that the number of columns and rows illustrated in Figure 6D is for illustrative purposes only, and that any number of rows and/or columns may be established. For example, columns may be established to expand the lying condition into lying on front, back, right side, left side, or the like. Further, rows may be established for any combination of possible intrinsic and paced cardiac activity. Orientation templates may be established for a patient, such as are illustrated in the table 604. For example, if the patient is experiencing intrinsic cardiac activity in a lying position, an orientation 13 may be the dominant orientation for an activation sequence vector. Likewise, if the patient is sitting during a period of left ventricular pacing, an orientation 31 is the dominant orientation for an activation sequence vector. The intersection of any row and column in the table 604 provides for orientation templates indicative of the patient's posture.

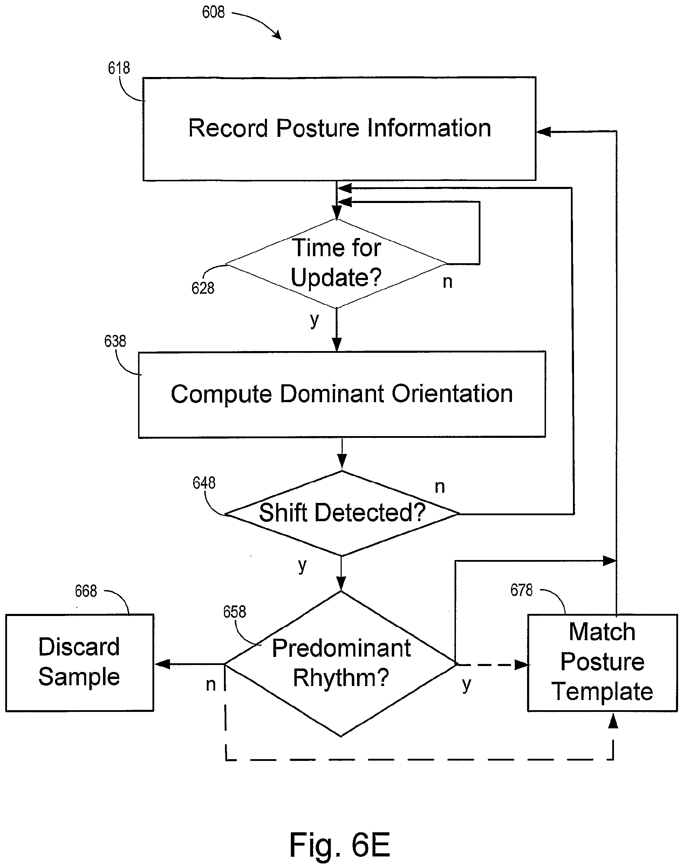

Figure 6E is a flow chart of an embodiment of methods for recording and storing posture information in accordance with the present invention. For example, posture templates, such as those illustrated in Table 604 illustrated in Figure 6D, may be established clinically at the time of implantation of a PIMD device, or during PIMD updates by a clinician. A method 608 may be performed continuously, or as otherwise desired, to determine and/or monitor a patient's posture. The method 608 includes a block 618 for storing posture information. For example, values of one or more cardiac activation sequence vector characteristics may be stored for future reference. Additionally or optionally, templates may be recorded for future matching, and/or current posture determinations may be recorded at block 618. A decision 628 is made to determine if a posture update is desired. Posture information may be recorded upon detection of a cardiac event, at regular intervals, continuously, or as otherwise desired. If an update is desired, the dominant orientation of a cardiac activation sequence may be determined at block 638. If a shift is detected at a decision 648, and a determination 658 is made to see if the patient in their predominant rhythm. If the patient is experiencing predominant cardiac rhythm, information from the dominant orientation computation 638 may be recorded at block 618, and/or optionally a match 678 may be performed to posture templates, such as those described with respect to Figure 6D. If the patient is not in a predominant rhythm, then the current sample of the dominant orientation from the block 638 may be discarded at a block 668, or the information from the dominant orientation calculation of block 638 may be used to compare to the templates 678, to determine posture information.

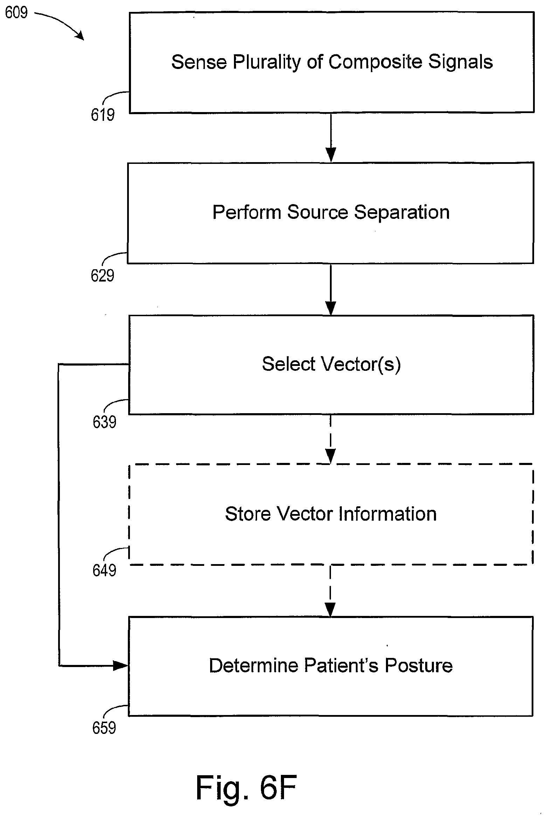

Figure 6F is a flow chart of an embodiment of a method 609 for determining a patient's posture in accordance with the present invention. A plurality of composite signals 619 are sensed concurrently using a plurality of subcutaneous non-intrathoracic cardiac electrodes. A source separation 629 is performed using the sensed plurality of composite signals 619. One or more cardiac signal vectors 639 are selected that are associated with all or a portion of one or more cardiac activation sequences, based on the source separation. If posture monitoring is desired, vector information 649 may be optionally stored for tracking purposes. A change in a patient's posture 659 may be detected using the one or more cardiac signal vectors.

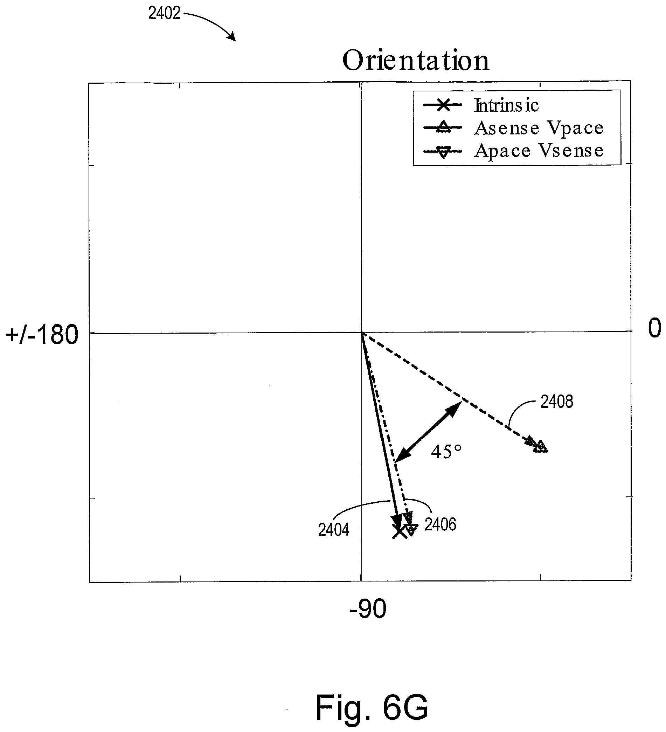

Referring now to Figure 6G, a graph 2402 illustrates vector angles for cardiac activation sequence orientations of intrinsic, ventricle originated, and atrium originated orientations demonstrates the significant departure of ventricle originated activation sequences from atrium originated activation sequences. The graph 2402 follows the same axes conventions as Figures 2 and 3A.

An intrinsic atrium-originated activation sequence vector 2404 and an atrial paced/ventricular sensed activation sequence vector 2406 both lie approximately 45 degrees from an atrial sensed/ventricular paced activation sequence vector 2408. The intrinsic atrium-originated activation sequence vector 2404 and the atrial paced/ventricular sensed activation sequence vector 2406 lie at approximately 80 degrees in the graph 2402. The atrial sensed/ventricular paced activation sequence vector 2408 lies at approximately 35 degrees in the graph 2402. This difference is readily detected using activation sequence monitoring and tracking.

Monitoring and tracking of cardiac activation sequences provides information about the progression of the depolarization wavefront through the heart, and therefore information about synchronization between the chambers of the heart. Vectors corresponding to the orientation of the depolarization wavefront, such as the vectors 2404, 2406, and 2408 in Figure 6G, may be used to determine cardiac asynchrony. Cardiac asynchrony information is useful for titrating CRT therapies, and maximizing hemodynamics. Closed loop control methods of the present invention are useful for updating CRT therapy in a patient, ultimately improving the patient's quality of life.

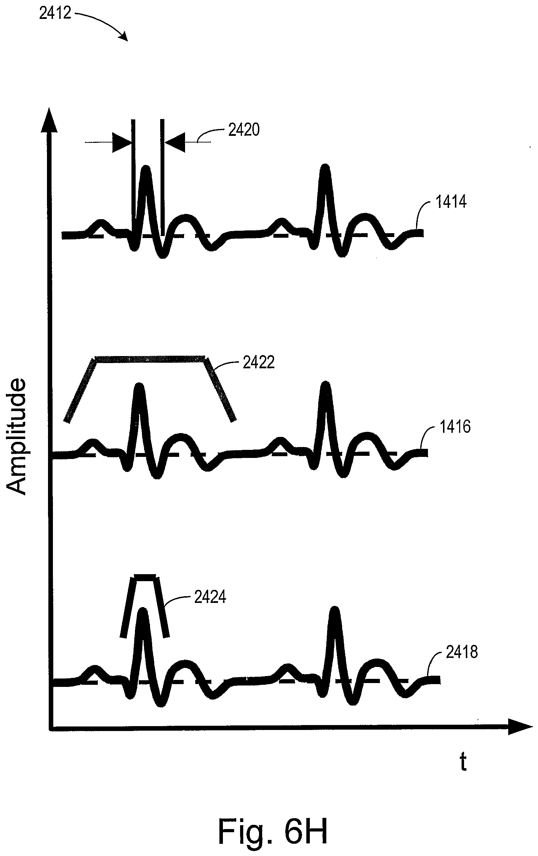

Figure 6H is a graph 2412 of asynchrony measurements in accordance with the present invention. The graph 2412 has time as its abscissa and signal amplitude as its ordinate. A first trace 2414, a second trace 2416, and a third trace 2418 illustrate cardiac signals such as electrocardiogram signals sensed from electrodes of a PIMD. The traces 2414, 2416, and 2418 may be used to measure asynchrony in accordance with the present invention. The first trace 2414 is illustrated with a QRS complex width 2420 used as a measure of asynchrony. As asynchrony increases, the QRS complex width 2420 increases. Measurement of the width of the QRS complex width 2420 may be done, for example, using morphological feature analysis.

The second trace 2416 illustrates a cardiac cycle window 2422 used to determine a dominant orientation for the vector associated with the whole cardiac cycle. The portion of the electrocardiogram signal of the trace 2416 that lies within the cardiac cycle window 2422 may be evaluated using cardiac activation sequence monitoring in accordance with the present invention. Changes in one or more characteristics of one or more vectors associated with the cardiac activation sequence within the cardiac cycle window 2422 may be used to determine levels of cardiac asynchrony. For example, a baseline may be established for the angle of the dominant vector orientation for the cardiac signal in the cardiac cycle window 2422. The further subsequent vector's angles differ from the baseline, the larger the asynchrony.

Although the dominant vector (the vector having the largest eigenvalue, or the vector with the largest power spectral density, as will be described in more detail below) is typically the vector that provides the best assessment of cardiac asynchrony, other vectors may also be used. For example, the third trace 2418 illustrates a QRS window 2424. The QRS window 2424 may provide a vector more closely associated with the depolarization wavefront from the cardiac ventricles. Changes in one or more vectors associated with the QRS window 2424 may provide a good measure of cardiac asynchrony. The variation of a characteristic, such as vector angle, over time may be directly correlated to cardiac asynchrony.

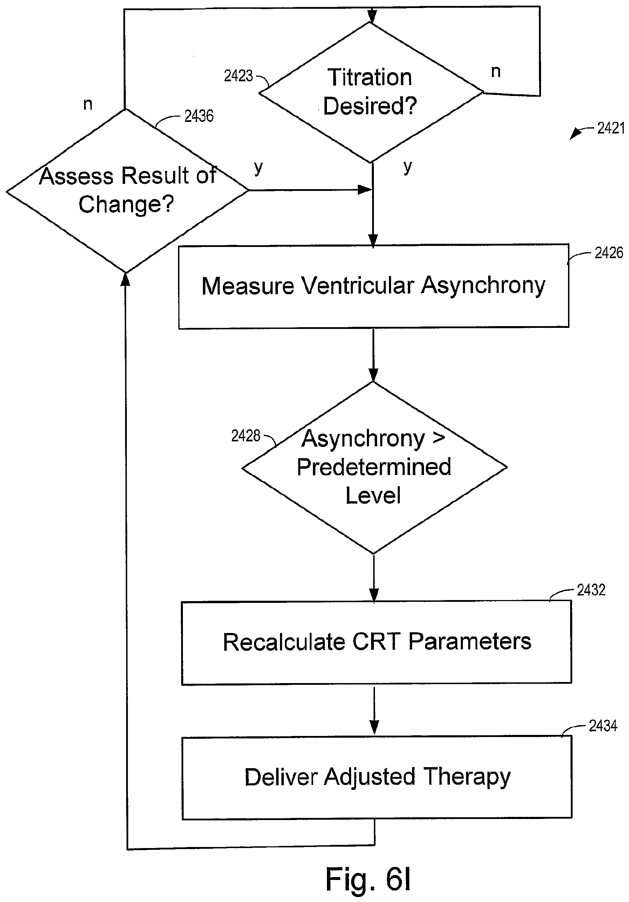

Figure 6I is a flow chart of a method 2421 of CRT titration in accordance with the present invention. The method 2421 may be initiated at timed intervals, on demand by a clinician, upon detection of a cardiac event, or as otherwise desired. Titration periods may be determined for each patient individually, or may be predetermined using clinical information. If titration 2423 is desired, a measure is made of a patient's ventricular asynchrony 2426, as was previously described.

If the asynchrony 2426 is measured to be beyond a predetermined value or threshold 2428, a recalculation 2432 is performed to determine optimum parameters given current conditions. The optimum parameters are then used to deliver an adjusted CRT therapy 2434.

It may be desirable to assess whether the patient responded to the adjusted CRT therapy 2434. If an assessment of the result is desired 2436, then a subsequent measure of the ventricular asynchrony 2426 may be performed immediately, or after an opportunity for the adjusted CRT therapy to take effect. If no assessment of the result is desired 2436, then the method returns back to the titration 2423 decision.

The method 2421 provides for a PIMD to automatically titrate CRT therapy when needed. The levels of therapy, as well as the delays, such as atrial-ventricular (AV) delays and interventricular (W) delays, may be adjusted to maintain improved hemodynamics for the patient. Auto-titration such as is illustrated in Figure 6I may also be combined with auto-capture and auto-threshold techniques to provide patients with adaptive systems that improve efficacy.

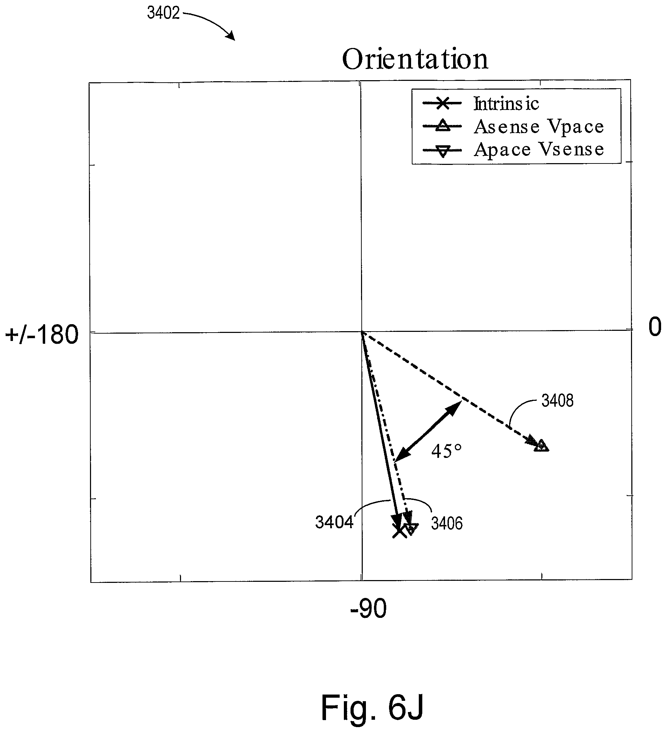

Referring now to Figure 6J, a graph 3402 illustrating vector angles for cardiac activation sequence orientations of intrinsic, ventricle-originated, and atrium-originated orientations demonstrates a significant distinction between ventricle and atrium originated activation sequences. The graph 3402 follows the same axes conventions as Figures 2 and 3A.

An intrinsic atrium-originated activation sequence vector 3404 and an atrial paced/ventricular sensed activation sequence vector 3406 both lie approximately 45 degrees from an atrial sensed/ventricular paced activation sequence vector 3408. The intrinsic atrium-originated activation sequence vector 3404 and the atrial paced/ventricular sensed activation sequence vector 3406 lie at approximately 80 degrees in the graph 3402. The atrial sensed/ventricular paced activation sequence vector 3408 lies at approximately 35 degrees in the graph 3402. This difference in angular orientation is readily detected using activation sequence monitoring and tracking.

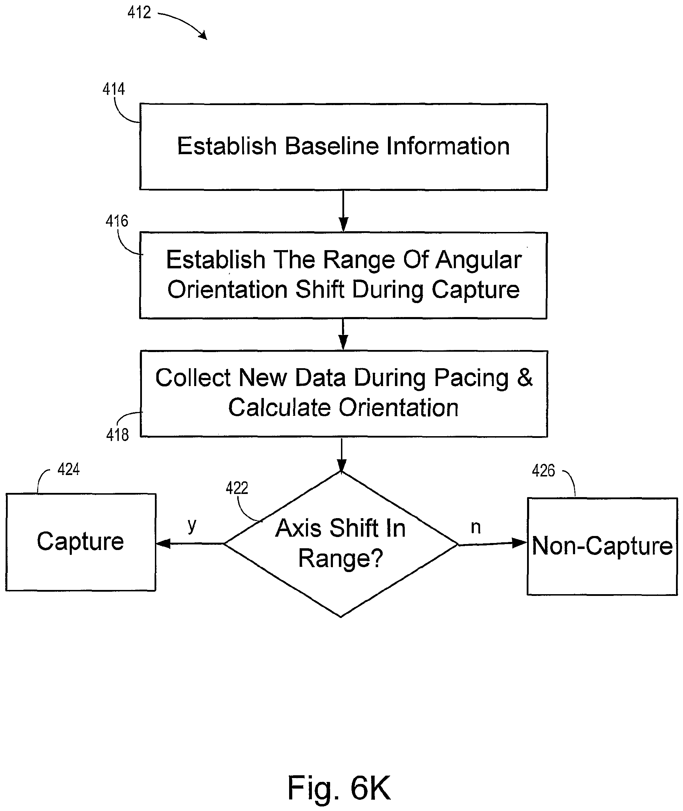

Figure 6K is a flow chart of a method 3412 of capture verification in accordance with the present invention. The method 3412 involves establishing baseline information 3414 regarding intrinsic cardiac activation sequence vector information. For example, baselines may be established using clinical trials, providing a range of normal variation for the study population, which then may be used as a representative baseline. In another example, during implantation of a PIMD, a clinician may establish individual baselines for individual patients. In yet another example, surface ECG information may be used to establish individual baselines for a patient before implantation of a PIMD.

The method 3412 further involves establishing ranges of orientation 3416 for cardiac capture. For example, an orientation outside an established range of normal variation from a clinical trial may serve to differentiate capture from non-capture. For example, detecting ventricular paced cardiac capture may be accomplished by detecting about a 45 degree phase change of a cardiac signal vector representative of cardiac capture relative to non-capture.

A PIMD implementing the method 3412 may collect new cardiac activation sequence data during pacing, and calculate a vector orientation 3418. If the axis shift is determined to be in the established capture range 3422 of orientation, then cardiac a capture 3424 condition exists. If the axis shift is determined not to be in the established capture range 3422 of orientation, then a non-capture condition 3426 exists.

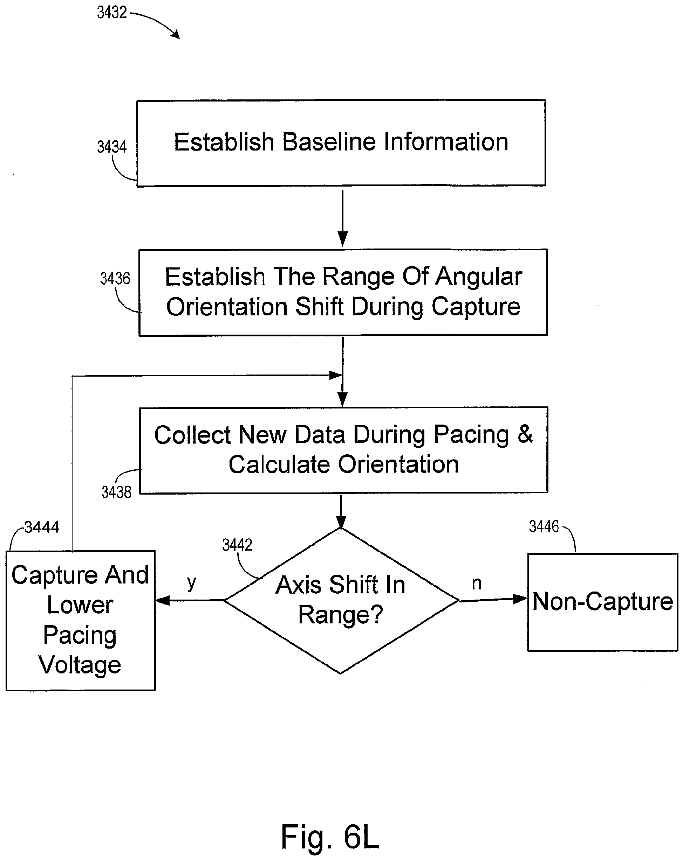

Figure 6L is a flow chart of a method 3432 of capture threshold adjustment in accordance with the present invention. The method 3432 involves establishing baseline information 3434 regarding intrinsic cardiac activation sequence vector information, such as by using clinical data or using patient specific baselining as was previously described.

The method 3432 further involves establishing ranges of orientation 3436 for cardiac capture as described previously. A PIMD utilizing the method 3432 may collect new cardiac activation sequence data during a pacing threshold adjustment test, and calculate a vector orientation 3438. If the axis shift is determined to be in the established capture range 3442 of orientation, then a cardiac capture 3444 condition exists, and the pacing threshold is lowered.

The threshold test may then continue at the lowered pacing level, and new data is collected, and the vector orientation 3438 is calculated for the new pacing level. If the axis shift is determined not to be in the established capture range 3422 of orientation, then a non-capture condition 3446 exists, and the pacing threshold information from the test is used by the PIMD to set a new pacing level for the patient.

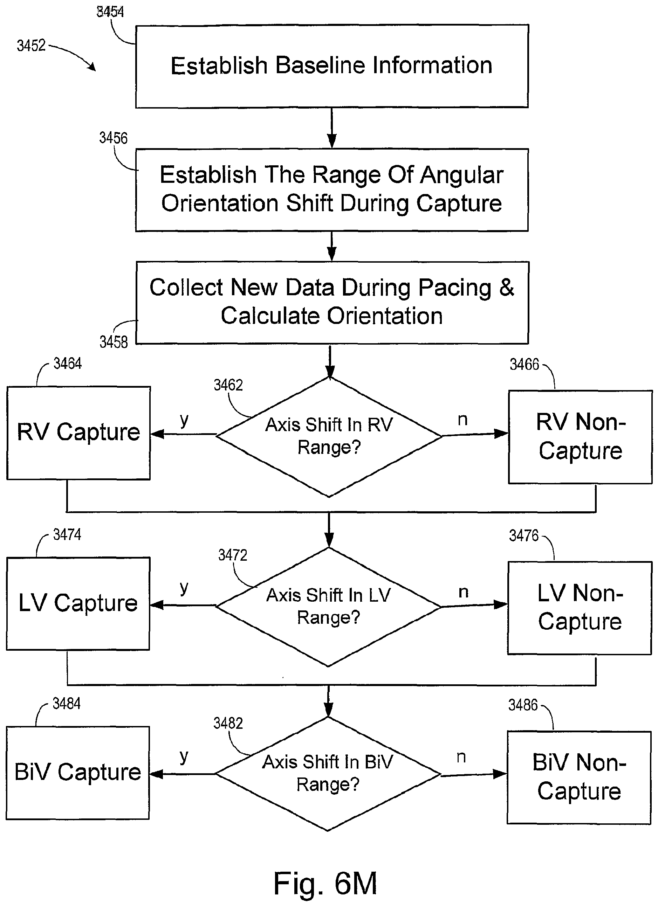

Figure 6M is a flow chart of a method 3452 of verifying left ventricular (LV), right ventricular (RV), and bi-ventricular (BiV) capture in accordance with the present invention. The method 3452 involves establishing baseline information 3454 regarding intrinsic cardiac activation sequence vector information, such as by using clinical data or using patient specific baselining as was previously described.

In this illustrative example, baseline information is established for a multiplicity of cardiac capture scenarios. In particular, Figure 6M illustrates verifying LV, RV, and BiV capture in accordance with the present invention. The method 3452 involves establishing ranges of orientation 3456 during RV, LV and BiV capture. A PIMD utilizing the method 3452 may collect new cardiac activation sequence data during pacing, and calculate a vector orientation 3458. If the axis shift is determined to be in the established RV capture range 3462 of orientation, then an RV cardiac capture 3464 condition exists. If the axis shift is determined not to be in the established RV capture range 3462 of orientation, then an RV non-capture condition 3466 exists.

The method 3452 then continues to evaluate other capture conditions. If the axis shift is determined to be in the established LV capture range 3472 of orientation, then an LV cardiac capture 3474 condition exists. If the axis shift is determined not to be in the established LV capture range 3472 of orientation, then an LV non-capture condition 3476 exists.

The method 3452 then continues to evaluate another capture condition. If the axis shift is determined to be in the established BiV capture range 3482 of orientation, then BiV cardiac capture 3484 condition exists. If the axis shift is determined not to be in the established BiV capture range 3482 of orientation, then a BiV non-capture condition 3486 exists.

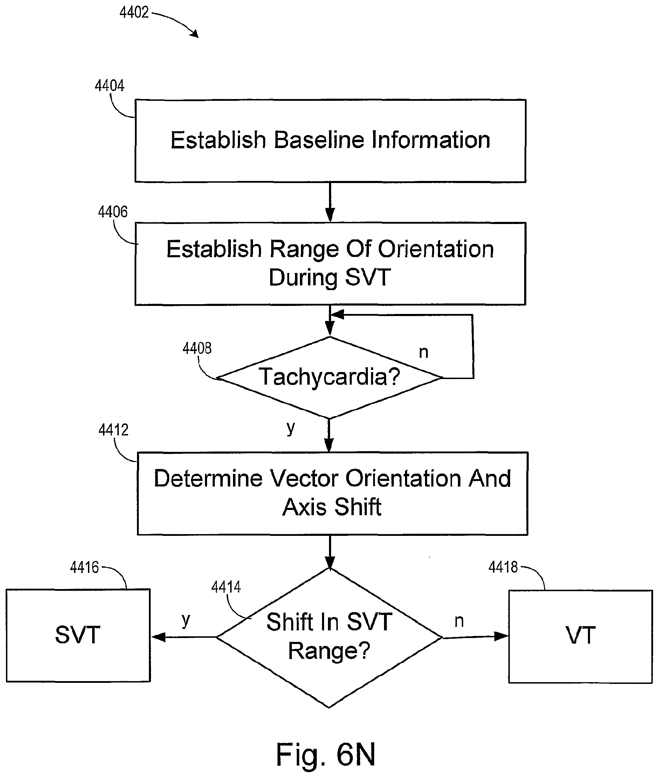

Figure 6N is a flow chart of a method 4402 of differentiating supra-ventricular tachycardia (SVT) from ventricular tachycardia (VT) in accordance with the present invention. The method 4402 establishes baseline information 4404 regarding cardiac activation sequence vector information. For example, baselines may be established using clinical trials, providing a range of normal variation for the study population, which then may be used as a representative baseline. In another example, during implantation of a PIMD, a clinician may establish individual baselines for individual patients. In yet another example, surface ECG information may be used to establish individual baselines for a patient before implantation of a PIMD.

The method 4402 then establishes ranges of orientation 4406 during SVT events. For example, an orientation outside an established range of normal variation from a clinical trial may serve to differentiate an SVT from a VT. For example, discriminating between supra-ventricular tachycardia and ventricular tachycardia may be accomplished by detecting about a 45 degree phase change of a cardiac signal vector representative of a ventricular tachycardia relative to a cardiac signal vector representative of a supra-ventricular tachycardia.

A PIMD utilizing the method 4402 may detect a tachycardia 4408. If the tachycardia 4408 is detected, a vector orientation determination 4412 is performed, and a change in axis shift may be found, such as a change in the angle of the cardiac activation sequence axis relative to the baseline 4404. If the axis shift is determined to be in the established SVT range of orientation 4406, then an SVT condition 4416 exists. If the axis shift is determined not to be in the established SVT range of orientation 4406, then a VT condition 4418 exists.

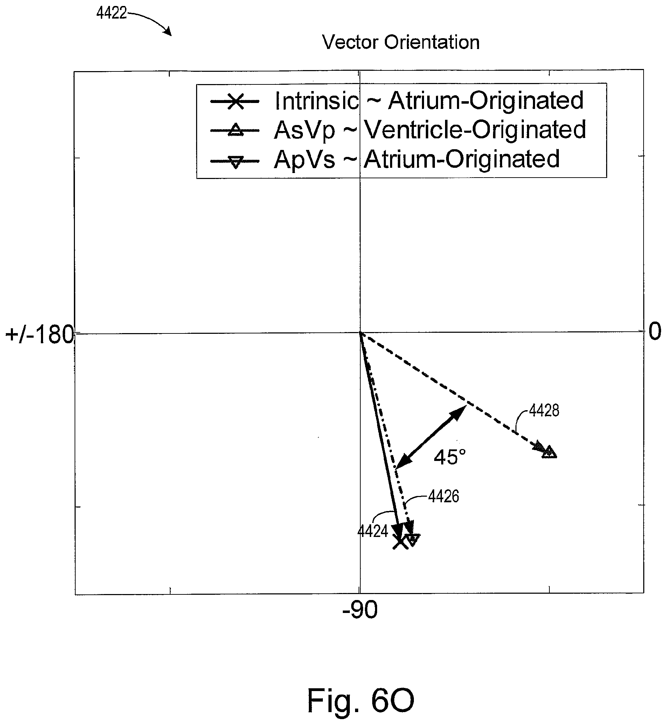

Referring now to Figure 6O, a graph 4422 illustrating vector angles for cardiac activation sequence orientations of intrinsic, ventricle originated, and atrium originated orientations demonstrates the significant departure of ventricle originated activation sequences from atrium originated activation sequences. The graph 4422 follows the same axes conventions as Figures 2 and 3A.

An intrinsic atrium-originated activation sequence vector 4424 and an atrial paced/ventricular sensed activation sequence vector 4426 both lie approximately 45 degrees from an atrial sensed/ventricular paced activation sequence vector 4428. The intrinsic atrium-originated activation sequence vector 4424 and the atrial paced/ventricular sensed activation sequence vector 4426 lie at approximately 80 degrees in the graph 4422. The atrial sensed/ventricular paced activation sequence vector 4428 lies at approximately 35 degrees in the graph 4422. This difference is readily detected using activation sequence monitoring and tracking.

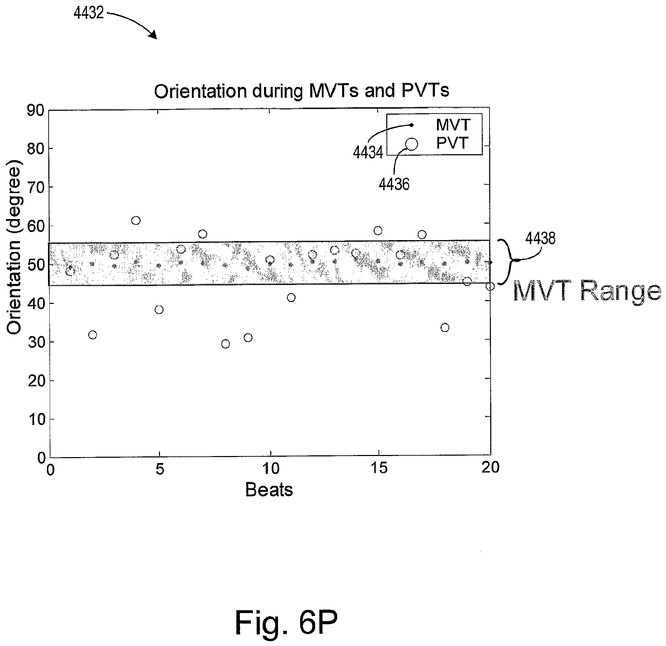

Figure 6P is a graph 4432 illustrating the variance of activation sequence vector angles during monomorphic ventricular tachycardia (MVT) and polymorphic ventricular tachycardia (PVT). The graph 4432 has 20 consecutive heartbeats indicated on its abscissa, and orientation of the activation sequence vector, in degrees, on its ordinate. The data depicted in the graph 4432 was randomly generated for illustrative purposes. Each point on the graph indicates the dominant orientation vector angle for 20 consecutive beats of a hypothetical MVT condition 4434 or a hypothetical PVT condition 4436. Data for the MVT condition 4434 is seen to lie within a variance range 4438. A PVT condition may produce a variance in the range of orientation angle significantly larger than the variance range 4438, as is illustrated in the graph of Figure 6P.

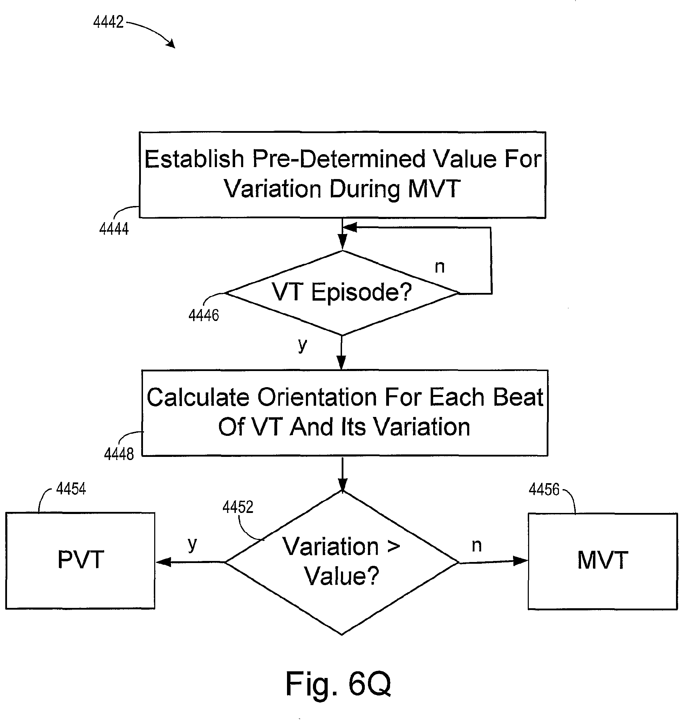

Figure 6Q is a flow chart of a method 4442 of differentiating MVT from PVT in accordance with the present invention. The method 4442 establishes one or more pre-determined values for MVT, such as by using clinical data or using baselining as was previously described. If VT is detected at detection block 4446, a vector orientation calculation 4448 is performed for several consecutive beats until a variance is determined. A comparison 4452 determines if the variance is greater than the pre-determined value(s) 4444. If the variance is greater than the pre-determined value(s) 4444, a PVT condition 4454 exists. If the variance is less than the pre-determined value(s) 4444, a MVT condition 4456 exists.

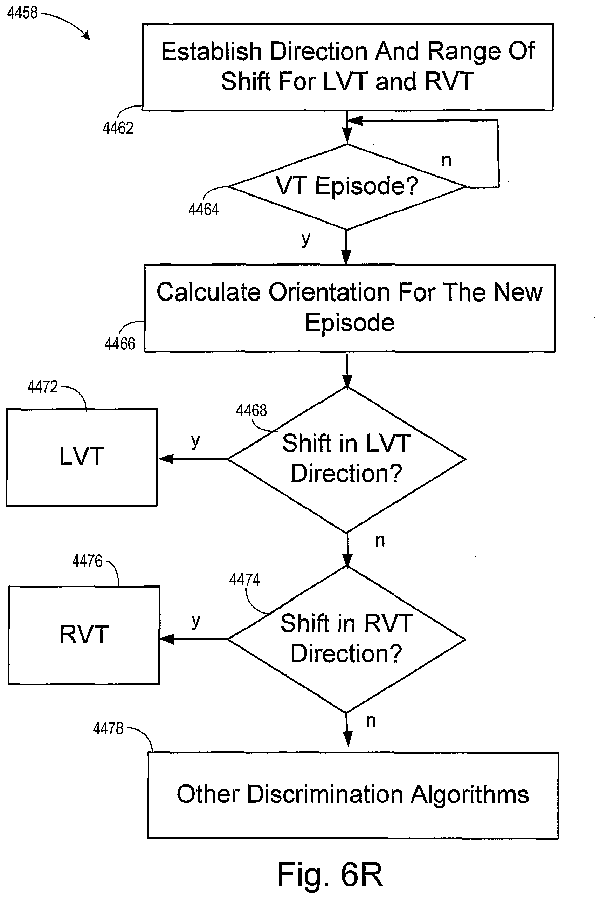

Figure 6R is a flow chart of a method 4458 of differentiating left ventricular tachycardia (LVT) from right ventricular tachycardia (RVT) in accordance with the present invention. The method 4458 establishes a direction and range of shift 4462 for LVT and RVT regarding cardiac activation sequence vector information. For example, the direction and range of shift 4462 for LVT and RVT may be established using clinical trials, providing a range of normal variation for the study population, which may then be used as a representative baseline. In another example, during implantation of a PIMD, a clinician may establish individual directions and ranges of shift 4462 of LVT and RVT for individual patients. In yet another example, surface ECG information may be used to establish individual baselines for a patient before implantation of a PIMD.

A PIMD utilizing the method 4458 may detect a ventricular tachycardia 4464. If the ventricular tachycardia 4464 is detected, an orientation for the new vector is calculated 4466. A comparison 4468 to the directions and ranges of shift 4462 of LVT is made, and if the axis shift is determined to be in the LVT direction, then an LVT condition 4472 exists. A comparison 4474 to the directions and ranges of shift 4462 of RVT is made, and if the axis shift is determined to be in the RVT direction, then an RVT condition 4476 exists. If no shifts in the LVT or RVT directions are observed, then other discrimination algorithms 4478 may be used to discriminate arrhythmias, such as other discrimination methods of the present invention.

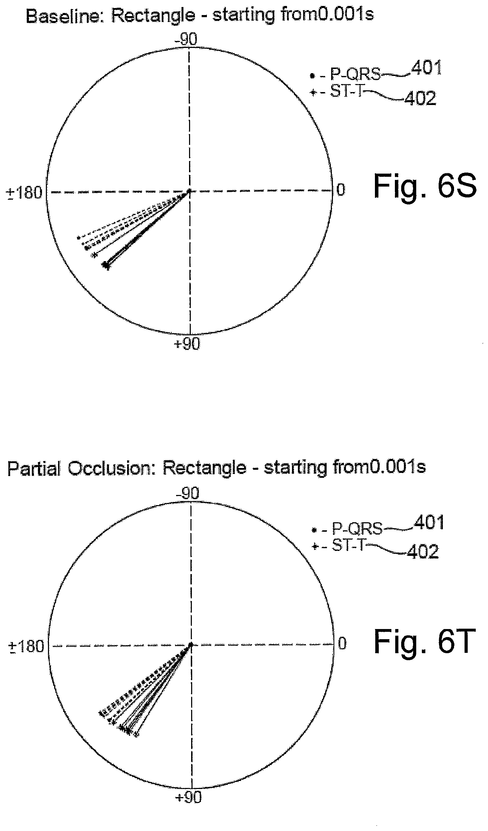

The following Figures 6S through 6AP are graphs of activation sequence vectors for the dominant vector determined from a windowed portion of cardiac cycles sensed during pre-clinical experiments in connection with an ischemia detection technique of the present invention. ECGs from a porcine subject were recorded using three surface patch electrodes spaced 1.5 inches apart. It is understood that the activation sequence vectors depicted in Figures 6S-6AP may be obtained by use of two or more implantable electrodes, configurations of which are discussed elsewhere herein. Moreover, the data acquired to develop the activation sequence vectors depicted in Figures 6S-6AP may be obtained by use an implantable cardiac device, configurations of which are discussed elsewhere herein.

Recordings were made of intrinsic cardiac function as well as LV paced cardiac function. Blood flow to a portion of the heart was varied using an occlusion of the left anterior descending (LAD) artery, with flow measured using a flow meter. Flow was varied between 100% open, 75% occluded, and 100% occluded conditions. The resulting data set was then analyzed using a source separation methodology, as will be further described below with respect to the discussion of Figures 14 through 16.

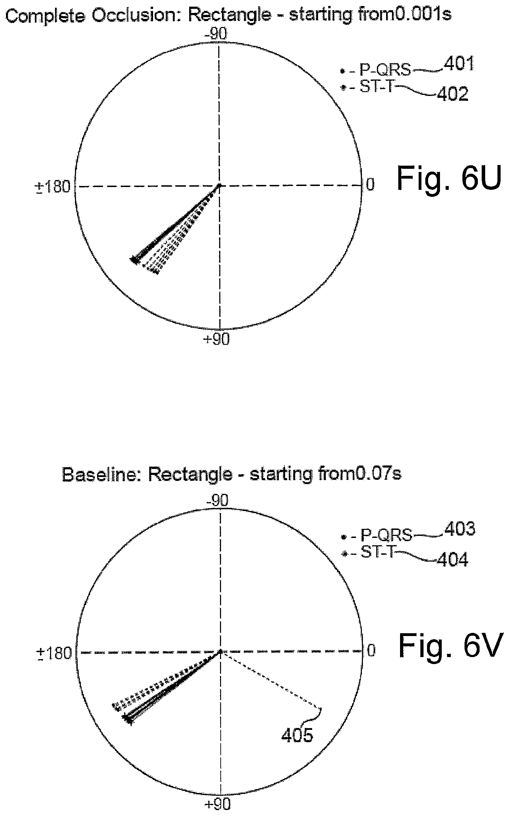

Figures 6S, 6T, and 6U are graphs illustrating activation sequence vector angles for a rectangular windowed portion of a cardiac signal, starting at 0.001 seconds from a predetermined reference, developed from a live porcine subject at baseline, partial LAD occlusion, and complete LAD occlusion respectively, in accordance with the present invention. In Figures 6S, 6T, and 6U, a group of P-QRS segment vectors 401 are identified by vectors terminating in dots, and a group of ST-T segment vectors 402 are identified by vectors terminating in stars. Figure 6S illustrates the vectors 401, 402 determined using a rectangular window starting at 0.001 seconds delay, for the baseline condition of zero occlusion (100% flow).

Figure 6T illustrates the vectors 401, 402 determined during a partial occlusion (75% occluded, 25% flow), with other parameters equal to those illustrated in Figure 6S. Figure 6U illustrates the vectors 401, 402 determined during total occlusion (100% occluded, 0% flow), with other parameters equal to those illustrated in Figure 6S. It is evident in the progression of the ST-T segment vectors 402 that occlusion may be detected acutely, as the approximate angle of the ST-T segment vectors 402 varies from about 155 degrees at baseline, to about 145 degrees at 75% occlusion, and about 130 degrees at complete occlusion. Another measure for occlusion may be the relationship between the angles of the P-QRS segment vectors 401 relative to the ST-T segment vectors 402. At baseline, the ST-T segment vectors 402 lie at angles greater than the P-QRS segment vectors 401, but for the 100% occluded condition, the ST-T segment vectors 402 lie at angles less than the P-QRS segment vectors 401.

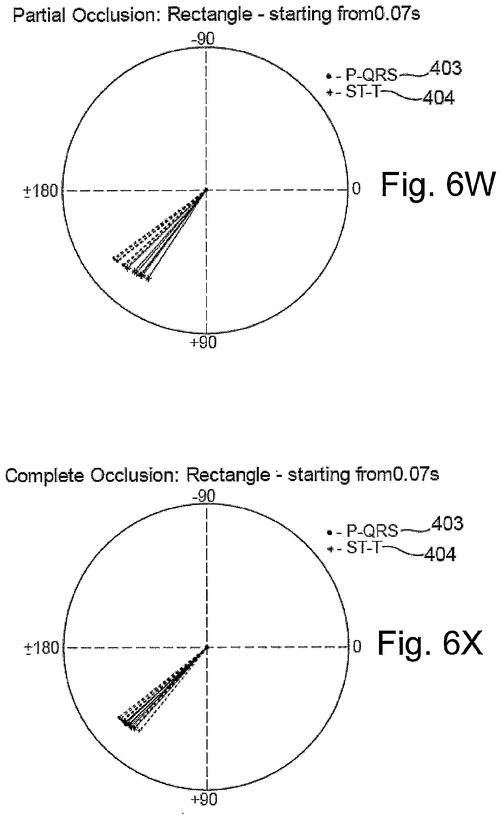

Figures 6V, 6W, and 6X are graphs illustrating activation sequence vector angles for a rectangular windowed portion of a cardiac signal, starting at 0.07 seconds, from a live porcine subject at baseline, partial LAD occlusion, and complete LAD occlusion respectively, in accordance with the present invention. In Figures 6V, 6W, and 6X, a group of P-QRS segment vectors 403 are identified by vectors terminating in dots, and a group of ST-T segment vectors 404 are identified by vectors terminating in stars. Figure 6V illustrates the vectors 403, 404 determined using a rectangular window starting at 0.07 seconds delay, for the baseline condition of zero occlusion (100% flow). A P-QRS vector 405 is ignored for purposes of this discussion.

Figure 6W illustrates the vectors 403, 404 determined during a partial occlusion (75% occluded, 25% flow), with other parameters equal to those illustrated in Figure 6V. Figure 6X illustrates the vectors 403, 404 determined during total occlusion (100% occluded, 0% flow), with other parameters equal to those illustrated in Figure 6V. The progression of the vectors 403, 404 for the rectangular window starting at 0.07 seconds does not appear to differentiate ischemia as specifically as was evident with respect to the graphs of Figures 6S, 6T, and 6U.

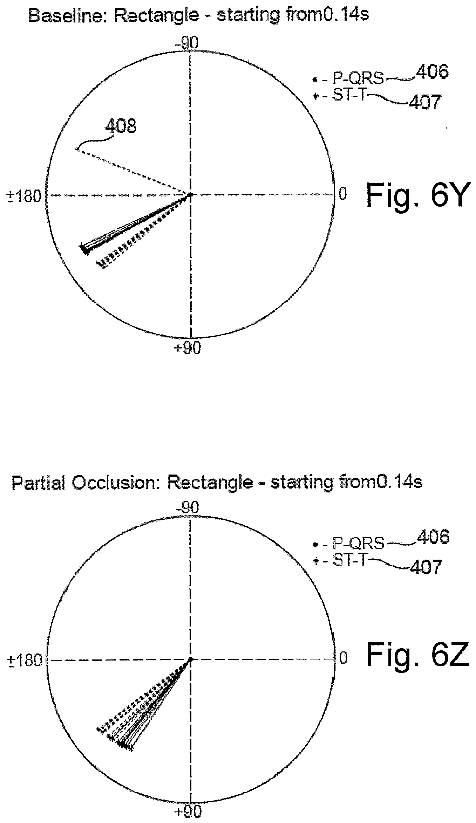

Figures 6Y, 6Z, and 6AA are graphs illustrating activation sequence vector angles for a rectangular windowed portion of a cardiac signal, starting at 0.14 seconds, from a live porcine subject at baseline, partial LAD occlusion, and complete LAD occlusion respectively, in accordance with the present invention. In Figures 6Y, 6Z, and 6AA, a group of P-QRS segment vectors 406 are identified by vectors terminating in dots, and a group of ST-T segment vectors 407 are identified by vectors terminating in stars. Figure 6Y illustrates the vectors 406, 407 determined using a rectangular window starting at 0.14 seconds delay, for the baseline condition of zero occlusion (100% flow). A P-QRS vector 408 is ignored for purposes of this discussion.

Figure 6Z illustrates the vectors 406, 407 determined during a partial occlusion (75% occluded, 25% flow), with other parameters equal to those illustrated in Figure 6Y. Figure 6AA illustrates the vectors 406, 407 determined during total occlusion (100% occluded, 0% flow), with other parameters equal to those illustrated in Figure 6Y. It is evident in the progression of the ST-T segment vectors 407 that occlusion may be detected acutely, as the approximate angle of the ST-T segment vectors 407 varies from about 155 degrees at baseline, to about 125 degrees at 75% occlusion and at full occlusion. Another measure for occlusion may be the relationship between the angles of the P-QRS segment vectors 406 relative to the ST-T segment vectors 407. At baseline, the ST-T segment vectors 407 lie at angles greater than the P-QRS segment vectors 406, but for the 75% and 100% occluded conditions, the ST-T segment vectors 407 lie at angles less than the P-QRS segment vectors 406.

Figures 6AB, 6AC, and 6AD are graphs illustrating activation sequence vector angles for a rectangular windowed portion of a cardiac signal, starting at 0.21 seconds, from a live porcine subject at baseline, partial LAD occlusion, and complete LAD occlusion respectively, in accordance with the present invention. In Figures 6AB, 6AC, and 6AD, a group of P-QRS segment vectors 408 are identified by vectors terminating in dots, and a group of ST-T segment vectors 409 are identified by vectors terminating in stars. Figure 6AB illustrates the vectors 408, 409 determined using a rectangular window starting at 0.21 seconds delay, for the baseline condition of zero occlusion (100% flow). A P-QRS vector 411 is ignored for purposes of this discussion.

Figure 6AC illustrates the vectors 408, 409 determined during a partial occlusion (75% occluded, 25% flow), with other parameters equal to those illustrated in Figure 6AB. Figure 6AD illustrates the vectors 408, 409 determined during total occlusion (100% occluded, 0% flow), with other parameters equal to those illustrated in Figure 6AB. It is evident in the progression of the ST-T segment vectors 409 that occlusion may be detected acutely, as the approximate angle of the ST-T segment vectors 409 varies from about 155 degrees at baseline, to about 125 degrees at 75% occlusion and at full occlusion. Another measure for occlusion may be the relationship between the angles of the P-QRS segment vectors 408 relative to the ST-T segment vectors 409. At baseline, the ST-T segment vectors 409 lie at angles greater than the P-QRS segment vectors 408, but for the 75% occluded condition, the ST-T segment vectors 409 typically lie at angles less than the P-QRS segment vectors 408.

Figures 6AE, 6AF, and 6AG are graphs illustrating activation sequence vector angles for a Hanning windowed portion of a cardiac signal, starting at 0.001 seconds, from a live porcine subject at baseline, partial LAD occlusion, and complete LAD occlusion respectively, in accordance with the present invention. In Figures 6AE, 6AF, and 6AG, a group of P-QRS segment vectors 412 are identified by vectors terminating in dots, and a group of ST-T segment vectors 413 are identified by vectors terminating in stars. Figure 6AE illustrates the vectors 412, 413 determined using a Hanning window starting at 0.001 seconds delay, for the baseline condition of zero occlusion (100% flow).

Figure 6AF illustrates the vectors 412, 413 determined during a partial occlusion (75% occluded, 25% flow), with other parameters equal to those illustrated in Figure 6AE. Figure 6AG illustrates the vectors 412, 413 determined during total occlusion (100% occluded, 0% flow), with other parameters equal to those illustrated in Figure 6AE. It is evident in the progression of the ST-T segment vectors 413 that occlusion may be detected acutely, as the approximate angle of the ST-T segment vectors 413 varies from about 155 degrees at baseline, to about 125 degrees at 75% occlusion, and about 135 degrees at full occlusion. Another measure for occlusion may be the relationship between the angles of the P-QRS segment vectors 412 relative to the ST-T segment vectors 413. At baseline, the ST-T segment vectors 413 lie at angles greater than the P-QRS segment vectors 401, but for the 75% occluded condition, the ST-T segment vectors 413 lie at angles less than the P-QRS segment vectors 412.

Figures 6AH, 6AI, and 6AJ are graphs illustrating activation sequence vector angles for a Hanning windowed portion of a cardiac signal, starting at 0.07 seconds, from a live porcine subject at baseline, partial LAD occlusion, and complete LAD occlusion respectively, in accordance with the present invention. In Figures 6AH, 6AI, and 6AJ, a group of P-QRS segment vectors 414 are identified by vectors terminating in dots, and a group of ST-T segment vectors 415 are identified by vectors terminating in stars. Figure 6AH illustrates the vectors 414, 415 determined using a Hanning window starting at 0.07 seconds delay, for the baseline condition of zero occlusion (100% flow).

Figure 6AI illustrates the vectors 414, 415 determined during a partial occlusion (75% occluded, 25% flow), with other parameters equal to those illustrated in Figure 6AH. Figure 6AJ illustrates the vectors 414, 415 determined during total occlusion (100% occluded, 0% flow), with other parameters equal to those illustrated in Figure 6AH. It is evident in the progression of the ST-T segment vectors 415 that occlusion may be detected acutely, as the approximate angle of the ST-T segment vectors 415 varies from about 170 to 185 degrees at baseline, to about 125 to 135 degrees at 75% occlusion, and about 130 to 140 degrees at full occlusion. Another measure for occlusion may be the relationship between the angles of the P-QRS segment vectors 414 relative to the ST-T segment vectors 415. At baseline, the ST-T segment vectors 415 lie at angles greater than the P-QRS segment vectors 414, but for the 75% occluded condition, the ST-T segment vectors 415 lie at angles less than the P-QRS segment vectors 414.

Figures 6AK, 6AL, and 6AM are graphs illustrating activation sequence vector angles for a Hanning windowed portion of a cardiac signal, starting at 0.14 seconds, from a live porcine subject at baseline, partial LAD occlusion, and complete LAD occlusion respectively, in accordance with the present invention. In Figures 6AK, 6AL, and 6AM, a group of P-QRS segment vectors 416 are identified by vectors terminating in dots, and a group of ST-T segment vectors 417 are identified by vectors terminating in stars. Figure 6AK illustrates the vectors 416, 417 determined using a Hanning window starting at 0.14 seconds delay, for the baseline condition of zero occlusion (100% flow). A P-QRS vector 418, a P-QRS vector 419, and an ST-T vector 421 are ignored for purposes of this discussion.

Figure 6AL illustrates the vectors 416, 417 determined during a partial occlusion (75% occluded, 25% flow), with other parameters equal to those illustrated in Figure 6AK. In Figure 6AL, a P-QRS vector 422 and a P-QRS vector 423 are ignored for purposes of this discussion. Figure 6AM illustrates the vectors 416, 417 determined during total occlusion (100% occluded, 0% flow), with other parameters equal to those illustrated in Figure 6AK. It is evident in the progression of the ST-T segment vectors 417 that occlusion may be detected acutely, as the approximate angle of the ST-T segment vectors 417 varies from about 150 to 165 degrees at baseline, to about 120 to 140 degrees at 75% occlusion, and about 130 to 145 degrees at full occlusion. Another measure for occlusion may be the relationship between the angles of the P-QRS segment vectors 416 relative to the ST-T segment vectors 417. At baseline, the ST-T segment vectors 417 lie at angles greater than the P-QRS segment vectors 416, but for the 75% occluded condition, the ST-T segment vectors 417 lie at angles less than the P-QRS segment vectors 416.

Figures 6AN, 6AO, and 6AP are graphs illustrating activation sequence vector angles for a Hanning windowed portion of a cardiac signal, starting at 0.21 seconds, from a live porcine subject at baseline, partial LAD occlusion, and complete LAD occlusion respectively, in accordance with the present invention. In Figures 6AN, 6AO, and 6AP, a group of P-QRS segment vectors 424 are identified by vectors terminating in dots, and a group of ST-T segment vectors 425 are identified by vectors terminating in stars. Figure 6AN illustrates the vectors 424, 425 determined using a Hanning window starting at 0.21 seconds delay, for the baseline condition of zero occlusion (100% flow). A P-QRS vector 426 and a P-QRS vector 427 are ignored for purposes of this discussion.

Figure 6AO illustrates the vectors 424, 425 determined during a partial occlusion (75% occluded, 25% flow), with other parameters equal to those illustrated in Figure 6AN. Figure 6AP illustrates the vectors 424, 425 determined during total occlusion (100% occluded, 0% flow), with other parameters equal to those illustrated in Figure 6AN. It is evident in the progression of the ST-T segment vectors 425 that occlusion may be detected acutely, as the approximate angle of the ST-T segment vectors 425 varies from about 150 to 165 degrees at baseline, to about 105 to 140 degrees at 75% occlusion, and about 120 to 125 degrees at full occlusion. Another measure for occlusion may be the relationship between the angles of the P-QRS segment vectors 424 relative to the ST-T segment vectors 425. At baseline, the ST-T segment vectors 425 lie at angles greater than the P-QRS segment vectors 424, but for the 100% occluded conditions, the ST-T segment vectors 425 lie at angles less than the P-QRS segment vectors 424.

Figure 7 is a top view of a PIMD 782 in accordance with the present invention, having at least three electrodes. Although multiple electrodes are illustrated in Figure 7 as located on the can, typically the can includes one electrode, and other electrodes are coupled to the can using a lead. The PIMD 782 shown in the embodiment illustrated in Figure 7 includes a first electrode 781a, a second electrode 781b, and a third electrode 781c provided with a can 703. The PIMD 782 detects and records cardiac activity. The can 703 is illustrated as incorporating a header 789 that may be configured to facilitate removable attachment between one or more leads and the can 703. The can 703 may include any number of electrodes positioned anywhere in or on the can 703, such as optional electrodes 781 d, 781e, 781 f, and 781g. Each electrode pair provides one vector available for the sensing of ECG signals.

Figure 8 is a block diagram of a process 850 useful for extracting vector information for cardiac activation sequence monitoring and tracking in accordance with the present invention. The process 850 starts at block 851, where multiple concurrent measurements are obtained between multiple respective electrode pairs, chosen from at least three electrodes. Block 852 provides for pre-filtering the collected signals with, for example, a linear-phase filter to suppress broadly incoherent noise, and to generally maximize the signal-to-noise ratio.

Block 853 indicates the computation of the cross-correlation matrix, which may be averaged over a relatively short time interval, such as about 1 second. This block enhances the components that are mutually correlated. Block 854 is then provided for computation of the eigenvalues of the cross-correlation matrix. The smaller eigenvalues, normally associated with noise, may then be used at block 855 to eliminate noise, by removing the noise components of the composite signals associated with those eigenvalues.

At block 856, signals may be separated from the composite signals using the eigenvalues. Separated sources may be obtained by taking linear combinations of the recorded signals, as specified in the eigenvectors corresponding to the larger eigenvalues. Optionally, block 857 provides for performing additional separation based on higher order statistics, if the cardiac signal or other signal of interest is not found among the signals separated at block 856.

At block 858, the cardiac signal may be identified based on the selection criteria, along with its associated vector, among the separated signals. Typically, the cardiac signal is found among the signals associated with the largest eigenvalues. Vector selection and updating systems and methods are further described in commonly assigned co-pending US Patent Application No. 10/876,008 filed on June 24, 2004 and entitled "Automatic Orientation Determination for ECG Measurements Using Multiple Electrodes" [Attorney Docket No. GUID.149PA], hereby incorporated herein by reference.

For purposes of illustration, and not of limitation, various embodiments of devices that may use cardiac activation sequence monitoring and tracking in accordance with the present invention are described herein in the context of PIMD's that may be implanted under the skin in the chest region of a patient. A PIMD may, for example, be implanted subcutaneously such that all or selected elements of the device are positioned on the patient's front, back, side, or other body locations suitable for monitoring cardiac activity and/or delivering cardiac stimulation therapy. It is understood that elements of the PIMD may be located at several different body locations, such as in the chest, abdominal, or subclavian region with electrode elements respectively positioned at different regions near, around, in, or on the heart.

The primary housing (e.g., the active or non-active can) of the PIMD, for example, may be configured for positioning outside of the rib cage at an intercostal or subcostal location, within the abdomen, or in the upper chest region (e.g., subclavian location, such as above the third rib). In one implementation, one or more leads incorporating electrodes may be located in direct contact with the heart, great vessel or coronary vasculature, such as via one or more leads implanted by use of conventional transvenous delivery approaches. In another implementation, one or more electrodes may be located on the primary housing and/or at other locations about, but not in direct contact with the heart, great vessel or coronary vasculature.

In a further implementation, for example, one or more electrode subsystems or electrode arrays may be used to sense cardiac activity and/or deliver cardiac stimulation energy in a PIMD configuration employing an active can or a configuration employing a non-active can. Electrodes may be situated at anterior and/or posterior locations relative to the heart. Examples of useful electrode locations and features that may be incorporated in various embodiments of the present invention are described in commonly owned, co-pending US Patent Application Nos. 10/465,520 filed June 19, 2003, entitled "Methods and Systems Involving Subcutaneous Electrode Positioning Relative to a Heart"; 10/795,126 filed March 5, 2004, entitled "Wireless ECG In Implantable Devices"; and 10/738,608 filed December 17, 2003, entitled "Noise Canceling Cardiac Electrodes," which are hereby incorporated herein by reference.

Certain configurations illustrated herein are generally described as capable of implementing various functions traditionally performed by an implantable cardioverter/defibrillator (ICD), and may operate in numerous cardioversion/defibrillation modes as are known in the art. Examples of ICD circuitry, structures and functionality, aspects of which may be incorporated in a PIMD of a type that may benefit from cardiac activation sequence monitoring and/or tracking are disclosed in commonly owned U.S. Patent Nos. 5,133,353; 5,179,945; 5,314,459; 5,318,597; 5,620,466; and 5,662,688, which are hereby incorporated herein by reference.

In particular configurations, systems and methods may perform functions traditionally performed by pacemakers, such as providing various pacing therapies as are known in the art, in addition to cardioversion/defibrillation therapies. Examples of pacemaker circuitry, structures and functionality, aspects of which may be incorporated in a PIMD of a type that may benefit from cardiac activation sequence monitoring and/or tracking methods and implementations are disclosed in commonly owned U.S. Patent Nos. 4,562,841; 5,284,136; 5,376,106; 5,036,849; 5,540,727; 5,836,987; 6,044,298; and 6,055,454, which are hereby incorporated herein by reference. It is understood that PIMD configurations may provide for non-physiologic pacing support in addition to, or to the exclusion of, bradycardia and/or anti-tachycardia pacing therapies.

A PIMD useful for extracting vector information for cardiac activation sequence monitoring and tracking in accordance with the present invention may implement diagnostic and/or monitoring functions as well as provide cardiac stimulation therapy. Examples of cardiac monitoring circuitry, structures and functionality, aspects of which may be incorporated in a PIMD of a type that may benefit from cardiac activation sequence monitoring and/or tracking methods and implementations are disclosed in commonly owned U.S. Patent Nos. 5,313,953; 5,388,578; and 5,411,031, which are hereby incorporated herein by reference.

Various embodiments described herein may be used in connection with congestive heart failure (CHF) monitoring, diagnosis, and/or therapy. A PIMD of the present invention may incorporate CHF features involving dual-chamber or bi-ventricular pacing therapy, cardiac resynchronization therapy, cardiac function optimization, or other CHF related methodologies. For example, any PIMD of the present invention may incorporate features of one or more of the following references: commonly owned US Pat. App. S/N 10/270,035, filed October 11, 2002, entitled "Timing Cycles for Synchronized Multisite Cardiac Pacing;" and US Patent Nos. 6,411,848; 6,285,907; 4,928,688; 6,459,929; 5,334,222; 6,026,320; 6,371,922; 6,597,951; 6,424,865; and 6,542,775, each of which is hereby incorporated herein by reference.

A PIMD may be used to implement various diagnostic functions, which may involve performing rate-based, pattern and rate-based, and/or morphological tachyarrhythmia discrimination analyses. Subcutaneous, cutaneous, and/or external sensors may be employed to acquire physiologic and non-physiologic information for purposes of enhancing tachyarrhythmia detection and termination. It is understood that configurations, features, and combination of features described in the present disclosure may be implemented in a wide range of implantable medical devices, and that such embodiments and features are not limited to the particular devices described herein.

Referring now to Figure 9, the implantable device illustrated in Figure 9 is an embodiment of a PIMD that may benefit from cardiac sequence monitoring and tracking in accordance with the present invention. In this example, the implantable device includes a cardiac rhythm management device (CRM) 900 including an implantable pulse generator 905 electrically and physically coupled to an intracardiac lead system 910.

Portions of the intracardiac lead system 910 are inserted into the patient's heart 990. The intracardiac lead system 910 includes one or more electrodes configured to sense electrical cardiac activity of the heart, deliver electrical stimulation to the heart, sense the patient's transthoracic impedance, and/or sense other physiological parameters, e,g, cardiac chamber pressure or temperature. Portions of the housing 901 of the pulse generator 905 may optionally serve as a can electrode.

Communications circuitry is disposed within the housing 901 for facilitating communication between the pulse generator 905 and an external communication device, such as a portable or bed-side communication station, patient-carried/worn communication station, or external programmer, for example. The communications circuitry may also facilitate unidirectional or bidirectional communication with one or more implanted, external, cutaneous, or subcutaneous physiologic or non-physiologic sensors, patient-input devices and/or information systems.

The pulse generator 905 may optionally incorporate a motion detector 920 that may be used to sense patient activity as well as various respiratory and cardiac related conditions. For example, the motion detector 920 may be optionally configured to sense snoring, activity level, and/or chest wall movements associated with respiratory effort, for example. The motion detector 920 may be implemented as an accelerometer positioned in or on the housing 901 of the pulse generator 905. If the motion sensor is implemented as an accelerometer, the motion sensor may also provide respiratory, e.g. rales, coughing, and cardiac, e.g. S1-S4 heart sounds, murmurs, and other acoustic information.

The lead system 910 and pulse generator 905 of the CRM 900 may incorporate one or more transthoracic impedance sensors that may be used to acquire the patient's respiratory waveform, or other respiratory-related information. The transthoracic impedance sensor may include, for example, one or more intracardiac electrodes 941, 942, 951-955, 963 positioned in one or more chambers of the heart 990. The intracardiac electrodes 941, 942, 951-955, 963 may be coupled to impedance drive/sense circuitry 930 positioned within the housing of the pulse generator 905.

In one implementation, impedance drive/sense circuitry 930 generates a current that flows through the tissue between an impedance drive electrode 951 and a can electrode on the housing 901 of the pulse generator 905. The voltage at an impedance sense electrode 952 relative to the can electrode changes as the patient's transthoracic impedance changes. The voltage signal developed between the impedance sense electrode 952 and the can electrode is detected by the impedance sense circuitry 930. Other locations and/or combinations of impedance sense and drive electrodes are also possible.

The lead system 910 may include one or more cardiac pace/sense electrodes 951-955 positioned in, on, or about one or more heart chambers for sensing electrical signals from the patient's heart 990 and/or delivering pacing pulses to the heart 990. The intracardiac sense/pace electrodes 951-955, such as those illustrated in Figure 9, may be used to sense and/or pace one or more chambers of the heart, including the left ventricle, the right ventricle, the left atrium and/or the right atrium. The lead system 910 may include one or more defibrillation electrodes 941, 942 for delivering defibrillation/cardioversion shocks to the heart.

The pulse generator 905 may include circuitry for detecting cardiac arrhythmias and/or for controlling pacing or defibrillation therapy in the form of electrical stimulation pulses or shocks delivered to the heart through the lead system 910. The pulse generator 905 may also incorporate circuitry, structures and functionality of the implantable medical devices disclosed in commonly owned U.S. Patent Nos. 5,203,348; 5,230,337; 5,360,442; 5,366,496; 5,397,342; 5,391,200; 5,545,202; 5,603,732; and 5,916,243; 6,360,127; 6,597,951; and US Patent Publication No. 2002/0143264, which are hereby incorporated herein by reference.

Figure 10 is a top view of a PIMD 1082 in accordance with the present invention, having at least three electrodes. One electrode is illustrated as an antenna 1005 of the PIMD that may also be used for radio-frequency (RF) communications. The PIMD 1082 shown in the embodiment illustrated in Figure 10 includes a first electrode 1098 and a second electrode 1099 coupled to a can 1003 through a header 1089, via an electrode module 1096. The first electrode 1098 and second electrode 1099 may be located on a lead 1083 (single or multiple lead, or electrode array), or may be located directly in or on the electrode module 1096.

The PIMD 1082 detects and records cardiac activity. The can 1003 is illustrated as incorporating the header 1089. The header 1089 may be configured to facilitate removable attachment between an electrode module 1096 and the can 1003, as is shown in the embodiment depicted in Figure 10. The header 1089 includes a female coupler 1092 configured to accept a male coupler 1093 from the electrode module 1096. The male coupler 1093 is shown having two electrode contacts 1094, 1095 for coupling one or more electrodes 1098, 1099 through the electrode module 1096 to the can 1003. An electrode 1081h and an electrode 1081k are illustrated on the header 1089 of the can 1003 and may also be coupled through the electrode module 1096 to the can 1003. The can 1003 may alternatively, or in addition to the header electrodes 1081h, 1081k and/or first and second electrodes 1098, 1099, include one or more can electrodes 1081 a, 1081b, 1081 c.

Recording and monitoring systems and methods that may benefit from cardiac activation sequence monitoring and tracking in accordance with the present invention are further described in commonly assigned co-pending US Patent Application No. 10/785,431 filed February 24, 2004 entitled "Reconfigurable Implantable Cardiac Monitoring And Therapy Delivery Device" hereby incorporated herein by reference.

Electrodes may also be provided on the back of the can 1003, typically the side facing externally relative to the patient after implantation. For example, electrodes 1081m, 1081p, and 1081r are illustrated as positioned in or on the back of the can 1003. Providing electrodes on both front and back surfaces of the can 1003 provides for a three-dimensional spatial distribution of the electrodes, which may provide additional discrimination capabilities for cardiac activation sequence monitoring and tracking in accordance with the present invention. Further description of three-dimensional configurations are described in US Patent Application No. 10/795,126 filed March 5, 2004, entitled "Wireless ECG In Implantable Devices," previously incorporated by reference.

In this and other configurations, the header 1089 incorporates interface features (e.g., electrical connectors, ports, engagement features, and the like) that facilitate electrical connectivity with one or more lead and/or sensor systems, lead and/or sensor modules, and electrodes. The header 1089 may also incorporate one or more electrodes in addition to, or instead of, the electrodes provided by the lead 1083, such as electrodes 1081h and 1081k, to provide more available vectors to the PIMD. The interface features of the header 1089 may be protected from body fluids using known techniques.

The PIMD 1082 may further include one or more sensors in or on the can 1003, header 1089, electrode module 1096, or lead(s) that couple to the header 1089 or electrode module 1096. Useful sensors may include electrophysiologic and non-electrophysiologic sensors, such as an acoustic sensor, an impedance sensor, a blood sensor, such as an oxygen saturation sensor (oximeter or plethysmographic sensor), a blood pressure sensor, minute ventilation sensor, or other sensor described or incorporated herein.

In one configuration, as is illustrated in Figure 11, electrode subsystems of a PIMD system are arranged about a patient's heart 1110. The PIMD system includes a first electrode subsystem, including a can electrode 1102, and a second electrode subsystem 1104 that includes at least two electrodes or at least one multi-element electrode. The second electrode subsystem 1104 may include a number of electrodes used for sensing and/or electrical stimulation and is connected to pulse generator 905 via lead 1106.

In various configurations, the second electrode subsystem 1104 may include a combination of electrodes. The combination of electrodes of the second electrode subsystem 1104 may include coil electrodes, tip electrodes, ring electrodes, multi-element coils, spiral coils, spiral coils mounted on non-conductive backing, screen patch electrodes, and other electrode configurations as will be described below. A suitable non-conductive backing material is silicone rubber, for example.

The can electrode 1102 is positioned on the housing 1101 that encloses the PIMD electronics. In one embodiment, the can electrode 1102 includes the entirety of the external surface of housing 1101. In other embodiments, various portions of the housing 1101 may be electrically isolated from the can electrode 1102 or from tissue. For example, the active area of the can electrode 1102 may include all or a portion of either the anterior or posterior surface of the housing 1101 to direct current flow in a manner advantageous for cardiac sensing and/or stimulation.

Portions of the housing may be electrically isolated from tissue to optimally direct current flow. For example, portions of the housing 1101 may be covered with a non-conductive, or otherwise electrically resistive, material to direct current flow. Suitable non-conductive material coatings include those formed from silicone rubber, polyurethane, or parylene, for example.

Figure 12 is a block diagram depicting various componentry of different arrangements of a PIMD in accordance with embodiments of the present invention. The components, functionality, and configurations depicted in Figure 12 are intended to provide an understanding of various features and combinations of features that may be incorporated in a PIMD. It is understood that a wide variety of device configurations are contemplated, ranging from relatively sophisticated to relatively simple designs. As such, particular PIMD configurations may include some componentry illustrated in Figure 12, while excluding other componentry illustrated in Figure 12.

Illustrated in Figure 12 is a processor-based control system 1205 which includes a micro-processor 1206 coupled to appropriate memory (volatile and/or non-volatile) 1209, it being understood that any logic-based control architecture may be used. The control system 1205 is coupled to circuitry and components to sense, detect, and analyze electrical signals produced by the heart and deliver electrical stimulation energy to the heart under predetermined conditions to treat cardiac arrhythmias and/or other cardiac conditions. The control system 1205 and associated components also provide pacing therapy to the heart. The electrical energy delivered by the PIMD may be in the form of low energy pacing pulses or high-energy pulses for cardioversion or defibrillation.

Cardiac signals are sensed using the electrode(s) 1214 and the can or indifferent electrode 1207 provided on the PIMD housing. Cardiac signals may also be sensed using only the electrode(s) 1214, such as in a non-active can configuration. As such, unipolar, bipolar, or combined unipolar/bipolar electrode configurations as well as multi-element electrodes and combinations of noise canceling and standard electrodes may be employed. The sensed cardiac signals are received by sensing circuitry 1204, which includes sense amplification circuitry and may also include filtering circuitry and an analog-to-digital (A/D) converter. The sensed cardiac signals processed by the sensing circuitry 1204 may be received by noise reduction circuitry 1203, which may further reduce noise before signals are sent to the detection circuitry 1202.

Noise reduction circuitry 1203 may also be incorporated after sensing circuitry 1204 in cases where high power or computationally intensive noise reduction algorithms are required. The noise reduction circuitry 1203, by way of amplifiers used to perform operations with the electrode signals, may also perform the function of the sensing circuitry 1204. Combining the functions of sensing circuitry 1204 and noise reduction circuitry 1203 may be useful to minimize the necessary componentry and lower the power requirements of the system.

In the illustrative configuration shown in Figure 12, the detection circuitry 1202 is coupled to, or otherwise incorporates, noise reduction circuitry 1203. The noise reduction circuitry 1203 operates to improve the SNR of sensed cardiac signals by removing noise content of the sensed cardiac signals introduced from various sources. Typical types of cardiac signal noise include electrical noise and noise produced from skeletal muscles, for example. A number of methodologies for improving the SNR of sensed cardiac signals in the presence of skeletal muscular induced noise, including signal separation techniques incorporating combinations of electrodes and multi-element electrodes, are described hereinbelow.

Detection circuitry 1202 may include a signal processor that coordinates analysis of the sensed cardiac signals and/or other sensor inputs to detect cardiac arrhythmias, such as, in particular, tachyarrhythmia. Rate based and/or morphological discrimination algorithms may be implemented by the signal processor of the detection circuitry 1202 to detect and verify the presence and severity of an arrhythmic episode. Examples of arrhythmia detection and discrimination circuitry, structures, and techniques, aspects of which may be implemented by a PIMD of a type that may benefit from cardiac activation sequence monitoring and/or tracking methods and implementations are disclosed in commonly owned U.S. Patent Nos. 5,301,677, 6,438,410, and 6,708,058, which are hereby incorporated herein by reference. Arrhythmia detection methodologies particularly well suited for implementation in cardiac monitoring and/or stimulation systems are described hereinbelow.

The detection circuitry 1202 communicates cardiac signal information to the control system 1205. Memory circuitry 1209 of the control system 1205 contains parameters for operating in various monitoring, defibrillation, and, if applicable, pacing modes, and stores data indicative of cardiac signals received by the detection circuitry 1202. The memory circuitry 1209 may also be configured to store historical ECG and therapy data, which may be used for various purposes and transmitted to an external receiving device as needed or desired.

In certain configurations, the PIMD may include diagnostics circuitry 1210. The - diagnostics circuitry 1210 typically receives input signals from the detection circuitry 1202 and the sensing circuitry 1204. The diagnostics circuitry 1210 provides diagnostics data to the control system 1205, it being understood that the control system 1205 may incorporate all or part of the diagnostics circuitry 1210 or its functionality. The control system 1205 may store and use information provided by the diagnostics circuitry 1210 for a variety of diagnostics purposes. This diagnostic information may be stored, for example, subsequent to a triggering event or at predetermined intervals, and may include system diagnostics, such as power source status, therapy delivery history, and/or patient diagnostics. The diagnostic information may take the form of electrical signals or other sensor data acquired immediately prior to therapy delivery.

According to a configuration that provides cardioversion and defibrillation therapies, the control system 1205 processes cardiac signal data received from the detection circuitry 1202 and initiates appropriate tachyarrhythmia therapies to terminate cardiac arrhythmic episodes and return the heart to normal sinus rhythm. The control system 1205 is coupled to shock therapy circuitry 1216. The shock therapy circuitry 1216 is coupled to the electrode(s) 1214 and the can or indifferent electrode 1207 of the PIMD housing.

Upon command, the shock therapy circuitry 1216 delivers cardioversion and defibrillation stimulation energy to the heart in accordance with a selected cardioversion or defibrillation therapy. In a less sophisticated configuration, the shock therapy circuitry 1216 is controlled to deliver defibrillation therapies, in contrast to a configuration that provides for delivery of both cardioversion and defibrillation therapies. Examples of PIMD high energy delivery circuitry, structures and functionality, aspects of which may be incorporated in a PIMD of a type that may benefit from aspects of the present invention are disclosed in commonly owned U.S. Patent Nos. 5,372,606; 5,411,525; 5,468,254; and 5,634,938, which are hereby incorporated herein by reference.

Arrhythmic episodes may also be detected and verified by morphology-based analysis of sensed cardiac signals as is known in the art. Tiered or parallel arrhythmia discrimination algorithms may also be implemented using both rate-based and morphologic-based approaches. Further, a rate and pattern-based arrhythmia detection and discrimination approach may be employed to detect and/or verify arrhythmic episodes, such as the approach disclosed in U.S. Patent Nos. 6,487,443; 6,259,947; 6,141,581; 5,855,593; and 5,545,186, which are hereby incorporated herein by reference.

In accordance with another configuration, a PIMD may incorporate a cardiac pacing capability in addition to, or to the exclusion of, cardioversion and/or defibrillation capabilities. As is shown in Figure 12, the PIMD includes pacing therapy circuitry 1230 that is coupled to the control system 1205 and the electrode(s) 1214 and can/indifferent electrodes 1207. Upon command, the pacing therapy circuitry 1230 delivers pacing pulses to the heart in accordance with a selected pacing therapy.

Control signals, developed in accordance with a pacing regimen by pacemaker circuitry within the control system 1205, are initiated and transmitted to the pacing therapy circuitry 1230 where pacing pulses are generated. A pacing regimen, such as those discussed and incorporated herein, may be modified by the control system 1205. In one particular application, a sense vector optimization approach of the present invention may be implemented to enhance capture detection and/or capture threshold determinations, such as by selecting an optimal vector for sensing an evoked response resulting from application of a capture pacing stimulus.

The PIMD shown in Figure 12 may be configured to receive signals from one or more physiologic and/or non-physiologic sensors. Depending on the type of sensor employed, signals generated by the sensors may be communicated to transducer circuitry coupled directly to the detection circuitry 1202 or indirectly via the sensing circuitry 1204. It is noted that certain sensors may transmit sense data to the control system 1205 without processing by the detection circuitry 1202.

Communications circuitry 1218 is coupled to the microprocessor 1206 of the control system 1205. The communications circuitry 1218 allows the PIMD to communicate with one or more receiving devices or systems situated external to the PIMD. By way of example, the PIMD may communicate with a patient-worn, portable or bedside communication system via the communications circuitry 1218. In one configuration, one or more physiologic or non-physiologic sensors (subcutaneous, cutaneous, or external of patient) may be equipped with a short-range wireless communication interface, such as an interface conforming to a known communications standard, such as Bluetooth or IEEE 802 standards. Data acquired by such sensors may be communicated to the PIMD via the communications circuitry 1218. It is noted that physiologic or non-physiologic sensors equipped with wireless transmitters or transceivers may communicate with a receiving system external of the patient.

The communications circuitry 1218 allows the PIMD to communicate with an external programmer. In one configuration, the communications circuitry 1218 and the programmer unit (not shown) use a wire loop antenna and a radio frequency telemetric link, as is known in the art, to receive and transmit signals and data between the programmer unit and communications circuitry 1218. In this manner, programming commands and data are transferred between the PIMD and the programmer unit during and after implant. Using a programmer, a physician is able to set or modify various parameters used by the PIMD. For example, a physician may set or modify parameters affecting monitoring, detection, pacing, and defibrillation functions of the PIMD, including pacing and cardioversion/defibrillation therapy modes.

Typically, the PIMD is encased and hermetically sealed in a housing suitable for implanting in a human body as is known in the art. Power to the PIMD is supplied by an electrochemical power source 1220 housed within the PIMD. In one configuration, the power source 1220 includes a rechargeable battery. According to this configuration, charging circuitry is coupled to the power source 1220 to facilitate repeated non-invasive charging of the power source 1220. The communications circuitry 1218, or separate receiver circuitry, is configured to receive RF energy transmitted by an external RF energy transmitter. The PIMD may, in addition to a rechargeable power source, include a non-rechargeable battery. It is understood that a rechargeable power source need not be used, in which case a long-life non-rechargeable battery is employed.

The detection circuitry 1202, which is coupled to a microprocessor 1206, may be configured to incorporate, or communicate with, specialized circuitry for processing sensed cardiac signals in manners particularly useful in a cardiac sensing and/or stimulation device. As is shown by way of example in Figure 12, the detection circuitry 1202 may receive information from multiple physiologic and non-physiologic sensors.

The detection circuitry 1202 may also receive information from one or more sensors that monitor skeletal muscle activity. In addition to cardiac activity signals, electrodes readily detect skeletal muscle signals. Such skeletal muscle signals may be used to determine the activity level of the patient. In the context of cardiac signal detection, such skeletal muscle signals are considered artifacts of the cardiac activity signal, which may be viewed as noise.

The components, functionality, and structural configurations depicted herein are intended to provide an understanding of various features and combination of features that may be incorporated in a PIMD. It is understood that a wide variety of PIMDs and other implantable cardiac monitoring and/or stimulation device configurations are contemplated, ranging from relatively sophisticated to relatively simple designs. As such, particular PIMD or cardiac monitoring and/or stimulation device configurations may include particular features as described herein, while other such device configurations may exclude particular features described herein.

The PIMD may detect a variety of physiological signals that may be used in connection with various diagnostic, therapeutic or monitoring implementations. For example, the PIMD may include sensors or circuitry for detecting respiratory system signals, cardiac system signals, and signals related to patient activity. In one embodiment, the PIMD senses intrathoracic impedance, from which various respiratory parameters may be derived, including, for example, respiratory tidal volume and minute ventilation. Sensors and associated circuitry may be incorporated in connection with a PIMD for detecting one or more body movement or body posture or position related signals. For example, accelerometers and GPS devices may be employed to detect patient activity, patient location, body orientation, or torso position.

Referring now to Figure 13, a PIMD of the present invention may be used within the structure of an advanced patient management (APM) system 1300. The advanced patient management system 1300 allows physicians to remotely and automatically monitor cardiac and respiratory functions, as well as other patient conditions. In one example, a PIMD implemented as a cardiac pacemaker, defibrillator, or resynchronization device may be equipped with various telecommunications and information technologies that enable real-time data collection, diagnosis, and treatment of the patient. Various PIMD embodiments described herein may be used in connection with advanced patient management. Methods, structures, and/or techniques described herein, which may be adapted to provide for remote patient/device monitoring, diagnosis, therapy, or other APM related methodologies, may incorporate features of one or more of the following references: US Patent Nos. 6,221,011; 6,270,457; 6,277,072; 6,280,380; 6,312,378; 6,336,903; 6,358,203; 6,368,284; 6,398,728; and 6,440,066, which are hereby incorporated herein by reference.

As is illustrated in Figure 13, the medical system 1300 may be used to implement coordinated patient measuring and/or monitoring, diagnosis, and/or therapy in accordance with embodiments of the invention. The medical system 1300 may include, for example, one or more patient-internal medical devices 1310, such as a PIMD, and one or more patient-external medical devices 1320, such as a monitor or signal display device. Each of the patient-internal 1310 and patient-external 1320 medical devices may include one or more of a patient monitoring unit 1312, 1322, a diagnostics unit 1314, 1324, and/or a therapy unit 1316, 1326.

The patient-external medical device 1320 performs monitoring, and/or diagnosis and/or therapy functions external to the patient (i.e., not invasively implanted within the patient's body). The patient-external medical device 1320 may be positioned on the patient, near the patient, or in any location external to the patient.

The patient-internal and patient-external medical devices 1310, 1320 may be coupled to one or more sensors 1341, 1342, 1345, 1346, patient input / trigger devices 1343, 1347 and/or other information acquisition devices 1344, 1348. The sensors 1341, 1342, 1345, 1346, patient input / trigger devices 1343, 1347, and/or other information acquisition devices 1344, 1348 may be employed to detect conditions relevant to the monitoring, diagnostic, and/or therapeutic functions of the patient-internal and patient-external medical devices 1310, 1320.

The medical devices 1310, 1320 may each be coupled to one or more patient-internal sensors 1341, 1345 that are fully or partially implantable within the patient. The medical devices 1310, 1320 may also be coupled to patient-external sensors positioned on, near, or in a remote location with respect to the patient. The patient-internal and patient-external sensors are used to sense conditions, such as physiological or environmental conditions, that affect the patient.

The patient-internal sensors 1341 may be coupled to the patient-internal medical device 1310 through one or more internal leads 1353. Still referring to Figure 13, one or more patient-internal sensors 1341 may be equipped with transceiver circuitry to support wireless communications between the one or more patient-internal sensors 1341 and the patient-internal medical device 1310 and/or the patient-external medical device 1320.

The patient-external sensors 1342 may be coupled to the patient-internal medical device 1310 and/or the patient-external medical device 1320 through one or more internal leads 1355 or through wireless connections. Patient-external sensors 1342 may communicate with the patient-internal medical device 1310 wirelessly. Patient-external sensors 1342 may be coupled to the patient-external medical device 1320 through one or more internal leads 1357 or through a wireless link.

In an embodiment of the present invention, the patient-external medical device 1320 includes a visual display configured to concurrently display non-electrophysiological signals and ECG signals. For example, the display may present the information visually. The patient-external medical device 1320 may also, or alternately, provide signals to other components of the medical system 1300 for presentation to a clinician, whether local to the patient or remote to the patient.

Referring still to Figure 13, the medical devices 1310, 1320 may be connected to one or more information acquisition devices 1344, 1348, such as a database that stores information useful in connection with the monitoring, diagnostic, or therapy functions of the medical devices 1310, 1320. For example, one or more of the medical devices 1310, 1320 may be coupled through a network to a patient information server 1330.

The input/trigger devices 1343, 1347 are used to allow the physician, clinician, and/or patient to manually trigger and/or transfer information to the medical devices 1310, 1320. The input/trigger devices 1343, 1347 may be particularly useful for inputting information concerning patient perceptions, such as a perceived cardiac event, how well the patient feels, and other information not automatically sensed or detected by the medical devices 1310, 1320. For example, the patient may trigger the input/trigger device 1343 upon perceiving a cardiac event. The trigger may then initiate the recording of cardiac signals and/or other sensor signals in the patient-internal device 1310. Later, a clinician may trigger the input/trigger device 1347, initiating the transfer of the recorded cardiac and/or other signals from the patient-internal device 1310 to the patient-external device 1320 for display and diagnosis. The input/trigger device 1347 may also be used by the patient, clinician, and/or physician as an activation stimulus to the PIMD to update and/or select a vector.

In one embodiment, the patient-internal medical device 1310 and the patient-external medical device 1320 may communicate through a wireless link between the medical devices 1310, 1320. For example, the patient-internal and patient-external devices 1310, 1320 may be coupled through a short-range radio link, such as Bluetooth, IEEE 802.11, and/or a proprietary wireless protocol. The communications link may facilitate uni-directional or bi-directional communication between the patient-internal 1310 and patient-external 1320 medical devices. Data and/or control signals may be transmitted between the patient-internal 1310 and patient-external 1320 medical devices to coordinate the functions of the medical devices 1310, 1320.

In another embodiment, patient data may be downloaded from one or more of the medical devices periodically or on command, and stored at the patient information server 1330. The physician and/or the patient may communicate with the medical devices and the patient information server 1330, for example, to acquire patient data or to initiate, terminate or modify recording and/or therapy.

The data stored on the patient information server 1330 may be accessible by the patient and the patient's physician through one or more terminals 1350, e.g., remote computers located in the patient's home or the physician's office. The patient information server 1330 may be used to communicate to one or more of the patient-internal and patient-external medical devices 1310, 1320 to provide remote control of the monitoring, diagnosis, and/or therapy functions of the medical devices 1310, 1320.

In one embodiment, the patient's physician may access patient data transmitted from the medical devices 1310, 1320 to the patient information server 1330. After evaluation of the patient data, the patient's physician may communicate with one or more of the patient-internal or patient-external devices 1310, 1320 through an APM system 1340 to initiate, terminate, or modify the monitoring, diagnostic, and/or therapy functions of the patient-internal and/or patient-external medical systems 1310, 1320.

In another embodiment, the patient-internal and patient-external medical devices 1310, 1320 may not communicate directly, but may communicate indirectly through the APM system 1340. In this embodiment, the APM system 1340 may operate as an intermediary between two or more of the medical devices 1310, 1320. For example, data and/or control information may be transferred from one of the medical devices 1310, 1320 to the APM system 1340. The APM system 1340 may transfer the data and/or control information to another of the medical devices 1310, 1320.

In one embodiment, the APM system 1340 may communicate directly with the patient-internal and/or patient-external medical devices 1310, 1320. In another embodiment, the APM system 1340 may communicate with the patient-internal and/or patient-external medical devices 1310, 1320 through medical device programmers 1360, 1370 respectively associated with each medical device 1310, 1320. As was stated previously, the patient-internal medical device 1310 may take the form of an implantable PIMD.

In accordance with one approach of the present invention, a PIMD may be implemented to separate cardiac signals for selection and monitoring of vectors in a robust manner using a blind source separation (BSS) technique. It is understood that all or certain aspects of the BSS technique described below may be implemented in a device or system (implantable or non-implantable) other than a PIMD, and that the description of BSS techniques implemented in a PIMD is provided for purposes of illustration, and not of limitation. For example, algorithms that implement a BSS technique as described below may be implemented for use by an implanted processor or a non-implanted processor, such as a processor of a programmer or computer of a patient-external device communicatively coupled to the PIMD.

Referring now to Figures 14 through 16, cardiac monitoring and/or stimulation devices and methods employing cardiac signal separation are described in accordance with the present invention. The PIMD may be implemented to separate signal components according to their sources and produce one or more cardiac signal vectors associated with all or a portion of one or more cardiac activation sequences based on the source separation. To achieve this, the methods and algorithms illustrated in Figures 14 through 16 may be implemented.

Figure 14 illustrates a portion of a cardiac activation sequence monitoring and/or tracking system 1425 in accordance with the present invention. A process 1414 is performed, providing a selected vector 1419 along with vector information including, for example, magnitude, angle, rates of change, trend information, and other statistics. The selected vector 1419 (and associated signal and other vector information) is available for a variety of uses 1420, such as, for example, arrhythmia discrimination, therapy titration, posture detection/monitoring, ischemia detection/monitoring, capture verification, disease diagnosis and/or progress information, or other use. In accordance with the present invention, the process may be used, and repeated, to monitor cardiac activation sequences, track changes in the progression of patient pathology, and to update sense vectors useful for cardiac sensing and/or stimulation, for example.

Figure 15 illustrates an embodiment of a signal source separation/update process 1500 useful for cardiac activation sequence monitoring and/or tracking in accordance with the present invention. A set of composite signals, including at least two and up to n signals, are selected for separation, where n is an integer. Each electrode provides a composite signal associated with an unknown number of sources. Pre-processing and/or pre-filtering 1612 may be performed on each of the composite signals. It may be advantageous to filter each composite signal using the same filtering function. Source separation 1614 is performed, providing at least one separated signal. If a treatment is desired, an appropriate treatment or therapy 1618 is performed. If continued source separation is desired, the process returns to perform such source separation 1614 and may iteratively separate 1616 more signals until a desired signal is found, or all signals are separated.

The separated signal or signals may then be used 1620 for some specified purpose, such as, for example, to confirm a normal sinus rhythm, determine a cardiac condition, define a noise signal, monitor cardiac activation sequence, determine patient posture, diagnose or monitor a disease state, or other desired use. Electrode arrays and/or the use of multiple electrodes provide for many possible vectors useful for sensing cardiac activity.

Updating the vector to monitor and/or track changes may be performed periodically, on demand, at a predetermined time, upon the occurrence of a predetermined event, continuously, or as otherwise desired. For example, a PIMD may regularly perform an update of the sense vector used for cardiac discrimination, to keep performance of the PIMD improved and/or adjusted and/or optimized and/or to track or monitor progression of changes. Updating may be useful, for example, when pathology, therapy, posture, or other system or patient change suggests a change in vector may be detected and/or useful.

For example, in an APM environment such as described previously, a PIMD in accordance with the present invention may have a controller and communications circuitry that transmits its cardiac composite signals to a bedside signal processor when the patient is asleep. The signal processor may perform a blind source separation and analysis of the composite signals during the patient's sleep cycle. The signal processor may then determine the appropriate vector or vectors for the PIMD, and reprogram the PIMD before the patient awakes. The PIMD may then operate with the latest programming until the next update.

Figure 16 illustrates further embodiments of a signal source separation process in greater detail, including some optional elements. Entry of the process at block 1622 provides access to a pre-processing facility 1612, illustrated here as including a covariance matrix computation block 1624 and/or a pre-filtering block 1626 such as, for example, a band-pass filtering block. The composite signals processed at pre-processing block 1612 are provided to a signal source separation block 1615, which may include functionality of the source separation block 1614 and iterative source separation block 1616 shown in Figure 15.

The signal source separation block 1615 includes a principal component analysis block 1628, which produces an associated set of eigenvectors and eigenvalues using a covariance matrix or composite signals provided by pre-processing block 1612. A determination 1630 is made as to whether one eigenvalue is significantly larger than any others in the set, making the dimension associated with this eigenvalue a likely candidate for association with the direction along which the power of the signal is maximized. If such a candidate is identified at block 1630, the candidate signal may immediately be separated 1631 and a determination 1633 made to confirm whether the candidate signal is a cardiac signal, before returning 1644 to the master PIMD routine that called the signal source separation process.

If there is no clear candidate eigenvalue, or if the largest value eigenvalue did not provide a signal of interest, an iterative process may be used to separate 1632 and search 1636 for the signal of interest (e.g., cardiac signal). This process 1632, 1636, 1634 may be repeated until such a signal is found, or no more signals are separable 1634 as determined by exceeding a predefined number of iterations $N_{max}$ or some other termination criterion. An example of such a criterion is an eigenvalue considered at the current iteration being proportionately smaller than the largest eigenvalues by some predetermined amount.

If the iterations 1634 are completed and a cardiac signal is not found at 1636, then an Independent component analysis 1635 may be attempted to further process the signals in an attempt to find the cardiac signal. If a cardiac signal is still not found at decision 1637, after exhausting all possibilities, then a set of default settings 1639 may be used, or an error routine may be initiated.

In another embodiment of the present invention, a method of signal separation involves sensing, at least in part implantably, two or more composite signals using three or more cardiac electrodes or electrode array elements. The method may further involve performing a source separation using the detected composite signals, the source separation producing two or more vectors. A first vector and a second vector may be selected from the set of vectors.

The use of the terms first and second vector are not intended to imply that the vectors are the first and second vectors separated from the composite signal, but that a first vector and a second vector are selected from among any vectors available for a given composite signal. First and second signals may be identified from the detected two or more composite signals using the first and second vectors respectively. The method then involves selecting either the first vector or the second vector as a selected vector based on a selection criterion.

Selection criteria may include finding the optimum vector for cardiac signal identification, finding a vector that provides the largest magnitude cardiac signal, or finding another particular signal of interest. For example, the first vector may be selected and used for cardiac activity monitoring, and the second vector may then be selected and used for skeletal muscle activity monitoring. The skeletal muscle signal may then be used to further discriminate arrhythmias from noise such as is further described in commonly owned US Patent Application Serial No: 10/816,464 entitled "Subcutaneous Cardiac Stimulation System with Patient Activity Sensing," filed April 1, 2004, which is hereby incorporated herein by reference.

With continued reference to Figures 14 through 16, one illustrative signal source separation methodology useful with the present invention is described below. Such an approach is particularly well suited for use in a PIMD system. It is to be understood that the example provided below is provided for non-limiting, illustrative purposes only. Moreover, it is understood that signal source separation within the context of the present invention need not be implemented using the specific processes described below, or each and every process described below.

A collected signal may be pre-filtered to suppress broadly incoherent noise and to generally optimize the signal-to-noise ratio (SNR). Any noise suppression in this step has the additional benefit of reducing the effective number of source signals that need to be separated. A Principal Component Analysis (PCA) may be performed on the collected and/or pre-filtered signal, producing a set of eigenvectors and associated eigenvalues describing the optimal linear combination, in a least-squares sense, of the recorded signals that makes the components coming from different sources orthogonal to one another. As an intermediate step to performing the PCA, an estimate of the spatial covariance matrix may be computed and averaged over a relatively short time interval (on the order of 2-3 beats), or over the windowed signal as described previously, to enhance those components that are mutually correlated.

Each eigenvalue corresponds to the power of the signal projected along the direction of each associated eigenvector. The cardiac signal component is typically identified by one of the largest eigenvalues. Occasionally, PCA does not achieve a substantially sufficient level of source independence. In such a case, an Independent Component Analysis (ICA) may be performed to determine the actual source direction, either upon the PCA-transformed signal, or directly upon the collected signal. The ICA consists of a unitary transformation based on higher-order statistical analysis.

For example, separation of two mixed sources may be achieved by rotating the complex variable formed from the signals on an angle that aligns their probability distributions with basis vectors. In another approach, an algorithm based on minimization of mutual information between components, as well as other approaches generally known in the field of ICA, may be used to achieve reconstructed source independence.

A PIMD may, for example, employ a hierarchical decision-making procedure that initiates a blind source separation algorithm upon the detection of a condition under which the target vector may change. By way of example, a local peak density algorithm or a curvature-based significant point methodology may be used as a high-level detection routine. Other sensors/information available to the PIMD may also trigger the initiation of a blind source separation algorithm.

The PIMD may compute an estimate of the covariance matrix. It may be sufficient to compute the covariance matrix for only a short time. Computation of the eigenvalues and eigenvectors required for the PCA may also be performed adaptively through an efficient updating algorithm.

The cardiac signal may be identified among the few (e.g., two or three) largest separated signals. One of several known algorithms may be used. For example, local peak density (LPD) or beat detection (BD) algorithms may be used. The LPD algorithm may be used to identify the cardiac signal by finding a signal that has an acceptable physiologic range of local peak densities by comparing the LPD to a predetermined range of peak densities known to be acceptable. The BD algorithm finds a signal that has a physiologic range of beat rate. In the case where two signals look similar, a morphology algorithm may be used for further discrimination. It may be beneficial to use the same algorithm at different levels of hierarchy: 1) initiation of blind source separation algorithm; 2) iterative identification of a cardiac signal.

Mathematical development of an example of blind source separation algorithm in accordance with the present invention is provided as follows. Assume there are $m$ source signals $s_1(t),...,s_m(t)$ that are detected inside of the body, including a desired cardiac signal and some other independent noise, which may, for example, include myopotential noise, electrocautery response, etc. These signals are recorded simultaneously from k sensing vectors derived from subcutaneous sensing electrodes, where all m signals may be resolved if k>m. By definition, the signals are mixed together into the overall voltage gradient sensed across the electrode array. In addition, there is usually an additive noise attributable, for example, to environmental noise sources. The relationship between the source signals s(t) and recorded signals x(t) is described below:

$$\begin{pmatrix} x_1(t) \\ x_2(t) \\ \vdots \\ x_k(t) \end{pmatrix} = \begin{pmatrix} y_1(t) \\ y_2(t) \\ \vdots \\ y_k(t) \end{pmatrix} + \begin{pmatrix} n_1(t) \\ n_2(t) \\ \vdots \\ n_k(t) \end{pmatrix} = \begin{pmatrix} a_{11} & a_{12} & ... & a_{1m} \\ a_{21} & a_{22} & ... & a_{2m} \\ \vdots & \vdots & \ddots & \vdots \\ a_{k1} & a_{k2} & ... & a_{km} \end{pmatrix} \begin{pmatrix} s_1(t) \\ s_2(t) \\ \vdots \\ s_m(t) \end{pmatrix} + \begin{pmatrix} n_1(t) \\ n_2(t) \\ \vdots \\ n_k(t) \end{pmatrix}$$

$$= x(t) = y(t) + n(t) = As(t) + n(t), \qquad m < k$$

Here, x(t) is an instantaneous linear mixture of the source signals and additive noise, y(t) is the same linear mixture without the additive noise, n(t) is environmental noise modeled as Gaussian noise, $A$ is an unknown mixing matrix, and s(t) are the unknown source signals considered here to include the desired cardiac signal and other biological artifacts. There is no assumption made about the underlying structure of the mixing matrix and the source signals, except for their spatial statistical independence. The objective is to reconstruct the source signals s(t) from the recorded signals x(t).

Reconstruction of the source signals s(t) from the recorded signals x(t) may involve pre-filtering x(t) to optimize the SNR (i.e., maximize the power of s(t) against that of n(t)). Here, a linear phase filter may be used to minimize time-domain dispersion (tails and ringing) and best preserve the underlying cardiac signal morphology. It is noted that the notation x(t) is substituted for the pre-filtered version of x(t).

An estimate of the spatial covariance matrix $R$ is formed as shown immediately below. This step serves to enhance the components of the signal that are mutually correlated and downplays incoherent noise.

$$R = \frac{1}{T_{(\sim 1\mathrm{sec})}} \sum_{t=1,T} \begin{pmatrix} x_1(t) \\ x_2(t) \\ \cdots \\ x_k(t) \end{pmatrix} * \begin{pmatrix} x_1(t) & x_2(t) & \cdots & x_k(t) \end{pmatrix} =$$

$$\frac{1}{T_{(\sim 1\mathrm{sec})}} \sum_{t=1,T} \begin{bmatrix} x_1(t)*x_1(t) & x_1(t)*x_2(t) & \cdots & x_1(t)*x_k(t) \\ x_2(t)*x_1(t) & x_2(t)*x_2(t) & \cdots & x_2(t)*x_k(t) \\ \cdots & \cdots & \ddots & \cdots \\ x_k(t)*x_1(t) & x_k(t)*x_2(t) & \cdots & x_k(t)*x_k(t) \end{bmatrix}$$

Eigenvalues and eigenvectors of the covariance matrix $R$ may be determined using singular value decomposition (SVD). By definition, the SVD factors $R$ as a product of three matrices $R = USV^T$, where $U$ and $V$ are orthogonal matrices describing amplitude preserving rotations, and $S$ is a diagonal matrix that has the squared eigenvalues $\sigma_1...\sigma_k$ on the diagonal in monotonically decreasing order. Expanded into elements, this SVD may be expressed as follows.

$$R = \begin{pmatrix} u_{11} & u_{12} & \cdots & u_{1k} \\ u_{21} & u_{22} & \cdots & u_{2k} \\ \vdots & \vdots & \ddots & \vdots \\ u_{k1} & u_{k2} & \cdots & u_{kk} \end{pmatrix} \begin{pmatrix} \sigma_1 & 0 & 0 & 0 \\ 0 & \sigma_2 & 0 & 0 \\ \vdots & \vdots & \ddots & \vdots \\ 0 & 0 & \cdots & \sigma_k \end{pmatrix} \begin{pmatrix} v_{11} & v_{12} & \cdots & v_{1k} \\ v_{21} & v_{22} & \cdots & v_{2k} \\ \vdots & \vdots & \ddots & \vdots \\ v_{k1} & v_{k2} & \cdots & v_{kk} \end{pmatrix}$$

The columns of matrix $V$ consist of eigenvectors that span a new coordinate system wherein the components coming from different sources are orthogonal to one another. Eigenvalues $\sigma_1...\sigma_k$ correspond respectively to columns 1...k of $V$. Each eigenvalue defines the signal "power" along the direction of its corresponding eigenvector. The matrix $V$ thus provides a rotational transformation of x(t) into a space where each separate component of x is optimally aligned, in a least-squares sense, with a basis vector of that space.

The largest eigenvalues correspond to the highest power components, which typically represent the mixed source signals $y_1(t),...,y_m(t)$. The lower eigenvalues typically are associated with additive noise $n_1(t),...,n_{k-m}(t)$. Each eigenvector may then be viewed as an optimal linear operator on x that maximizes the power of the corresponding independent signal component. As a result, the transformed signal is found as:

$$\hat{y}(t) = \begin{pmatrix} \hat{y}_1(t) \\ \vdots \\ \hat{y}_m(t) \end{pmatrix} = \begin{pmatrix} v_{11} & v_{21} & \cdots & v_{k1} \\ \vdots & \vdots & \ddots & \vdots \\ v_{1m} & v_{2m} & \cdots & v_{km} \end{pmatrix} * \begin{pmatrix} x_1(t) \\ x_2(t) \\ \vdots \\ x_k(t) \end{pmatrix}$$

The component estimates $\hat{y}_1(t),...,\hat{y}_m(t)$ of $y_1(t),...,y_m(t)$ are aligned with the new orthogonal system of coordinates defined by eigenvectors. As a result, they should be orthogonal to each other and thus independent.

In an alternative implementation, eigenvalues and eigenvectors of the covariance matrix $R$ may be determined using eigenvalue decomposition (ED). By definition, the ED solves the matrix equation $RV = SV$ so that $S$ is a diagonal matrix having the eigenvalues $\sigma_1...\sigma_k$ on the diagonal, in monotonically decreasing order, and so that matrix $V$ contains the corresponding eigenvectors along its columns. The resulting eigenvalues and associated eigenvectors may be applied in similar manner to those resulting from the SVD of covariance matrix $R$.

In an alternative implementation, eigenvalues and eigenvectors are computed directly from x(t) by forming a rectangular matrix $X$ of k sensor signals collected during a time sECGent of interest, and performing an SVD directly upon $X$. The matrix $X$ and its decomposition may be expressed as follows.

$$X = \begin{pmatrix} x_1(t) \\ x_2(t) \\ \vdots \\ x_k(t) \end{pmatrix} = \begin{pmatrix} x_1(t_1) & x_1(t_2) & \dots & x_1(t_T) \\ x_2(t_1) & x_2(t_2) & \cdots & x_2(t_T) \\ \vdots & \vdots & \ddots & \vdots \\ x_k(t_1) & x_k(t_2) & \cdots & x_k(t_T) \end{pmatrix} = USV^T$$

Note that in cases where T > k, a so-called "economy-size" SVD may be used to fmd the eigenvalues and eigenvectors efficiently. Such an SVD may be expressed as follows, expanded into elements.

$$X = USV^T = \begin{pmatrix} u_{11} & u_{12} & \dots & u_{1T} \\ u_{21} & u_{22} & \dots & u_{2T} \\ \vdots & \vdots & \ddots & \vdots \\ u_{k1} & u_{k2} & \dots & u_{kT} \end{pmatrix} \begin{pmatrix} \sigma_1 & 0 & \dots & 0 \\ 0 & \sigma_2 & \dots & 0 \\ \vdots & \vdots & \ddots & \vdots \\ 0 & 0 & \dots & \sigma_k \end{pmatrix} \begin{pmatrix} v_{11} & v_{12} & \dots & v_{1k} \\ v_{21} & v_{22} & \dots & v_{2k} \\ \vdots & \vdots & \ddots & \vdots \\ v_{k1} & v_{k2} & \dots & v_{kk} \end{pmatrix}$$

A similar economy-sized SVD may also be used for the less typical case where k > T. The matrices *S* and *V* resulting from performing the SVD of data matrix *X* may be applied in the context of this present invention identically as the matrices *S* and *V* resulting from performing the SVD on the covariance matrix *R*.

At this point, the mutual separation of $\hat{y}_1(t),...,\hat{y}_m(t)$ would be completed, based on the covariance statistics. Occasionally, information from covariance is not sufficient to achieve source independence. This happens, for example, when the cardiac signal is corrupted with electrocautery, which may cause perturbations from the linearly additive noise model. In such a case, Independent Component Analysis (ICA) may be used to further separate the signals.

The ICA seeks to find a linear transformation matrix W that inverts the mixing matrix A in such manner as to recover an estimate of the source signals. The operation may be described as follows.

$$s(t) = \begin{pmatrix} s_1(t) \\ s_2(t) \\ \vdots \\ s_{...}(t) \end{pmatrix} = W\,y(t) \approx A^{-1}y(t)$$

Here we substitute s(t) for the recovered estimate of the source signals. The signal vector *y(t)* corresponds to either the collected sensor signal vector *x(t)* or to the signal $\hat{y}(t)$ separated with PCA. The matrix *W* is the solution of an optimization problem that maximizes the independence between the components $s_1(t),...,s_m(t)$ of s(t) = *Wy(t)*. We treat the components of *s(t)* as a vector of random variables embodied in the vector notation s, so that the desired transformation would optimize some cost function $C(\mathbf{s}) = C([s_1(t),...,s_m,(t)])$ that measures the mutual independence of these components. Given the joint probability density function (pdf) *f*(**s**) and the factorized pdf $\overline{f}(\mathbf{s}) = f_1(s_1)f_2(s_2)...f_m(s_m)$, or given estimates of these pdf's, we may solve the following.

$$\min_W C(\mathbf{s}) = \min_W \int D\big(f(\mathbf{s}), \overline{f}(\mathbf{s})\big)d\mathbf{s}$$

The function $D(f(\mathbf{s}), \overline{f}(\mathbf{s}))$ may be understood as a standard distance measure generally known in the art, such as for example an absolute value difference $|f(\mathbf{s})\text{-}\overline{f}(\mathbf{s})|$, Euclidean distance $(f(\mathbf{s})\text{-}\overline{f}(\mathbf{s}))^2$, or p-norm $(f(\mathbf{s})\text{-}\overline{f}(\mathbf{s}))^p$. The distance measure approaches zero as *f*(**s**) approaches $\overline{f}(\mathbf{s})$, which by the definition of statistical independence, occurs as the components of **s** approach mutual statistical independence.

In an alternative implementation, the distance measure may take the form of a Kullback-Liebler divergence (KLD) between *f*(**s**) and $\overline{f}(\mathbf{s})$, yielding cost function optimizations in either of the following forms.

$$\min_W C(\mathbf{s}) = \min_W \int f(\mathbf{s})\log\frac{f(\mathbf{s})}{\overline{f}(\mathbf{s})}d\mathbf{s}$$

*or*

$$= \frac{\min}{W} \int \bar{f}(\mathbf{s}) \log \frac{\bar{f}(\mathbf{s})}{f(\mathbf{s})} d\mathbf{s}$$

Since the KLD is not symmetric, the two alternative measures are related but not precisely equal. One measure could be chosen, for example, if a particular underlying data distribution favors convergence with that measure.

Several alternative approaches may be used to measure the mutual independence of the components of **s.** These may include the maximum likelihood method, maximization of negentropy or its approximation, and minimization of mutual information.

In the maximum likelihood method, the desired matrix $W$ is found as a solution of the following optimization problem,

$$\max_{W} \sum_{j=1}^{T} \sum_{i=1}^{m} \log f_i(s_i(t_j)) + T \log|\det W| = \max_{W} \sum_{j=1}^{T} \sum_{i=1}^{m} \log f_i(w_i^T y(t_j)) + T \log|\det W|$$

where $w_i$ are columns of the matrix $W$. In the negentropy method, the cost function is defined in terms of differences in entropy between **s** and a corresponding Gaussian random variable, resulting in the following optimization problem,

$$\max_{W} \left\{ H(\mathbf{s}_{gauss}) - H(\mathbf{s}) \right\} = \max_{W} \left\{ -\int f(\mathbf{s}_{gauss}) \log f(\mathbf{s}_{gauss}) d\mathbf{s}_{gauss} + \int f(\mathbf{s}) \log(\mathbf{s}) d\mathbf{s} \right\}$$

where $H(\mathbf{s})$ is the entropy of random vector **s,** and $\mathbf{s}_{gauss}$ is a Gaussian random vector chosen to have a covariance matrix substantially the same as that of **s.**

In the minimization of mutual information method, the cost function is defined in terms of the difference between the entropy of **s** and the sum of the individual entropies of the components of **s,** resulting in the following optimization problem

$$\min_{W} \left\{ -\sum_{i=1}^{m} \int f(s_i) \log f(s_i) ds_i + \int f(\mathbf{s}) \log f(\mathbf{s}) d\mathbf{s} \right\}$$

All preceding cost function optimizations having an integral form may be implemented using summations by approximating the underlying pdf's with discrete pdf's, for example as the result of estimating the pdf using well-known histogram methods. We note that knowledge of the pdf, or even an estimate of the pdf, may be difficult to implement in practice due either to computational complexity, sparseness of available data, or both. These difficulties may be addressed using cost function optimization methods based upon kurtosis, a statistical parameter that does not require a pdf.

In an alternative method a measure of independence could be expressed via kurtosis, equivalent to the fourth-order statistic defined as the following for the $i^{th}$ component of **s**

$$kurt(s_i) = E\{s_i^4\} - 3(E\{s_i^2\})^2$$

In this case W is found as a matrix that maximizes kurtosis of **s**=$W$**y** over all the components of **s** (understanding **y** to be a vector of random variables corresponding to the components of $y(t)$). In all the previous examples of ICA optimization the solution $W$ could be found via numerical methods such as steepest descent, Newton iteration, etc., well known and established in the art. These methods could prove numerically intensive to implement in practice, particularly if many estimates of statistics in s must be computed for every iteration in $W$.

Computational complexity may be addressed several ways. To begin, the ICA could be performed on the PCA-separated signal $\hat{y}(t)$ with the dimensionality reduced to only the first few (or in the simplest case, two) principal components. For situations where two principal components are not sufficient to separate the sources, the ICA could still be performed pairwise on two components at a time, substituting component pairs at each iteration of $W$ (*or* group of iterations of $W$).

In one example, a simplified two-dimensional ICA may be performed on the PCA separated signals. In this case, a unitary transformation could be found as a Givens rotation matrix with rotation angle $\theta$,

$$W(\theta) = \begin{pmatrix} \cos\theta & \sin\theta \\ -\sin\theta & \cos\theta \end{pmatrix}$$

where $s(t) = W(\theta)y(t)$. Here $W(\theta)$ maximizes the probability distribution of each component along the basis vectors, such that the following is satisfied.

$$\theta = \arg\max_{\theta} \sum_{t=1}^{T} \log f(s(t)|\theta)$$

This optimal rotation angle may be found by representing vectors y(t) and s(t) as

$$\xi = e^{i4\theta} E(\rho^4 e^{i4\varphi'}) = e^{i4\theta} E[(s_1 + is_2)^4] = e^{i4\theta}(\kappa_{40}^s + \kappa_{04}^s)$$

complex variables in the polar coordinate form $y = y_1 + iy_2 = \rho e^{i\varphi}$, $s = s_1 + is_2 = \rho e^{i\varphi'}$ and finding the relationships between their angles $\varphi, \varphi' : \varphi = \varphi' + \theta$, where $\theta$ is the rotation that relates the vectors. Then, the angle $\theta$ may be found from the fourth order-statistic of a complex variable $\xi$, where $\kappa^s$ is kurtosis, of the signal s(t).
By definition, source kurtosis is unknown, but may be found based on the fact that the amplitude of the source signal and mixed signals are the same. As a result,

$$4\theta = \hat{\xi}\, sign(\hat{\gamma})$$

with

$$\gamma = E[\rho^4] - 8 = \kappa_{40}^s + \kappa_{04}^s \text{ and } \rho^2 = s_1^2 + s_2^2 = y_1^2 + y_2^2$$

In summary, the rotation angle may be estimated as:

$$\theta = \frac{1}{4} angle(\hat{\xi}\, sign(\hat{\gamma}))$$

where

$$\hat{\xi} = \frac{1}{T}\sum_{t=1,T} \rho_t^4 e^{i4\varphi(t)} = \frac{1}{T}\sum_{t=1,T}(y_1(t) + iy_2(t))^4,$$

$$\hat{\gamma} = \frac{1}{T}\sum_{t=1,T}\rho_t^4 - 8 = \frac{1}{T}\sum_{t=1,T}(y_1^2(t) + iy_2^2(t))^4 - 8$$

After the pre-processing step, the cardiac signal is normally the first or second most powerful signal. In addition, there is usually in practice only one source signal that is temporally white. In this case, rotation of the two-dimensional vector $y = y_1 + iy_2 = \rho e^{i\varphi}$ is all that is required. In the event that more than two signals need to be separated, the Independent Component Analysis process may be repeated in pair-wise fashion over the m(m-1)/2 signal pairs until convergence is reached, usually taking about $\left(1 + \sqrt{m}\right)$ iterations.

A PIMD that implements the above-described processes may robustly separate the cardiac signal from a low SNR signal recorded from the implantable device. Such a PIMD robustly separates cardiac signals from noise to allow for improved sensing of cardiac rhythms and arrhythmias.

The system operates by finding a combination of the spatially collected low SNR signals that makes cardiac signal and noise orthogonal to each other (independent). This combination achieves relatively clean extraction of the cardiac signal even from negative SNR conditions.

A PIMD may operate in a batch mode or adaptively, allowing for on-line or offline implementation. To save power, the system may include the option for a hierarchical decision-making routine that uses algorithms known in the art for identifying presence of arrhythmias or noise in the collected signal and initiating the methods of the present invention.

Various modifications and additions can be made to the preferred embodiments discussed hereinabove without departing from the scope of the present invention. Accordingly, the scope of the present invention should not be limited by the particular embodiments described above, but should be defined only by the claims.

**Claims**

1.    An implantable cardiac system, comprising:

>    means for sensing, at least in-part within a patient, a plurality of composite signals using a plurality of cardiac electrodes; and
>    means for performing a source separation using the sensed plurality of composite signals, the source separation producing one or more cardiac signal vectors associated with all or a portion of one or more cardiac activation sequences; wherein the means for performing the source separation includes means for performing an initial source separation and a subsequent source separation; and
>    means for detecting one or more of an angle change and a trajectory change in a characteristic of the one or more cardiac signal vectors relative to a baseline.

2.    The system of claim 1, comprising at least one means for detecting a cardiac disease or condition using the change in the characteristic, or means for detecting anomalous cardiac electrical activity using the change in the characteristic.

3.    The system of claim 1, wherein the detecting means further comprises means for detecting one or more of a magnitude change of the one or more cardiac signal vectors, a variance change of the one or more cardiac signal vectors, a power spectral density change of the angle of the one or more cardiac signal vectors, a power spectral density change of the magnitude of the one or more cardiac signal vectors, an activation velocity change, and a temporal profile change of the one or more cardiac signal vectors, a rate of change of angle of the one or more cardiac signal vectors, a rate of change of magnitude of the one or more cardiac signal vectors, a rate of change of variance of the one or more cardiac signal vectors, a rate of change of velocity of the one or more cardiac signal vectors, a rate of change of temporal profile of the one or more cardiac signal vectors, a trend of the angle of the one or more cardiac signal vectors, a trend of the magnitude of the one or more cardiac signal vectors, a trend of the variance of the one or more cardiac signal vectors, a trend of the velocity of the one or more cardiac signal vectors, a trend of the temporal profile of the one or more cardiac signal vectors, or means for using a window configured to enhance a morphological feature of the plurality of composite cardiac signals.

4.    The system of claim 1, wherein the detecting means comprises means for coordinating or indicating need for one or more of initiation, adjustment, optimization, and termination of treatment for one or more of a disease and a condition in response to detecting the change, means for coordinating or indicating need for one or more of bradycardia therapy, tachycardia therapy, cardiac resynchronization therapy, neural stimulation therapy, vascular intervention therapy, and pharmacologic therapy based on detecting the change.

5.    The system of claim 1, wherein the detecting means comprises means for triggering an alert or means for diagnosing a cardiac disease or condition.

6.    The system of claim 1, comprising means for effecting communication between the performing means and a patient-external device.

7. The system of claim 1, comprising means for measuring one or more of a patient's posture, activity, movement, heart-rate, heart rhythm, respiration, blood-pressure, blood gas concentration, blood chemistry, temperature, heart-sound, cardiac output, cardiac stroke volume, cardiac wall motion, peripheral or pulmonary fluid status, autonomic system status, thoracic impedance, and heart-rate variability.

8. The system of claim 1, comprising means for tracking and trending the one or more cardiac signal vectors, means for detecting ischemia, means for detecting or discriminating cardiac arrhythmias, means for characterizing a cardiac response to one or more pacing pulses, means for determining patient posture, or means for adjusting a cardiac resynchronization therapy.

9. The implantable cardiac system of claim 1, comprising:

   a plurality of implantable electrodes configured for sensing a composite signal, thereby providing a plurality of composite signals, wherein the means for sensing includes the plurality of implantable electrodes, the plurality of implantable electrodes including the plurality of cardiac electrodes;
   a housing configured for implantation in a patient;
   a controller provided in the housing;
   a signal processor, wherein the means for performing a source separation includes the signal processor; and
   a memory coupled to the signal processor;
   wherein the signal processor is configured to perform the source separation using the sensed plurality of composite signals, the source separation producing one or more cardiac signal vectors associated with all or a portion of one or more cardiac activation sequences, and to store vector information in the memory.

10. The system of claim 9, wherein the signal processor is provided in the housing.

11. The system of claim 9, wherein each of the plurality of electrodes is supported by the housing.

12. The system of claim 9, comprising one or more sensors configured to measure one or more of a patient's posture, activity, movement, heart-rate, heart rhythm, respiration, blood-pressure, blood gas concentration, blood chemistry, temperature, heart-sound, cardiac output, cardiac stroke volume, cardiac wall motion, peripheral or pulmonary fluid status, autonomic system status, thoracic impedance, and heart-rate variability, wherein information developed from the one or more sensors is used by the signal processor to interpret the one or more cardiac signal vectors.

13. The system of claim 9, wherein the one or more cardiac activation sequences are indicative of a QRS complex of a cardiac cycle, a P wave of a cardiac cycle, a T wave of a cardiac cycle, or an ST segment of a cardiac cycle.

14. The system of claim 9, wherein at least one of the controller and signal processor is configured to detect a cardiac condition using the vector information.

15. The system of claim 9, wherein at least one of the controller and signal processor is configured to detect a change in a characteristic of the one or more cardiac signal vectors relative to a baseline.

16. The system of claim 15, wherein the change detected by the at least one of the controller and signal processor comprises one or more of an angle change of the one or more cardiac signal vectors, a magnitude change of the one or more cardiac signal vectors, a variance change of the one or more cardiac signal vectors, a power spectral density change of the angle of the one or more cardiac signal vectors, a power spectral density change of the magnitude of the one or more cardiac signal vectors, a trajectory change of the one or more cardiac signal vectors, an activation velocity change, a temporal profile change of the one or more cardiac signal vectors, a rate of change of angle of the one or more cardiac signal vectors, a rate of change of magnitude of the one or more cardiac signal vectors, a rate of change of variance of the one or more cardiac signal vectors, a rate of change of velocity of the one or more cardiac signal vectors, a rate of change of temporal profile of the one or more cardiac signal vectors, a trend of the angle of the one or more cardiac signal vectors, a trend of the magnitude of the one or more cardiac signal vectors, a trend of the variance of the one or more cardiac signal vectors, a trend of the velocity of the one or more cardiac signal vectors, a trend of the temporal profile of the one or more cardiac signal vectors, and a change detected by using a window configured to enhance a morphological feature of the plurality of composite cardiac signals.

**17.** The system of claim 15, wherein the at least one of the controller and signal processor is configured to coordinate or indicate need for one or more of initiating, adjusting, optimizing and terminating treatment for one or more of a disease and a condition in response to detecting the change or to coordinate or indicate need for one or more of bradycardia therapy, tachycardia therapy, cardiac resynchronization therapy, neural stimulation therapy, vascular intervention therapy, and pharmacologic therapy based on detecting the change.

**18.** The system of claim 15, wherein the change detected by the at least one of the controller and signal processor is used to trigger an alert or diagnose a cardiac disease or condition.

**19.** The system of claim 15, wherein the change detected by the at least one of the controller and signal processor is detected beat-by-beat, within a cardiac cycle, within a predetermined time period, at a predetermined time, in response to reception of an external stimulus, or in response to reception of a patient-activated stimulus.

**20.** The system of claim 9, wherein the signal processor is configured to perform the source separation using the plurality of composite signals sensed over a plurality of cardiac cycles, during one cardiac cycle, during a time-period of less than about one cardiac cycle, during a time-period ranging between about one cardiac cycle and about ten cardiac cycles, or a time-period of more than about ten cardiac cycles.

**21.** The system of claim 9, wherein the signal processor is configured to perform a blind source separation.

**22.** The system of claim 9, wherein the at least one of the controller and signal processor is configured to select one or more of the cardiac signal vectors, store information associated with the selected one or more cardiac signal vectors, and track the selected one or more cardiac signal vectors using the vector information stored in the memory.

**23.** The system of claim 9, wherein the at least one of the controller and signal processor is configured to perform a subsequent source separation using a plurality of subsequently detected composite signals and detect a change in one or more cardiac signal vectors selected by the at least one of the controller and signal processor.

**24.** The system of claim 9, wherein the controller is configured to deliver one or more pacing pulses, and the at least one of the controller and signal processor is configured to classify a cardiac response to a pacing pulse using the vector information stored in the memory.

**25.** The system of claim 24, wherein the at least one of the controller and signal processor is configured to classify the cardiac response as capture or non-capture, as fusion or intrinsic cardiac activity, classify the cardiac response based on whether or not the vector falls within a predetermined range of angles, classify the cardiac response based on a morphology of one or more signals associated with the one or more cardiac signal vectors, classify the cardiac response based on a morphological change, relative to a baseline, of the one or more signals associated with the one or more cardiac signal vectors, discriminate between left ventricular non-capture and right ventricular non-capture in response to delivery of bi-ventricular pacing pulses, recommend an action based on the classified cardiac response to the one or more pacing pulses, trigger one or more threshold tests or increase one or more pacing amplitudes in response to classifying the cardiac response as non-capture, or discriminate between one or more of capture, non-capture, fusion, and pseudofusion.

**26.** The system of claim 9, wherein the at least one of the controller and signal processor is configured to discriminate between one or more of arrhythmia and non-arrhythmia, super-ventricular tachycardia and ventricular tachycardia, polymorphic ventricular tachycardia and monomorphic ventricular tachycardia, left-ventricular tachycardia and right-ventricular tachycardia, and premature atrial contraction and premature ventricular contraction.

**27.** The system of claim 9, wherein the at least one of the controller and signal processor is configured to discriminate the cardiac arrhythmias by determining a beat-to-beat variance of a characteristic of the one or more cardiac signal vectors, discriminate premature atrial contraction by detecting a positive phase of a dominant orientation of the one or more cardiac signal vectors, discriminate premature ventricular contraction by detecting a negative phase of a dominant orientation of one or more cardiac signal vectors, or discriminate between one or more of: supra-ventricular tachycardia and atrioventricular nodal supra-ventricular tachycardia; left atrial supra-ventricular tachycardia and right atrial supra-ventricular tachycardia; and ventricular tachycardia and ventricular fibrillation.

**28.** The system of claim 9, wherein the at least one of the controller and signal processor is configured to adjust a cardiac resynchronization therapy using one or both of the one or more cardiac signal vectors and the signals

36

associated with the one or more cardiac signal vectors.

29. The system of claim 28, wherein the at least one of the controller and signal processor is configured to adjust the cardiac resynchronization therapy by one or more of initiating the therapy, terminating the therapy, and altering one or more parameters of the therapy, to determine an angle of the one or more cardiac signal vectors, determine a dominant orientation of the one or more cardiac signal vectors, adjust the therapy such that an angle of a dominant QRS vector moves in a direction of an angle of a baseline QRS vector, adjust the therapy such that a predetermined angle moves toward a pre-determined vector angle, adjust the therapy such that a magnitude of the difference between a first angle and a second angle of the one or more cardiac signal vectors is smaller than a pre-determined threshold, and adjust the therapy to reduce the magnitude of cardiac asynchrony.

30. The system of claim 28, wherein the at least one of the controller and signal processor is configured to store information associated with the one or more cardiac signal vectors, perform a subsequent source separation providing updated vector information, and compare the updated vector information to the stored information to determine a level of cardiac asynchrony.

31. The system of claim 28, wherein the at least one of the controller and signal processor is configured to determine a dominant orientation of the one or more cardiac signal vectors and compare the dominant orientation to a baseline value to provide information related to the diagnosis of congestive heart failure in a patient or to provide information related to the progression of congestive heart failure in the patient.

32. The system of claim 28, wherein the at least one of the controller and signal processor is configured to determine patient response to the cardiac resynchronization therapy using the one or more cardiac signal vectors.

33. The system of claim 28, wherein the at least one of the controller and signal processor is configured to adjust the cardiac resynchronization therapy by recalculating one or more cardiac resynchronization therapy parameters and adjusting the cardiac resynchronization therapy using the recalculated one or more cardiac resynchronization therapy parameters, or to adjust at least one of an inter-ventricular delay, an inter-atrial delay, an intra-ventricular delay, an atrial-ventricular delay, a stimulation site, a pacing chamber, a rate limiter, and a pacing mode.

34. The system of claim 28, wherein the at least one of the controller and signal processor is configured to perform one or more of storing adjustments to the cardiac resynchronization therapy in the memory, communicating the adjustments to the cardiac resynchronization therapy to a patient-external device, and displaying the adjustments to the cardiac resynchronization therapy on a visual display.

35. The system of claim 9, wherein the at least one of the controller and signal processor is configured to detect a change of the selected one or more cardiac signal vectors using one or both of the selected one or more cardiac signal vectors and the signals associated with the selected one or more cardiac signal vectors, the change indicative of cardiac ischemia.

36. The system of claim 35, wherein the portion of one or more cardiac activation sequences comprises an ST segment, and the at least one of the controller and signal processor is configured to detect an elevation or depression of the ST segment.

37. The system of claim 35, wherein the at least one of the controller and the signal processor is configured to predict an arrhythmia or an increased risk of arrhythmia using the detected change, or predict a myocardial infarction using the detected change.

38. The system of claim 35, wherein the at least one of the controller and signal processor is configured to select one or more vectors preferentially useful in detecting ischemia.

39. The system of claim 9, wherein the at least one of the controller and signal processor is configured to detect a change in a patient's posture using the one or more cardiac signal vectors.

40. The system of claim 39, wherein the at least of the controller and signal processor is configured to detect a change in the one or more cardiac signal vectors, and discriminate between one of a postural related change and a cardiac rhythm related change using the detected change in the one or more cardiac signal vectors.

**41.** The system of claim 39, wherein the at least one of the controller and signal processor is configured to interpret the one or more cardiac signal vectors using the detected posture change, to interpret a sensor signal selected from the group consisting of heart-rate, heart rhythm, respiration, blood-pressure, temperature, heart sound, cardiac output, cardiac stroke volume, cardiac wall motion, peripheral fluid status, pulmonary fluid status, thoracic impedance, and heart-rate variability using the detected posture change, to interpret one or both of the patient's physiologic and pathologic status using both the selected sensor signal and the patient's detected posture change, or to interpret one or both of the patient's physiologic and pathologic trend using both the selected sensor signal and the patient's detected posture change.

**42.** The system of claim 39, wherein the at least one of the controller and signal processor is configured to store templates of one or more characteristics of the one or more cardiac signal vectors associated with sitting, standing, and lying postures of the patient in the memory, to determine attributes of the one or more cardiac signal vectors, and associate the determine attributes with the patient's posture using a look-up table of cardiac signal vector attributes based on postural changes, to categorize the patient's cardiac rhythm and match one or more characteristics of the one or more cardiac signal vectors to preestablished values associated with the rhythm category, or to coordinate one or more of storing the patient's posture information in the memory, communicating the patient's posture information to a patient-external device, and displaying the patient's posture information on a visual display.

**Patentansprüche**

**1.** Implantierbares Herzsystem, welches aufweist:

Mittel zum Erfassen, zumindest teilweise innerhalb eines Patienten, mehrerer zusammengesetzter Signale unter Verwendung mehrerer Herzelektroden; und
Mittel zum Durchführen einer Quellentrennung unter Verwendung der erfassten mehreren zusammengesetzten Signale, wobei die Quellentrennung einen oder mehrere Herzsignalvektoren erzeugt, die mit sämtlichen oder einem Teil von einer oder mehreren Herzaktivierungssequenzen assoziiert sind; wobei die Mittel zum Durchführen der Quellentrennung Mittel zum Durchführen einer anfänglichen Quellentrennung und einer nachfolgenden Quellentrennung enthalten; und
Mittel zum Erfassen einer oder mehrerer von einer Winkeländerung und einer Trajektorieänderung in einer Charakteristik des einen oder der mehreren Herzsignalvektoren relativ zu einer Basislinie.

**2.** System nach Anspruch 1, aufweisend zumindest ein Mittel zum Erfassen einer Herzkrankheit oder eines Herzzustands unter Verwendung der Änderung in der Charakteristik, oder Mittel zum Erfassen einer anomalen elektrischen Herzaktivität unter Verwendung der Änderung in der Charakteristik.

**3.** System nach Anspruch 1, bei dem die Erfassungsmittel weiterhin aufweisen: Mittel zum Erfassen von einer/einem oder mehreren von einer Größenänderung des einen oder der mehreren Herzsignalvektoren, einer Varianzänderung des einen oder der mehreren Herzsignalvektoren, einer spektralen Energiedichteänderung des Winkels des einen oder der mehreren Herzsignalvektoren, einer spektralen Energiedichteänderung der Größe des einen oder der mehreren Herzsignalvektoren, einer Aktivierungsgeschwindigkeitsänderung und einer zeitlichen Profiländerung des einen oder der mehreren Herzsignalvektoren, einer Rate der Winkeländerung des einen oder der mehreren Herzsignalvektoren, einer Rate der Größenänderung des einen oder der mehreren Herzsignalvektoren, einer Rate der Änderung der Varianz des einen oder mehreren Herzsignalvektoren, einer Rate der Geschwindigkeitsänderung des einen oder der mehreren Herzsignalvektoren, einer Rate der zeitlichen Profiländerung des einen oder der mehreren Herzsignalvektoren, eines Trends des Winkels des einen oder der mehreren Herzsignalvektoren, eines Trends der Größe des einen oder der mehreren Herzsignalvektoren, eines Trends der Varianz des einen oder der mehreren Herzsignalvektoren, eines Trends der Geschwindigkeit des einen oder der mehreren Herzsignalvektoren, eines Trends des zeitlichen Profils des einen oder der mehreren Herzsignalvektoren, oder Mittel zur Verwendung eines Fensters, das konfiguriert ist zum Vergrößern eines morphologischen Merkmals der mehreren zusammengesetzten Herzsignale.

**4.** System nach Anspruch 1, bei dem die Erfassungsmittel aufweisen:

Mittel zum Koordinieren oder Anzeigen der Notwendigkeit für eine oder mehrere von einer Initiierung, einer Einstellung einer Optimierung und einer Beendigung einer Behandlung für eine/einen oder mehrere von einer Krankheit und einem Zustand als Antwort auf die Erfassung der Änderung, Mittel zum Koordinieren oder Anzeigen der Notwendigkeit für eine oder mehrere von einer Bradykardietherapie, Tachykardietherapie, Herzresynchronisationstherapie, Nervenstimulationstherapie, vaskulären Interventionstherapie und pharmakologischen Therapie auf der Grundlage der Erfassung der Änderung.

**5.** System nach Anspruch 1, bei dem die Erfassungsmittel aufweisen: Mittel zum Auslösen eines Alarms oder Mittel zum Diagnostizieren einer Herzkrankheit oder eines Herzzustands.

**6.** System nach Anspruch 1, aufweisend Mittel zum Bewirken einer Kommunikation zwischen den Durchführungsmitteln und einer patientenexternen Vorrichtung.

**7.** System nach Anspruch 1, aufweisend Mittel zum Messen von einer/einem oder mehreren von einer Patientenkörperhaltung, Aktivität, Bewegung, Herzfrequenz, Herzrhythmus, Atmung, Blutdruck, Blutgaskonzentration, chemische Blutzusammensetzung, Temperatur, Herzton, Herzausgangsleistung, Herzschlagvolumen, Herzwandbewegung, peripherem oder Lungenfluidzustand, autonomem Systemzustand, thorakaler Impedanz und Herzfrequenzvariabilität.

**8.** System nach Anspruch 1, aufweisend Mittel zum Nachführen und Trenderfassen des einen oder der mehreren Herzsignalvektoren, Mittel zum Erfassen von Ischämie, Mittel zum Erfassen oder Unterscheiden von Herzarrhythmien, Mittel zum Charakterisieren einer Herzantwort auf einen oder mehrere Schrittgabeimpulse, Mittel zum Bestimmen der Patientenkörperhaltung oder Mittel zum Einstellen einer Herzresynchronisationstherapie.

**9.** Implantierbares Herzsystem nach Anspruch 1, welches aufweist:

mehrere implantierbare Elektroden, die zum Erfassen eines zusammengesetzten Signals konfiguriert sind, wodurch sie mehrere zusammengesetzte Signale bereitstellen, wobei die Mittel zum Erfassen die mehreren implantierbaren Elektroden enthalten und die mehreren implantierbaren Elektroden die mehreren Herzelektroden enthalten;
ein Gehäuse, das für die Implantierung in einem Patienten konfiguriert ist;
eine in dem Gehäuse angeordnete Steuervorrichtung;
einen Signalprozessor, wobei die Mittel zum Durchführen einer Quellentrennung den Signalprozessor enthalten; und
einen mit dem Signalprozessor gekoppelten Speicher;
wobei der Signalprozessor konfiguriert ist zum Durchführen der Quellentrennung unter Verwendung der erfassten mehreren zusammengesetzten Signale, welche Quellentrennung einen oder mehrere Herzsignalvektoren erzeugt, die mit allen oder einem Teil von einer oder mehreren Herzaktivierungssequenzen assoziiert sind, und zum Speichern von Vektorinformationen in dem Speicher.

**10.** System nach Anspruch 9, bei dem der Signalprozessor in dem Gehäuse angeordnet ist.

**11.** System nach Anspruch 9, bei dem jede der mehreren Elektroden durch das Gehäuse gestützt ist.

**12.** System nach Anspruch 9, aufweisend einen oder mehrere Sensoren, die konfiguriert sind zum Messen von einer/einem oder mehreren von einer Patientenkörperhaltung, Aktivität, Bewegung, Herzfrequenz, Herzrhythmus, Atmung, Blutdruck, Blutgaskonzentration, chemischer Blutzusammensetzung, Temperatur, Herzton, Herzausgangsleistung, Herzschlagvolumen, Herzwandbewegung, peripherem oder Lungenfluidzustand, autonomem Systemzustand, thorakaler Impedanz und Herzfrequenzvariabilität, wobei von dem einen oder den mehreren Sensoren entwickelte Informationen von dem Signalprozessor verwendet werden, um den einen oder die mehreren Herzsignalvektoren zu interpretieren.

**13.** System nach Anspruch 9, bei dem die eine oder die mehreren Herzaktivierungssequenzen hinweisend sind auf einen QRS-Komplex eines Herzzyklus, eine P-Welle eines Herzzyklus, eine T-Welle eines Herzzyklus oder ein ST-Segment eines Herzzyklus.

**14.** System nach Anspruch 9, bei dem zumindest eine/einer von der Steuervorrichtung und dem Signalprozessor konfiguriert ist, einen Herzzustand unter Verwendung der Vektorinformationen zu erfassen.

**15.** System nach Anspruch 9, bei dem zumindest eine/einer von der Steuervorrichtung und dem Signalprozessor konfiguriert ist, eine Änderung in einer Charakteristik des einen oder der mehreren Herzsignalvektoren relativ zu einer Basislinie zu erfassen.

16. System nach Anspruch 15, bei dem die durch die/den zumindest eine/einen von der Steuervorrichtung und dem Signalprozessor erfasste Änderung aufweist: eine/einen oder mehrere von einer Winkeländerung des einen oder der mehreren Herzsignalvektoren, eine Größenänderung des einen oder der mehreren Herzsignalvektoren, eine Varianzänderung des einen oder der mehreren Herzsignalvektoren, eine spektralen Energiedichteänderung des Winkels des einen oder der mehreren Herzsignalvektoren, eine spektralen Energiedichteänderung der Größe des einen oder der mehreren Herzsignalvektoren, eine Trajektorienänderung des einen oder der mehreren Herzsignalvektoren, eine Aktivierungsgeschwindigkeitsänderung, eine zeitliche Profiländerung des einen oder der mehreren Herzsignalvektoren, einer Rate der Winkeländerung des einen oder der mehreren Herzsignalvektoren, eine Rate der Größenänderung des einen oder der mehreren Herzsignalvektoren, einer Rate der Varianzänderung des einen oder der mehreren Herzsignalvektoren, einer Rate des Geschwindigkeitsänderungen des einen oder der mehreren Herzsignalvektoren, eine Rate der zeitlichen Profiländerung des einen oder der mehreren Herzsignalvektoren, einen Trend des Winkels des einen oder der mehreren Herzsignalvektoren, einen Trend der Größe des einen oder der mehreren Herzsignalvektoren, einen Trend der Varianz des einen oder der mehreren Herzsignalvektoren, einen Trend der Geschwindigkeit des einen oder der mehreren Herzsignalvektoren, einen Trend des zeitlichen Profils des einen oder der mehreren Herzsignalvektoren, und einer durch Verwendung eines Fensters, das konfiguriert ist, ein morphologisches Merkmal der mehreren zusammengesetzten Herzsignale zu vergrößern, erfasste Änderung.

17. System nach Anspruch 15, bei dem die/der zumindest eine von der Steuervorrichtung und dem Signalprozessor konfiguriert ist zum Koordinieren oder Anzeigen der Notwendigkeit für eine oder mehrere von einer Initiierung, Einstellung, Optimierung und Beendigung der Behandlung für eine/einen oder mehrere von einer Krankheit und einem Zustand als Antwort auf die Erfassung der Änderung, oder zum Koordinieren oder Anzeigen der Notwendigkeit für eine oder mehrere von einer Bradykardietherapie, Tachykardietherapie, Herzresynchronisationstherapie, Nervenstimulationstherapie, vaskulärer Interventionstherapie und pharmakologischer Therapie auf der Grundlage der Erfassung der Änderung.

18. System nach Anspruch 15, bei dem die durch die/den von zumindest einer/einem von der Steuervorrichtung und dem Signalprozessor erfasste Änderung verwendet wird, um einen Alarm auszulösen oder eine Herzkrankheit oder einen Herzzustand zu diagnostizieren.

19. System nach Anspruch 15, bei dem die durch die/den zumindest eine/einen von der Steuervorrichtung und dem Signalprozessor erfasste Änderung Schlag für Schlag, innerhalb eines Herzzyklus, innerhalb einer vorbestimmten Zeitperiode, zu einer vorbestimmten Zeit, als Antwort auf den Empfang eines externen Reizes oder als Antwort auf den Empfang eines patientenaktivierten Reizes erfasst wird.

20. System nach Anspruch 9, bei dem der Signalprozessor konfiguriert ist zum Durchführen der Quellentrennung unter Verwendung der mehreren zusammengesetzten Signale, die über mehrere Herzzyklen, während eines Herzzyklus, während einer Zeitperiode, die kürzer als etwa ein Herzzyklus ist, während einer Zeitperiode im Bereich zwischen etwa einem Herzzyklus und etwa zehn Herzzyklen oder einer Zeitperiode von mehr als etwa zehn Herzzyklen erfasst wurden.

21. System nach Anspruch 9, bei dem der Signalprozessor konfiguriert ist zum Durchführen einer Blindquellentrennung.

22. System nach Anspruch 9, bei dem die/der zumindest eine von der Steuervorrichtung und dem Signalprozessor konfiguriert ist, einen oder mehrere der Herzsignalvektoren auszuwählen, Informationen zu speichern, die mit dem/den einen oder mehreren ausgewählten Herzsignalvektoren assoziiert sind, und den/die ausgewählten einen oder mehreren Herzsignalvektoren unter Verwendung der in dem Speicher gespeicherten Vektorinformationen nachzuführen.

23. System nach Anspruch 9, bei dem die/der zumindest eine von der Steuervorrichtung und dem Signalprozessor konfiguriert ist zum Durchführen einer nachfolgenden Quellentrennung unter Verwendung mehrerer nachfolgend erfasster zusammengesetzter Signale und zum Erfassen einer Änderung in einem oder mehreren der ausgewählten Herzsignalvektoren durch die/den zumindest eine/einen von der Steuervorrichtung und dem Signalprozessor.

24. System nach Anspruch 9, bei dem die Steuervorrichtung konfiguriert ist zum Liefern von einem oder mehreren Schrittgabeimpulsen, und die/der zumindest eine von der Steuervorrichtung und dem Signalprozessor konfiguriert ist zum Klassifizieren einer Herzantwort auf einen Schrittgabeimpuls unter Verwendung der in dem Speicher gespeicherten Vektorinformationen.

25. System nach Anspruch 24, bei dem die/der zumindest eine von der Steuervorrichtung und dem Signalprozessor konfiguriert ist zum Klassifizieren der Herzantwort als Erfassung oder Nichterfassung, als Fusion oder intrinsische Herzaktivität, Klassifizieren der Herzantwort auf der Grundlage dessen, ob der Vektor in einen vorbestimmten Bereich von Winkeln fällt oder nicht, Klassifizieren der Herzantwort auf der Grundlage einer Morphologie von einem oder mehreren Signalen, die mit dem einen oder den mehreren Herzsignalvektoren assoziiert sind, Klassifizieren der Herzantwort auf der Grundlage einer morphologischen Änderung relativ zu einer Basislinie des einen oder der mehreren Signale, die mit dem einen oder den mehreren Herzsignalvektoren assoziiert sind, Unterscheiden zwischen linksventrikulärer Nichterfassung und rechtsventrikulärer Nichterfassung als Antwort auf die Zuführung von biventrikulären Schrittgabeimpulsen, Empfehlen einer Aktion auf der Grundlage der klassifizierten Herzantwort auf den einen oder die mehreren Schrittgabeimpulse, Auslösen eines oder mehrerer Schwellenwerttests oder Erhöhen einer oder mehrerer Schrittgabeamplituden als Antwort auf die Klassifizierung der Herzantwort als Nichterfassung, oder Unterscheiden zwischen einem oder mehreren von Erfassung, Nichterfassung, Fusion und Pseudofusion.

26. System nach Anspruch 9, bei dem die/der zumindest eine von der Steuervorrichtung und dem Signalprozessor konfiguriert ist zum Unterscheiden zwischen einer oder mehreren von Arhythmie und Nichtarhythmie, superventrikulärer Tachykardie und ventrikulärer Tachykardie, polymorpher ventrikulärer Tachykardie und monomorpher ventrikulärer Tachykardie, linksventrikulärer Tachykardie und rechtsventrikulärer Tachykardie, und vorzeitiger atrialer Kontraktion und vorzeitiger ventrikulärer Kontraktion.

27. System nach Anspruch 9, bei dem die/der zumindest eine von der Steuervorrichtung und dem Signalprozessor konfiguriert ist zum Unterscheiden der Herzarrhythmien durch Bestimmen einer Schlag-für-Schlag-Varianz einer Charakteristik des einen oder der mehreren Herzsignalvektoren, Unterscheiden einer vorzeitigen atrialen Kontraktion durch Erfassen einer positiven Phase einer dominanten Orientierung des einen oder der mehreren Herzsignalvektoren, Unterscheiden einer vorzeitigen ventrikulären Kontraktion durch Erfassen einer negativen Phase einer dominanten Orientierung des einen oder der mehreren Herzsignalvektoren, oder Unterscheiden zwischen einer/einem oder mehreren von: supraventrikulärer Tachykardie und atrioventrikulärer nodaler supraventrikulärer Tachykardie; linksatrialer supraventrikulärer Tachykardie und rechtsatrialer supraventrikulärer Tachykardie; und ventrikulärer Tachykardie und ventrikulärem Flimmern.

28. System nach Anspruch 9, bei dem die/der zumindest eine von der Steuervorrichtung und dem Signalprozessor konfiguriert ist zum Einstellen einer Herzresynchronisationstherapie unter Verwendung von einem oder beiden von dem einen oder den mehreren Herzsignalvektoren und den mit dem einen oder den mehreren Herzsignalvektoren assoziierten Signalen.

29. System nach Anspruch 28, bei dem die/der zumindest eine von der Steuervorrichtung und dem Signalprozessor konfiguriert ist zum Einstellen der Herzsynchronisationstherapie durch eines oder mehrere des Initiierens der Therapie, des Beendens der Therapie und der Änderung eines oder mehrerer Parameter der Therapie, zum Bestimmen eines Winkels des einen oder der mehreren Herzsignalvektoren, Bestimmen einer dominanten Orientierung des einen oder der mehreren Herzsignalvektoren, Einstellen der Therapie derart, dass ein Winkel eines dominanten QRS-Vektors sich in einer Richtung eines Winkels eines QRS-Basislinienvektors bewegt, Einstellen der Therapie derart, dass ein vorbestimmter Winkel sich zu einem vorbestimmten Vektorwinkel hin bewegt, Einstellen der Therapie derart, dass eine Größe der Differenz zwischen einem ersten Winkel und einem zweiten Winkel des einen oder der mehreren Herzsignalvektoren kleiner als ein vorbestimmter Schwellenwert ist, und Einstellen der Therapie zum Verringern der Größe von Herzasynchronismus.

30. System nach Anspruch 28, bei dem die/der zumindest eine von der Steuervorrichtung und dem Signalprozessor konfiguriert ist zum Speichern von Informationen, die mit dem einen oder den mehreren Herzsignalvektoren assoziiert sind, Durchführen einer nachfolgenden Quellentrennung zum Bereitstellen aktualisierter Vektorinformationen, und Vergleichen der aktualisierten Vektorinformationen mit den gespeicherten Informationen, um einen Pegel des Herzasynchronismus zu bestimmen.

31. System nach Anspruch 28, bei dem die/der zumindest eine von der Steuervorrichtung und dem Signalprozessor konfiguriert ist zum Bestimmen einer dominanten Orientierung des einen oder der mehreren Herzsignalvektoren und zum Vergleichen der dominanten Orientierung mit einem Basislinienwert, um auf die Diagnose von dekompensierter Herzinsuffizienz in einem Patienten bezogene Informationen bereitzustellen oder auf den Fortschritt von dekompensierter Herzinsuffizienz in den Patienten bezogene Informationen bereitzustellen.

**32.** System nach Anspruch 28, bei dem die/der zumindest eine von der Steuervorrichtung und dem Signalprozessor konfiguriert ist zum Bestimmen einer Patientenantwort auf die Herzresynchronisationstherapie unter Verwendung des einen oder der mehreren Herzsignalvektoren.

**33.** System nach Anspruch 28, bei dem die/der zumindest eine von der Steuervorrichtung und dem Signalprozessor konfiguriert ist zum Einstellen der Herzresynchronisationstherapie durch Neuberechnen eines oder mehrerer Herzresynchronisationstherapieparameter und Einstellen der Herzresynchronisationstherapie unter Verwendung des/der neu berechneten einen oder mehreren Herzresynchronisationstherapieparmeter, oder zum Einstellen von zumindest einer/einem von einer interventrikulären Verzögerung, einer interatrialen Verzögerung, einer intraventrikulären Verzögerung, einer atrioventrikulären Verzögerung, einer Stimulationsstelle, einer Schrittgabekammer, einem Ratenbegrenzer und einem Schrittgabemodus.

**34.** System nach Anspruch 28, bei dem die/der zumindest eine von der Steuervorrichtung und dem Signalprozessor konfiguriert ist zum Durchführen von einer/einem oder mehreren von Speichereinstellungen zu der Herzsynchronisationstherapie in dem Speicher, Kommunizieren der Einstellungen zu der Herzresynchronisationstherapie zu einer patientenexternen Vorrichtung und Anzeigen der Einstellungen zu der Herzresynchronisationstherapie auf einer visuellen Anzeigevorrichtung.

**35.** System nach Anspruch 9, bei dem die/der zumindest eine von der Steuervorrichtung und dem Signalprozessor konfiguriert ist zum Erfassen einer Änderung des/der ausgewählten einen oder mehreren Herzsignalvektoren unter Verwendung von einem oder beiden von dem einen oder den mehreren ausgewählten Herzsignalvektoren und den mit dem einen oder den mehreren ausgewählten Herzsignalvektoren assoziierten Signalen, wobei die Änderung eine Herzischämie anzeigt.

**36.** System nach Anspruch 35, bei dem der Teil von einer oder mehreren Herzaktivierungssequenzen ein ST-Segment aufweist und die/der zumindest eine von der Steuervorrichtung und dem Signalprozessor konfiguriert ist zum Erfassen einer Erhöhung oder Senkung des ST-Segments.

**37.** System nach Anspruch 35, bei dem die/der zumindest eine von der Steuervorrichtung und dem Signalprozessor konfiguriert ist zum Vorhersagen einer Arrhythmie oder einer erhöhten Gefahr von Arrhythmie unter Verwendung der erfassten Änderung, oder zum Vorhersagen eines Myokardinfarkts unter Verwendung der erfassten Änderung.

**38.** System nach Anspruch 35, bei dem die/der zumindest eine von der Steuervorrichtung und dem Signalprozessor konfiguriert ist zum Auswählen eines oder mehrerer Vektoren, die besonders nützlich bei der Erfassung von Ischämie sind.

**39.** System nach Anspruch 9, bei dem die/der zumindest eine von der Steuervorrichtung und dem Signalprozessor konfiguriert ist zum Erfassen einer Änderung in der Körperhaltung eines Patienten unter Verwendung des einen oder der mehreren Herzsignalvektoren.

**40.** System nach Anspruch 39, bei dem die/der zumindest eine von der Steuervorrichtung und dem Signalprozessor konfiguriert ist zum Erfassen einer Änderung des einen oder der mehreren Herzsignalvektoren, und zum Unterscheiden zwischen einer von einer körperhaltungsbezogenen Änderung und einer auf den Herzrhythmus bezogenen Änderung unter Verwendung der erfassten Änderung des einen oder der mehreren Herzsignalvektoren.

**41.** System nach Anspruch 39, bei dem die/der zumindest eine von der Steuervorrichtung und dem Signalprozessor konfiguriert ist zum Interpretieren des einen oder der mehreren Herzsignalvektoren unter Verwendung der erfassten Haltungsänderung, zum Interpretieren eines Sensorsignals, das ausgewählt ist aus der Gruppe bestehend aus Herzfrequenz, Herzrhythmus, Atmung, Blutdruck, Temperatur, Herzton, Herzausgangsleistung, Herzschlagvolumen, Herzwandbewegung, peripherem Fluidzustand, Lungenfluidzustand, thorakaler Impedanz und Herzfrequenzvariabilität unter Verwendung der erfassten Haltungsänderung, zum Interpretieren von einem oder beiden von dem physiologischen und dem pathologischen Zustand des Patienten unter Verwendung sowohl des ausgewählten Sensorsignals als auch der erfassten Körperhaltungsänderung des Patienten, oder zum Interpretieren von einem oder beiden von dem physiologischen und dem pathologischen Trend des Patienten unter Verwendung sowohl des ausgewählten Sensorsignals als auch der erfassten Körperhaltungsänderung des Patienten.

**42.** System nach Anspruch 39, bei dem die/der zumindest eine von der Steuervorrichtung und dem Signalprozessor konfiguriert ist zum Speichern von Schablonen von einer oder mehreren Charakteristiken des einen oder der mehreren Herzsignalvektoren, die mit sitzender, stehender und liegender Körperhaltung des Patienten assoziiert sind, in dem Speicher, zum Bestimmen von Attributen des einen oder der mehreren Herzsignalvektoren, und Assoziieren der bestimmten Attribute mit der Körperhaltung des Patienten unter Verwendung einer Nachschlagtabelle von Herzsignalvektorattributen auf der Grundlage von Körperhaltungsänderungen, um den Herzrhythmus des Patienten zu kategorisieren und eine oder mehrere Charakteristiken des einen oder der mehreren Herzsignalvektoren mit vorher aufgestellten Werten, die mit der Rhythmuskategorie assoziiert sind, zu vergleichen, oder zum Koordinieren einer oder mehrerer Speicherungen der Körperhaltungsinformationen des Patienten in dem Speicher, Kommunizieren der Haltungsinformationen des Patienten zu einer patientenexternen Vorrichtung und Anzeigen der Körperhaltungsinformationen des Patienten auf einer visuellen Anzeigevorrichtung.

**Revendications**

1. Système cardiaque implantable, comprenant :

   un moyen pour détecter, au moins en partie à l'intérieur d'un patient, une pluralité de signaux composites en utilisant une pluralité d'électrodes cardiaques ; et
   un moyen pour réaliser une séparation de sources en utilisant la pluralité détectée de signaux composites, la séparation de sources produisant un ou plusieurs vecteur(s) de signal cardiaque qui est/sont associé(s) à la totalité ou à une partie d'une ou de plusieurs séquence(s) d'activation cardiaque ; dans lequel le moyen pour réaliser la séparation de sources inclut un moyen pour réaliser une séparation de sources initiale et une séparation de sources ultérieure ; et
   un moyen pour détecter une ou plusieurs variation(s) prise(s) parmi une variation d'angle et une variation de trajectoire d'une caractéristique des un ou plusieurs vecteurs de signal cardiaque par rapport à une ligne de base.

2. Système selon la revendication 1, comprenant au moins un moyen pour détecter une maladie ou condition cardiaque en utilisant la variation de la caractéristique, ou un moyen pour détecter une activité électrique cardiaque anormale en utilisant la variation de la caractéristique.

3. Système selon la revendication 1, dans lequel le moyen de détection comprend en outre un moyen pour détecter un ou plusieurs paramètre(s) pris parmi une variation d'amplitude des un ou plusieurs vecteurs de signal cardiaque, une variation de variance des un ou plusieurs vecteurs de signal cardiaque, une variation de densité spectrale de puissance de l'angle des un ou plusieurs vecteurs de signal cardiaque, une variation de densité spectrale de puissance de l'amplitude des un ou plusieurs vecteurs de signal cardiaque, une variation de vitesse d'activation et une variation de profil temporel des un ou plusieurs vecteurs de signal cardiaque, un taux de variation de l'angle des un ou plusieurs vecteurs de signal cardiaque, un taux de variation de l'amplitude des un ou plusieurs vecteurs de signal cardiaque, un taux de variation de la variance des un ou plusieurs vecteurs de signal cardiaque, un taux de variation de la vitesse des un ou plusieurs vecteurs de signal cardiaque, un taux de variation du profil temporel des un ou plusieurs vecteurs de signal cardiaque, une tendance de l'angle des un ou plusieurs vecteurs de signal cardiaque, une tendance de l'amplitude des un ou plusieurs vecteurs de signal cardiaque, une tendance de la variance des un ou plusieurs vecteurs de signal cardiaque, une tendance de la vitesse des un ou plusieurs vecteurs de signal cardiaque, une tendance du profil temporel des un ou plusieurs vecteurs de signal cardiaque, ou un moyen pour utiliser une fenêtre qui est configurée de manière à améliorer une caractéristique morphologique de la pluralité de signaux cardiaques composites.

4. Système selon la revendication 1, dans lequel le moyen de détection comprend un moyen pour coordonner ou pour indiquer le besoin d'une ou de plusieurs action(s) prise(s) parmi une initiation, un réglage, une optimisation et un arrêt d'un traitement pour un ou plusieurs événement(s) pris parmi une maladie et une condition en réponse à la détection de la variation, un moyen pour coordonner ou pour indiquer le besoin d'une ou de plusieurs thérapie(s) prise(s) parmi une thérapie de bradycardie, une thérapie de tachycardie, une thérapie de resynchronisation cardiaque, une thérapie de stimulation neurale, une thérapie d'intervention vasculaire et une thérapie pharmacologique sur la base de la détection de la variation.

5. Système selon la revendication 1, dans lequel le moyen de détection comprend un moyen pour déclencher une alerte ou un moyen pour diagnostiquer une maladie ou condition cardiaque.

6. Système selon la revendication 1, comprenant un moyen pour effectuer une communication entre le moyen pour réaliser et un dispositif externe au patient.

**7.** Système selon la revendication 1, comprenant un moyen pour mesurer un ou plusieurs paramètre(s), chez un patient, pris parmi la posture, l'activité, le mouvement/déplacement, la fréquence cardiaque, le rythme cardiaque, la respiration, la pression sanguine, la concentration des gaz dans le sang, la composition chimique du sang, la température, le bruit cardiaque, la sortie cardiaque, le volume d'éjection systolique cardiaque, le mouvement/ déplacement de la paroi cardiaque, l'état du fluide périphérique ou pulmonaire, l'état du système autonome, l'impédance thoracique et la variabilité de la fréquence cardiaque.

**8.** Système selon la revendication 1, comprenant un moyen pour suivre les un ou plusieurs vecteurs de signal cardiaque et pour déterminer la tendance de ces mêmes un ou plusieurs vecteurs de signal cardiaque, un moyen pour détecter l'ischémie, un moyen pour détecter ou discriminer les arythmies cardiaques, un moyen pour caractériser une réponse cardiaque à une ou plusieurs impulsion(s) de stimulation cardiaque, un moyen pour déterminer la posture du patient, ou un moyen pour régler une thérapie de resynchronisation cardiaque.

**9.** Système cardiaque implantable selon la revendication 1, comprenant :

une pluralité d'électrodes implantables qui sont configurées de manière à détecter un signal composite, d'où ainsi la production d'une pluralité de signaux composites, dans lequel le moyen pour détecter inclut les électrodes de la pluralité d'électrodes implantables, les électrodes de la pluralité d'électrodes implantables incluant les électrodes de la pluralité d'électrodes cardiaques ;
un boîtier qui est configuré de manière à être implanté sur un patient ;
un contrôleur qui est prévu dans le boîtier ;
un processeur de signal, dans lequel le moyen pour réaliser une séparation de sources inclut le processeur de signal ; et
une mémoire qui est couplée au processeur de signal ; dans lequel :

le processeur de signal est configuré de manière à réaliser la séparation de sources en utilisant la pluralité détectée de signaux composites, la séparation de sources produisant un ou plusieurs vecteur(s) de signal cardiaque qui est/sont associé(s) à la totalité ou à une partie d'une ou de plusieurs séquence(s) d'activation cardiaque, et de manière à stocker une information vectorielle dans la mémoire.

**10.** Système selon la revendication 9, dans lequel le processeur de signal est prévu dans le boîtier.

**11.** Système selon la revendication 9, dans lequel chacune de la pluralité d'électrodes est supportée par le boîtier.

**12.** Système selon la revendication 9, comprenant un ou plusieurs capteur(s) qui est/sont configuré(s) de manière à mesurer un ou plusieurs paramètre(s), chez un patient, pris parmi la posture, l'activité, le mouvement/déplacement, la fréquence cardiaque, le rythme cardiaque, la respiration, la pression sanguine, la concentration des gaz dans le sang, la composition chimique du sang, la température, le bruit cardiaque, la sortie cardiaque, le volume d'éjection systolique cardiaque, le mouvement/ déplacement de la paroi cardiaque, l'état du fluide périphérique ou pulmonaire, l'état du système autonome, l'impédance thoracique et la variabilité de la fréquence cardiaque, dans lequel l'information qui est développée à partir des un ou plusieurs capteurs est utilisée par le processeur de signal pour interpréter les un ou plusieurs vecteurs de signal cardiaque.

**13.** Système selon la revendication 9, dans lequel les une ou plusieurs séquences d'activation cardiaque sont indicatives d'un complexe QRS d'un cycle cardiaque, d'une onde P d'un cycle cardiaque, d'une onde T d'un cycle cardiaque ou d'un segment ST d'un cycle cardiaque.

**14.** Système selon la revendication 9, dans lequel au moins un moyen pris parmi le contrôleur et le processeur de signal est configuré de manière à détecter une condition cardiaque en utilisant l'information vectorielle.

**15.** Système selon la revendication 9, dans lequel au moins un moyen pris parmi le contrôleur et le processeur de signal est configuré de manière à détecter une variation d'une caractéristique des un ou plusieurs vecteur(s) de signal cardiaque par rapport à une ligne de base.

**16.** Système selon la revendication 15, dans lequel la variation qui est détectée par l'au moins un moyen pris parmi le contrôleur et le processeur de signal comprend un ou plusieurs paramètre(s) pris parmi une variation d'angle des un ou plusieurs vecteurs de signal cardiaque, une variation d'amplitude des un ou plusieurs vecteurs de signal cardiaque, une variation de variance des un ou plusieurs vecteurs de signal cardiaque, une variation de densité spectrale de puissance de l'angle des un ou plusieurs vecteurs de signal cardiaque, une variation de densité spectrale de puissance de l'amplitude des un ou plusieurs vecteurs de signal cardiaque, une variation de trajectoire des un ou plusieurs vecteurs de signal cardiaque, une variation de vitesse d'activation, une variation de profil temporel des un ou plusieurs vecteurs de signal cardiaque, un taux de variation de l'angle des un ou plusieurs vecteurs de signal cardiaque, un taux de variation de l'amplitude des un ou plusieurs vecteurs de signal cardiaque, un taux de variation de la variance des un ou plusieurs vecteurs de signal cardiaque, un taux de variation de la vitesse des un ou plusieurs vecteurs de signal cardiaque, un taux de variation du profil temporel des un ou plusieurs vecteurs de signal cardiaque, une tendance de l'angle des un ou plusieurs vecteurs de signal cardiaque, une tendance de l'amplitude des un ou plusieurs vecteurs de signal cardiaque, une tendance de la variance des un ou plusieurs vecteurs de signal cardiaque, une tendance de la vitesse des un ou plusieurs vecteurs de signal cardiaque, une tendance du profil temporel des un ou plusieurs vecteurs de signal cardiaque, et une variation détectée en utilisant une fenêtre qui est configurée de manière à améliorer une caractéristique morphologique de la pluralité de signaux cardiaques composites.

17. Système selon la revendication 15, dans lequel l'au moins un moyen pris parmi le contrôleur et le processeur de signal est configuré de manière à coordonner ou à indiquer le besoin d'une ou de plusieurs action(s) prise(s) parmi une initiation, un réglage, une optimisation et un arrêt d'un traitement pour un ou plusieurs événement(s) pris parmi une maladie et une condition en réponse à la détection de la variation, ou de manière à coordonner ou à indiquer le besoin d'une ou de plusieurs thérapie(s) prise(s) parmi une thérapie de bradycardie, une thérapie de tachycardie, une thérapie de resynchronisation cardiaque, une thérapie de stimulation neurale, une thérapie d'intervention vasculaire et une thérapie pharmacologique sur la base de la détection de la variation.

18. Système selon la revendication 15, dans lequel la variation qui est détectée par l'au moins un moyen pris parmi le contrôleur et le processeur de signal est utilisée pour déclencher une alerte ou pour diagnostiquer une maladie ou une condition cardiaque.

19. Système selon la revendication 15, dans lequel la variation qui est détectée par l'au moins un moyen pris parmi le contrôleur et le processeur de signal est détectée battement par battement, à l'intérieur d'un cycle cardiaque, à l'intérieur d'une période temporelle prédéterminée, à un instant prédéterminé, en réponse à la réception d'un stimulus externe, ou en réponse à la réception d'un stimulus activé par le patient.

20. Système selon la revendication 9, dans lequel le processeur de signal est configuré de manière à réaliser la séparation de sources en utilisant les signaux de la pluralité de signaux composites qui sont détectés sur une pluralité de cycles cardiaques, pendant un seul cycle cardiaque, pendant une période temporelle inférieure à environ un seul cycle cardiaque, pendant une période temporelle qui s'inscrit dans la plage entre environ un seul cycle cardiaque et environ dix cycles cardiaques, ou pendant une période temporelle de plus d'environ dix cycles cardiaques.

21. Système selon la revendication 9, dans lequel le processeur de signal est configuré de manière à réaliser une séparation de sources en aveugle.

22. Système selon la revendication 9, dans lequel l'au moins un moyen pris parmi le contrôleur et le processeur de signal est configuré de manière à sélectionner un ou plusieurs des vecteurs de signal cardiaque, de manière à stocker une information qui est associée aux un ou plusieurs vecteurs de signal cardiaque sélectionnés et de manière à suivre les un ou plusieurs vecteurs de signal cardiaque sélectionnés en utilisant l'information vectorielle qui est stockée dans la mémoire.

23. Système selon la revendication 9, dans lequel l'au moins un moyen pris parmi le contrôleur et le processeur de signal est configuré de manière à réaliser une séparation de sources ultérieure en utilisant une pluralité de signaux composites détectés ultérieurement et de manière à détecter une variation d'un ou de plusieurs vecteur(s) de signal cardiaque qui est/sont sélectionné(s) par l'au moins un moyen pris parmi le contrôleur et le processeur de signal.

24. Système selon la revendication 9, dans lequel le contrôleur est configuré de manière à délivrer une ou plusieurs impulsion(s) de stimulation cardiaque, et l'au moins un moyen pris parmi le contrôleur et le processeur de signal est configuré de manière à classifier une réponse cardiaque à une impulsion de stimulation cardiaque en utilisant l'information vectorielle qui est stockée dans la mémoire.

25. Système selon la revendication 24, dans lequel l'au moins un moyen pris parmi le contrôleur et le processeur de signal est configuré de manière à classifier la réponse cardiaque en tant que capture ou que non capture, en tant qu'activité cardiaque de fusion ou qu'activité cardiaque intrinsèque, de manière à classifier la réponse cardiaque sur la base de si oui ou non le vecteur tombe à l'intérieur d'une plage d'angles prédéterminée, de manière à classifier la réponse cardiaque sur la base d'une morphologie d'un signal ou de plusieurs signaux qui est/sont associé(s) aux un ou plusieurs vecteurs de signal cardiaque, de manière à classifier la réponse cardiaque sur la base d'une variation morphologique, par rapport à une ligne de base, des un ou plusieurs signaux qui sont associés aux un ou plusieurs vecteurs de signal cardiaque, de manière à effectuer une discrimination entre une non capture de ventricule gauche et une non capture de ventricule droit en réponse à la délivrance d'impulsions de stimulation cardiaque biventriculaires, de manière à recommander une action sur la base de la réponse cardiaque classifiée aux une ou plusieurs impulsions de stimulation cardiaque, de manière à déclencher un ou plusieurs test(s) de seuil ou de manière à augmenter une ou plusieurs amplitude(s) de stimulation cardiaque en réponse à la classification de la réponse cardiaque en tant que non capture, ou de manière à effectuer une discrimination entre un ou plusieurs événement(s) pris parmi une capture, une non capture, une fusion et une pseudo-fusion.

26. Système selon la revendication 9, dans lequel l'au moins un moyen pris parmi le contrôleur et le processeur de signal est configuré de manière à effectuer une discrimination entre un ou plusieurs événement(s) pris parmi une arythmie et une non arythmie, une tachycardie supraventriculaire et une tachycardie ventriculaire, une tachycardie ventriculaire polymorphe et une tachycardie ventriculaire monomorphe, une tachycardie de ventricule gauche et une tachycardie de ventricule droit, et une contraction d'oreillette prématurée et une contraction de ventricule prématurée.

27. Système selon la revendication 9, dans lequel l'au moins un moyen pris parmi le contrôleur et le processeur de signal est configuré de manière à discriminer les arythmies cardiaques en déterminant une variance battement à battement d'une caractéristique des un ou plusieurs vecteurs de signal cardiaque, de manière à discriminer une contraction d'oreillette prématurée en détectant une phase positive d'une orientation dominante des un ou plusieurs vecteurs de signal cardiaque, de manière à discriminer une contraction de ventricule prématurée en détectant une phase négative d'une orientation dominante des un ou plusieurs vecteurs de signal cardiaque, ou de manière à effectuer une discrimination entre un ou plusieurs événement(s) pris parmi : une tachycardie supraventriculaire et une tachycardie supraventriculaire de noeud atrioventriculaire ; une tachycardie supra-ventriculaire d'oreillette gauche et une tachycardie supraventriculaire d'oreillette droite ; et une tachycardie ventriculaire et une fibrillation ventriculaire.

28. Système selon la revendication 9, dans lequel l'au moins un moyen pris parmi le contrôleur et le processeur de signal est configuré de manière à régler une thérapie de resynchronisation cardiaque en utilisant soit les un ou plusieurs vecteurs de signal cardiaque, soit les signaux qui sont associés aux un ou plusieurs vecteurs de signal cardiaque, soit les un ou plusieurs vecteurs de signal cardiaque et les signaux qui sont associés aux un ou plusieurs vecteurs de signal cardiaque.

29. Système selon la revendication 28, dans lequel l'au moins un moyen pris parmi le contrôleur et le processeur de signal est configuré de manière à régler la thérapie de resynchronisation cardiaque soit en initiant la thérapie, soit en arrêtant la thérapie, soit en altérant un ou plusieurs paramètre(s) de la thérapie, soit en effectuant plusieurs des actions précitées, de manière à déterminer un angle des un ou plusieurs vecteurs de signal cardiaque, de manière à déterminer une orientation dominante des un ou plusieurs vecteurs de signal cardiaque, de manière à régler la thérapie de telle sorte qu'un angle d'un vecteur QRS dominant se déplace dans une direction d'un angle d'un vecteur QRS de ligne de base, de manière à régler la thérapie de telle sorte qu'un angle prédéterminé se déplace en direction d'un angle de vecteur ou vectoriel prédéterminé, de manière à régler la thérapie de telle sorte qu'une amplitude de la différence entre un premier angle et un second angle des un ou plusieurs vecteurs de signal cardiaque soit inférieure à un seuil prédéterminé, et de manière à régler la thérapie afin de réduire l'amplitude de l'asynchronie cardiaque.

30. Système selon la revendication 28, dans lequel l'au moins un moyen pris parmi le contrôleur et le processeur de signal est configuré de manière à stocker une information qui est associée aux un ou plusieurs vecteurs de signal cardiaque, de manière à réaliser une séparation de sources ultérieure qui fournit une information vectorielle mise à jour, et de manière à comparer l'information vectorielle mise à jour avec l'information stockée de manière à déterminer un niveau de l'asynchronie cardiaque.

31. Système selon la revendication 28, dans lequel l'au moins un moyen pris parmi le contrôleur et le processeur de signal est configuré de manière à déterminer une orientation dominante des un ou plusieurs vecteurs de signal cardiaque et de manière à comparer l'orientation dominante à une valeur de ligne de base de manière à fournir une information qui concerne le diagnostic d'une insuffisance cardiaque congestive ou globale chez un patient ou de manière à fournir une information qui concerne la progression d'une insuffisance cardiaque congestive ou globale chez le patient.

**32.** Système selon la revendication 28, dans lequel l'au moins un moyen pris parmi le contrôleur et le processeur de signal est configuré de manière à déterminer une réponse de patient à la thérapie de resynchronisation cardiaque en utilisant les un ou plusieurs vecteurs de signal cardiaque.

**33.** Système selon la revendication 28, dans lequel l'au moins un moyen pris parmi le contrôleur et le processeur de signal est configuré de manière à régler la thérapie de resynchronisation cardiaque en recalculant un ou plusieurs paramètre(s) de thérapie de resynchronisation cardiaque et en réglant la thérapie de resynchronisation cardiaque en utilisant les un ou plusieurs paramètre(s) de thérapie de resynchronisation cardiaque recalculés, ou de manière à régler au moins un paramètre pris parmi un retard interventriculaire, un retard inter-oreillette, un retard intraventriculaire, un retard oreillette-ventricule, un site de stimulation, une chambre de stimulation cardiaque, un limiteur de fréquence et un mode de stimulation cardiaque.

**34.** Système selon la revendication 28, dans lequel l'au moins un moyen pris parmi le contrôleur et le processeur de signal est configuré de manière à effectuer une ou plusieurs action(s) prise(s) parmi le stockage de réglages apportés à la thérapie de resynchronisation cardiaque dans la mémoire, la communication des réglages apportés à la thérapie de resynchronisation cardiaque à un dispositif externe au patient, et l'affichage des réglages apportés à la thérapie de resynchronisation cardiaque sur un affichage visuel.

**35.** Système selon la revendication 9, dans lequel l'au moins un moyen pris parmi le contrôleur et le processeur de signal est configuré de manière à détecter une variation des un ou plusieurs vecteurs de signal cardiaque sélectionnés en utilisant soit les un ou plusieurs vecteurs de signal cardiaque sélectionnés, soit les signaux qui sont associés aux un ou plusieurs vecteurs de signal cardiaque sélectionnés, soit les un ou plusieurs vecteurs de signal cardiaque sélectionnés et les signaux qui sont associés aux un ou plusieurs vecteurs de signal cardiaque sélectionnés, la variation étant indicative d'une ischémie cardiaque.

**36.** Système selon la revendication 35, dans lequel la partie d'une ou de plusieurs séquence(s) d'activation cardiaque comprend un segment ST, et l'au moins un moyen pris parmi le contrôleur et le processeur de signal est configuré de manière à détecter une élévation ou une diminution du segment ST.

**37.** Système selon la revendication 35, dans lequel l'au moins un moyen pris parmi le contrôleur et le processeur de signal est configuré de manière à prédire une arythmie ou un risque accru d'arythmie en utilisant la variation détectée, ou de manière à prédire un infarctus du myocarde en utilisant la variation détectée.

**38.** Système selon la revendication 35, dans lequel l'au moins un moyen pris parmi le contrôleur et le processeur de signal est configuré de manière à sélectionner un ou plusieurs vecteur(s) utile(s) de façon préférentielle au niveau de la détection d'une ischémie.

**39.** Système selon la revendication 9, dans lequel l'au moins un moyen pris parmi le contrôleur et le processeur de signal est configuré de manière à détecter une variation au niveau de la posture du patient en utilisant les un ou plusieurs vecteurs de signal cardiaque.

**40.** Système selon la revendication 39, dans lequel l'au moins un moyen pris parmi le contrôleur et le processeur de signal est configuré de manière à détecter une variation au niveau des un ou plusieurs vecteurs de signal cardiaque, et de manière à effectuer une discrimination entre une variation prise parmi une variation concernant la posture et une variation concernant le rythme cardiaque en utilisant la variation détectée au niveau des un ou plusieurs vecteurs de signal cardiaque.

**41.** Système selon la revendication 39, dans lequel l'au moins un moyen pris parmi le contrôleur et le processeur de signal est configuré de manière à interpréter les un ou plusieurs vecteurs de signal cardiaque en utilisant la variation de posture détectée, de manière à interpréter un signal de capteur qui est sélectionné parmi le groupe qui est constitué par la fréquence cardiaque, le rythme cardiaque, la respiration, la pression sanguine, la température, le bruit cardiaque, la sortie cardiaque, le volume d'éjection systolique cardiaque, le mouvement/déplacement de la paroi cardiaque, l'état du fluide périphérique, l'état du fluide pulmonaire, l'impédance thoracique et la variabilité de la fréquence cardiaque en utilisant la variation de posture détectée, de manière à interpréter soit l'état physiologique du patient, soit l'état pathologique du patient, soit les deux en utilisant à la fois le signal de capteur sélectionné et la variation de posture détectée du patient, ou de manière à interpréter soit la tendance physiologique du patient, soit la tendance pathologique du patient, soit les deux en utilisant à la fois le signal de capteur sélectionné et la variation de posture détectée du patient.

42. Système selon la revendication 39, dans lequel l'au moins un moyen pris parmi le contrôleur et le processeur de signal est configuré de manière à stocker des gabarits d'une ou de plusieurs caractéristique(s) des un ou plusieurs vecteurs de signal cardiaque qui est/sont associé(e)(s) à des postures que sont la position assise, la position debout et la position allongée du patient dans la mémoire, de manière à déterminer des attributs des un ou plusieurs vecteurs de signal cardiaque, et de manière à associer les attributs déterminés avec la posture du patient en utilisant une table de consultation d'attributs de vecteurs de signal cardiaque qui sont basés sur des variations de posture, de manière à catégoriser le rythme cardiaque du patient et de manière à faire correspondre une ou plusieurs caractéristique(s) des un ou plusieurs vecteurs de signal cardiaque à des valeurs préétablies qui sont associées à la catégorie de rythme, ou de manière à coordonner une ou plusieurs action(s) prise(s) parmi le stockage de l'information de posture de patient dans la mémoire, la communication de l'information de posture de patient à un dispositif externe au patient et l'affichage de l'information de posture de patient sur un affichage visuel.

# Fig. 1A

EP 1 809 172 B1

Fig. 1B

Fig. 2

300

-90

+/- 180

0

P vector

QRS Vector

320

310

90

Fig. 3A

Fig. 3B

400

410

430

440

420

# Fig. 4

Fig. 5A

Fig. 5B

Fig. 5C

Fig. 5D

600

610
Establish Baseline Information

620
Establish Evaluation Criteria

630
Compare Current Information to Baseline

640
Criteria Met ?

y

n

650
Pattern A

660
Pattern A Complement

Fig. 6A

605

612

Establish Baseline Information

622

Establish Evaluation Criteria

632

Compare Current Information to Baseline

642

First Criteria Met ?

y        n

652

Pattern A

662

Pattern A Complement

Second Criteria Met ?

y        n

672

682

Pattern B

692

Pattern B Complement

Fig. 6B

Rhythm 1     Rhythm 1

Posture 1     Posture 2

607

617

Posture change

627

t

Orientation 1

Orientation 2

637

647

## Fig. 6C

604

644     654     664

| | Sitting | Standing | Lying |
|---|---|---|---|
| Intrinsic | Orientation 11 | Orientation 12 | Orientation 13 |
| RV pacing | Orientation 21 | Orientation 22 | Orientation 23 |
| LV pacing | Orientation 31 | Orientation 32 | Orientation 33 |

614

624

634

## Fig. 6D

Fig. 6E

609

Sense Plurality of Composite Signals

619

Perform Source Separation

629

Select Vector(s)

639

Store Vector Information

649

Determine Patient's Posture

659

Fig. 6F

Fig. 6G

Fig. 6H

Fig. 6I

Fig. 6J

412

414

Establish Baseline Information

416

Establish The Range Of Angular
Orientation Shift During Capture

Collect New Data During Pacing &
Calculate Orientation

418

424

Capture

422

y

Axis Shift In
Range?

n

426

Non-Capture

Fig. 6K

3432

Establish Baseline Information
3434

Establish The Range Of Angular
Orientation Shift During Capture
3436

Collect New Data During Pacing &
Calculate Orientation
3438

3444

Capture And
Lower
Pacing
Voltage

y

3442

Axis Shift In
Range?

n

3446

Non-Capture

Fig. 6L

3452

3454

Establish Baseline Information

3456

Establish The Range Of Angular
Orientation Shift During Capture

Collect New Data During Pacing &
Calculate Orientation

3458

3464

RV Capture

3462

y ← Axis Shift In RV
Range? → n

3466

RV Non-
Capture

3474

LV Capture

3472

y ← Axis Shift In LV
Range? → n

3476

LV Non-
Capture

3484

BiV Capture

3482

y ← Axis Shift In BiV
Range? → n

3486

BiV Non-
Capture

Fig. 6M

4402

4404 → | **Establish Baseline Information** |

4406 → | **Establish Range Of Orientation During SVT** |

4408 — **Tachycardia?** — n

y

4412 → | **Determine Vector Orientation And Axis Shift** |

4416 → | **SVT** |   y   4414 — **Shift In SVT Range?**   n   4418 → | **VT** |

## Fig. 6N

Fig. 6O

Fig. 6P

4442

Establish Pre-Determined Value For
Variation During MVT

4444

VT Episode? — n

4446

y

Calculate Orientation For Each Beat
Of VT And Its Variation

4448

4454

PVT

y ← Variation >
Value? → n

4452

4456

MVT

Fig. 6Q

4458

Establish Direction And Range Of
Shift For LVT and RVT

4462

4464    VT Episode?    n

y

Calculate Orientation For The New
Episode

4466

4472

LVT

4468

y    Shift in LVT
Direction?

n

4476

RVT

4474

y    Shift in RVT
Direction?

n

4478

Other Discrimination Algorithms

Fig. 6R

Baseline: Rectangle - starting from0.001s

Fig. 6S

Partial Occlusion: Rectangle - starting from0.001s

Fig. 6T

Complete Occlusion: Rectangle - starting from0.001s

-90

• – P-QRS—401
• – ST-T—402

±180 — 0 | Fig. 6U

+90

Baseline: Rectangle - starting from0.07s

-90

• – P-QRS—403
• – ST-T—404

±180 — 0 | Fig. 6V

405

+90

Partial Occlusion: Rectangle - starting from0.07s

Fig. 6W

Complete Occlusion: Rectangle - starting from0.07s

Fig. 6X

Baseline: Rectangle - starting from0.14s

Fig. 6Y

Partial Occlusion: Rectangle - starting from0.14s

Fig. 6Z

Complete Occlusion: Rectangle - starting from0.14s

-90

• - P-QRS——406
*- ST-T——407

±180

0

Fig. 6AA

+90

Baseline: Rectangle - starting from0.21s

-90

• - P-QRS——408
*- ST-T——409

±180

C

Fig. 6AB

411

+90

Partial Occlusion: Rectangle - starting from 0.21s

* – P-QRS —— 408
+ – ST-T —— 409

Fig. 6AC

Complete Occlusion: Rectangle - starting from 0.21s

* – P-QRS —— 408
+ – ST-T —— 409

Fig. 6AD

Baseline: Hanning - starting from0.001s

Fig. 6AE

Partial Occlusion: Hanning - starting from0.001s

Fig. 6AF

Complete Occlusion: Hanning - starting from0.001s

-90

• - P-QRS —412
+ - ST-T —413

±180 — 0    Fig. 6AG

+90

Baseline: Hanning - starting from0.07s

-90

• - P-QRS —414
+ - ST-T —415

±180 — 0    Fig. 6AH

+90

Partial Occlusion: Hanning - starting from0.07s

Fig. 6AI

Complete Occlusion: Hanning - starting from0.07s

Fig. 6AJ

Baseline: Hanning - starting from0.14s

Fig. 6AK

Partial Occlusion: Hanning - starting from0.14s

Fig. 6AL

84

Complete Occlusion: Hanning - starting from0.14s

Fig. 6AM

Baseline: Hanning - starting from0.21s

Fig. 6AN

Partial Occlusion: Hanning - starting from 0.21s

Fig. 6AO

Complete Occlusion: Hanning - starting from 0.21s

Fig. 6AP

Fig. 7

850 —

851 ⟶ Obtain Multiple Measurements Between Multiple
Electrode Pairs, Chosen From At Least 3
Electrodes

852 ⟶ Pre-Filter Signals To Suppress Incoherent Noise

853 ⟶ Compute The Cross-Correlation Matrix

854 ⟶ Compute Eigenvalues Of The Cross-Correlation
Matrix

855 ⟶ Eliminate Noise Sources Associated With Smaller
Eigenvalues

856 ⟶ Obtain Separated Sources

857 ⟶ Perform Additional Separation Based On Higher
Order Statistics, If Necessary

858 ⟶ Identify Selected Vector

Fig. 8

Fig. 9

Fig. 10

# Fig. 11

## Fig. 12

Fig. 13

1425

```
            ┌─────────────────────────────────┐
            │              ·                  │
            │                                 │
            │                                 │
            │         Source Separation       │
            │                                 │
            │                                 │
            │                                 │
     1414   └─────────────────────────────────┘
                             │
                             ▼
            ┌─────────────────────────────────┐
            │                                 │
            │                                 │
            │                                 │
            │     Vector Selection/Information │
            │                                 │
     1419   │                                 │
            └─────────────────────────────────┘
                             │
```

1420

| Arrythmia Discrimination | Therapy Titration | Posture Monitoring | • • • • | Ischemia Monitoring/ Detection | Capture Verification |

# Fig. 14

Fig. 15

Fig. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03003905 A
- US 10876008 B
- US 10465520 B
- US 10795126 B
- US 10738608 B
- US 5133353 A
- US 5179945 A
- US 5314459 A
- US 5318597 A
- US 5620466 A
- US 5662688 A
- US 4562841 A
- US 5284136 A
- US 5376106 A
- US 5036849 A
- US 5540727 A
- US 5836987 A
- US 6044298 A
- US 6055454 A
- US 5313953 A
- US 5388578 A
- US 5411031 A
- US 27003502 A
- US 6411848 B
- US 6285907 B
- US 4928688 A
- US 6459929 B
- US 5334222 A
- US 6026320 A
- US 6371922 B
- US 6597951 B
- US 6424865 B
- US 6542775 B
- US 5203348 A
- US 5230337 A
- US 5360442 A
- US 5366496 A
- US 5397342 A
- US 5391200 A
- US 5545202 A
- US 5603732 A
- US 5916243 A
- US 6360127 B
- US 20020143264 A
- US 10785431 B
- US 5301677 A
- US 6438410 B
- US 6708058 B
- US 5372606 A
- US 5411525 A
- US 5468254 A
- US 5634938 A
- US 6487443 B
- US 6259947 B
- US 6141581 A
- US 5855593 A
- US 5545186 A
- US 6221011 B
- US 6270457 B
- US 6277072 B
- US 6280380 B
- US 6312378 B
- US 6336903 B
- US 6358203 B
- US 6368284 B
- US 6398728 B
- US 6440066 B
- US 81646404 A